# EUROPEAN PATENT SPECIFICATION

(11) **EP 2 044 201 B1**
(45) Date of publication and mention of the grant of the patent: **23.05.2012**
(21) Application number: 06761876.9
(22) Date of filing: 21.07.2006
(51) Int. Cl.: C12N 15/29, C12N 15/82, A01H 5/00

(54) **A MUTANT HISTIDINE KINASE THAT CONFERS SPONTANEOUS NODULATION IN PLANTS**
SPONTANE KNÖLLCHENBILDUNG IN PFLANZEN VERMITTELNDE MUTANTE HISTIDINKINASE
NODULATION SPONTANÉE DANS LES PLANTES INDUITE PAR UNE HISTIDINE KINASE MUTANTE

(43) Date of publication of application: 08.04.2009
(73) Proprietor: Plant Bioscience Limited, Norwich, Norfolk NR4 7UH (GB)
(72) Inventor: TIRICHINE, Leila, F-92300 Levallois-Perret (FR); STOUGAARD, Jens, DK-8000 Aarhus C (DK); SANDAL, Niels, Nørgaard, DK-8381 Tilst (DK); MADSEN, Lene, H, DK-8210 Aarhus V (DK); RADUTOIU, Elena, Simona, DK-8210 Aarhus V (DK)
(74) Representative: Simpson, Verena
(86) International application number: PCT/DK2006/050031
(87) International publication number: WO 2008/009287

(56) References cited:
- WO-A-02/099079
- WO-A-02/102841
- US-B2- 6 768 041
- DATABASE UniProt [Online] 16 May 2006 (2006-05-16), "CHASE; Lantibiotic regulatory protein (Histidine kinase related protein, C-terminal; CHASE; Tripartite hybrid signal transduction histidine kinase, BarA type)." XP002425011 retrieved from EBI accession no. UNIPROT:Q1SE10 Database accession no. Q1SE10
- DATABASE EMBL [Online] 18 February 2006 (2006-02-18), "Cucurbita maxima mRNA for putative histidine kinase" XP002425012 retrieved from EBI accession no. EMBL:AJ628045 Database accession no. AJ628045
- PICHON M ET AL: "ENOD12 GENE EXPRESSION AS A MOLECULAR MARKER FOR COMPARING RHIZOBIUM-DEPENDENT AND -INDEPENDENT NODULATION IN ALFALFA" MOLECULAR PLANT-MICROBE INTERACTIONS, APS PRESS, ST. PAUL, MN, US, vol. 7, no. 6, 1994, pages 740-747, XP008060992 ISSN: 0894-0282
- JIMENEZ-ZURDO J I ET AL: "EXPRESSION PROFILES OF 22 NOVEL MOLECULAR MARKERS FOR ORGANOGENETIC PATHWAYS ACTING IN ALFALFA NODULE DEVELOPMENT" MOLECULAR PLANT-MICROBE INTERACTIONS, APS PRESS, ST. PAUL, MN, US, vol. 13, no. 1, January 2000 (2000-01), pages 96-106, XP008060993 ISSN: 0894-0282
- TIRICHINE LEILA ET AL: "A gain-of-function mutation in a cytokinin receptor triggers spontaneous root nodule organogenesis" SCIENCE (WASHINGTON D C), vol. 315, no. 5808, January 2007 (2007-01), pages 104-107, XP002424882 ISSN: 0036-8075
- LEVY J ET AL: "A putative Ca2+ and calmodulin-dependent protein kinase required for bacterial and fungal symbioses" SCIENCE, AMERICAN ASSOCIATION FOR THE ADVANCEMENT OF SCIENCE,, US, vol. 303, no. 5662, 27 February 2004 (2004-02-27), pages 1361-1364, XP002370541 ISSN: 0036-8075
- MITRA R M ET AL: "A Ca2+/calmodulin-dependent protein kinase required for symbiotic nodule development: Gene identification by transcript-based cloning" PROCEEDINGS OF THE NATIONAL ACADEMY OF SCIENCES OF USA, NATIONAL ACADEMY OF SCIENCE, WASHINGTON, DC, US, vol. 101, no. 13, 30 March 2004 (2004-03-30), pages 4701-4705, XP002370542 ISSN: 0027-8424
- OLDROYD G E D ET AL: "CALCIUM, KINASES AND NODULATION SIGNALLING IN LEGUMES" NATURE REVIEWS MOLECULAR CELL BIOLOGY, MACMILLAN MAGAZINES, LONDON, GB, vol. 5, no. 7, July 2004 (2004-07), pages 566-576, XP008061025

## Description

### Background of the invention

The growth of agricultural crops is generally limited by the availability of nitrogen, and at least 50% of global requirement is met by the application of synthetic fertilisers in the form of ammonia, nitrate or urea. However, there is a growing need to exploit one of the most important natural sources of nitrogen for agriculture, namely biological nitrogen fixation.

The primary source of biological nitrogen fixation are *Rhizobium* or *Rhizobia* spp and the actinobacterium *Frankia* spp, which are a small group of prokaryotes that produce nitrogenases, and form endosymbiotic associations with plants conferring the ability to fix nitrogen. Although many plants can associate with nitrogen-fixing bacteria, only a few plants, all members of the Rosid I Clade, form an endosymbiotic association with *Rhizobia* spp and *Frankia* spp., which are unique in that most of the nitrogen is transferred to and assimilated by the host plant. The *Leguminosae* plant family, which includes soybean, bean, pea, peanut, chickpea, cowpea, lentil, pigeonpea, alfalfa and clover, are the most agronomically important members of this small group of nitrogen-fixing plants. Biological nitrogen fixation via the endosymbiotic association reduces the need for expensive nitrogen fertilizers in legume crops and is an important feature of sustainable agriculture. Legumes can also utilize nitrogen available in the soil, such that when levels of soil nitrate are high, nodule formation is suppressed and the plant shifts from nitrogen metabolism to growth on nitrate (Wopereis et al., 2000).

*Rhizobium*-legume symbiosis involves the interaction of a set of plant and bacterial genes in a complex process leading to the initiation and development of root nodules. Organogenesis of nodules is triggered by the rhizobial microsymbiont, but the legume host plant encodes the developmental program responsible for building the nodule tissues and for regulating the process. Low molecular weight lipo-chitin-oligosaccharides (Nod-factors), synthesized and secreted by *Rhizobia,* are major signal molecules that trigger this process. The major Nod-factor secreted by the *Mesorhizobium loti* microsymbiont of *Lotus* is a pentameric N-acetylglucosamine carrying a *cis*-vaccenic acid and a carbamoyl group at the non-reducing terminal residue together with a 4-O-acetylfucose at the reducing terminal residue. Perception of Nod-factor in *Lotus* is mediated by NFR1 and NFR5 receptor kinases (Radutoiu et al., 2003 Nature 425: 585-592; Madsen et al., 2003 Nature 425: 637-640), that together with an LRR receptor-kinase gene, *SymRK,* communicate with a common signal transduction pathway, shared with mycorrhizal symbiosis (Oldroyd and Downie, 2004 Mol. Cell Biology 5: 566-576). This common pathway is encoded by seven genes, *SymRK, Castor, Pollux, Nup133, CCaMK* [*Sym15], Sym6* and *Sym24.* Analysis of mutants has shown that NFR1/NFR5 receptor(s), *SymRK* encoded LRR protein kinase, CASTOR/POLLUX cation channel(s) and nucleoporin133 are required for the induction of calcium spiking, one of the earliest physiological responses detectable in root hairs exposed to purified Nod-factor.

To establish symbiosis, *Rhizobia* gain access to single plant cells by endocytosis where they are installed in symbiosomes surrounded by a peribacteroid membrane. In *Lotus,* infection occurs via an infection thread that takes the bacteria through root hairs into the root cortex and distributes them to cells, which become infected symbiosome containing nitrogen-fixing cells. In response to attached bacteria, root hairs deform and curl, setting up a pocket that provides a site for infection thread initiation (Geurts et al., 2005 Curr. Opinion Plant Biol., 8: 346-352). Infection threads are plant-derived structures originating from plasma membrane invagination, accompanied by external deposition of cell wall material. In advance of the inward progressing intracellular thread, root cortical cells dedifferentiate and re-enter the cell cycle to initiate the nodule primordium. Later in the process, pattern formation and cell differentiation specify tissue and cell types including the infected cells that endocytose *Rhizobia.* In the mature functional nodule, peripheral vascular bundles are connected to the root vasculature and the main tissues/cell types can be distinguished (Pawlowski and Bisseling, 1996, Plant Cell 8: 1899-1913).

Analysis of a group of nodulation mutants, including some that fail to show calcium oscillations in response to Nod-factor signals, has revealed that in addition to the lack of nodulation, these mutants are unable to form endosymbioses with arbuscular mycorrhizal fungi. This implies that a common symbiotic signal transduction pathway is shared by two types of endosymbiotic relationships, namely root nodule symbiosis, which is largely restricted to the legume family, and arbuscular mycorrhizal symbiosis, which is common to the majority of land plant species. This suggests that there may be a few key genes which dispose legumes to engage in nodulation, and which are missing from crop plants such as cereals. The identification of these key genes, which encode functions which are indispensable for establishing a nitrogen fixing system in legumes, and their transfer and expression in non-nodulating plants, has long been a goal of molecular plant breeders. This could have a significant agronomic impact on the cultivation of cereals such as rice, where production of two harvests a year may require fertilisation with up to 400 kg nitrogen per hectare.

Root nodule symbiosis depends on a successful interaction between the plant host and its cognate symbiont that includes the step of nod factor recognition by the host plant. The identification of genes that regulate nodulation in Legumes would provide the tools to optimise and modify this process to the benefit of agriculture.

WO02/099079 describes methods for increasing plant yield growth and productivity by manipulating expression of cytokinin response regulators (e.g. histidine protein kinases). EBI Accession No. UNIPROT:Q1SE10 Database accession no. Q1SE10 discloses a histidine kinase related protein (tripartite hybrid signal transduction histidine kinase). EBI Accession No. EMBL:AJ628045 Database accession no. AJ628045 discloses a *Cucurbita maxima* mRNA for a putative histidine kinase. US 6,768,041 discloses polynucleotides isolated from Pine and Eucalyptus encoding receptor-like kinases, histidine kinases, and response regulators and their use to modify plant cell responses during growth, development or stress. WO02/10284 discloses a NORK gene and NORK protein from *Medicago sativa,* which is involved in one of the steps of symbiotic nitrogen fixation, and their use for producing transgenic plants. Pichon, M et al., 1994 MPMI 7(6): 740 - 747 describes the occurrence of spontaneous nodulation in some alfalfa genotypes (Nar+), and uses *ENOD*12 gene expression to monitor their ontogeny. Jiménez-Zurdo JI et al 2000 MPMPI 13(1): 96-106 describes 22 nodule associated ESTs and their expression was investigated during ontogeny of spontaneous nodules and induced nodules.

In summary, there is a need to improve nodule formation capability and nitrogen fixation properties in legume crops, as well as to transfer this pathway into non-nodulating crops in order to meet the nutritional needs of a growing global population, while minimising the future use of nitrogen fertilisers and their associated negative environmental impact.

### Summary of the invention

A first embodiment of the invention is a DNA molecule encoding a mutant histidine kinase polypeptide comprising an amino acid sequence selected from among: SEQ ID NO: 6, 15 and 16, wherein the amino acid residue 266 [Phe] in SEQ ID NO: 6 and 266 [Xaa] in SEQ ID NO: 15 and residue 267 [Xaa] in SEQ ID NO: 16 is selected from among isoleucine, serine, threonine, valine, methionine, alanine, phenylalanine, tyrosine, tryptophan, arginine, lysine, glycine, histidine, aspartate, asparagine, glutamate, glutamine, proline and cysteine,
and (b) a truncation of (a), wherein said polypeptide is capable of inducing spontaneous nodule formation when expressed in a plant.

A second embodiment of the invention is a mutant histidine kinase polypeptide comprising an amino acid sequence selected from among: (a) SEQ ID NO: 6, 15 and 16, wherein the amino acid residue 266 [Phe] in SEQ ID NO: 6 and residue 266 [Xaa] in SEQ ID NO: 15 and residue 267 [Xaa] in SEQ ID NO: 16 is selected from among isoleucine, serine, threonine, valine, methionine, alanine, phenylalanine, tyrosine, tryptophan, arginine, lysine, glycine, histidine, aspartate, asparagine, glutamate, glutamine, proline and cysteine; and (b) a truncation of (a) capable of inducing spontaneous nodule formation in a plant.

A further embodiment of the invention is a genetically modified plant characterised by having a nucleotide sequence encoding a polypeptide comprising an mutant histidine kinase consisting of an amino acid sequence selected from among: (a) SEQ ID NO: 6, 15 and 16, wherein the amino acid residue 266 [Phe] in SEQ ID NO: 6 and residue 266 [Xaa] in SEQ ID NO: 15 and residue 267 [Xaa] in SEQ ID NO: 16 is selected from among isoleucine, serine, threonine, valine, methionine, alanine, phenylalanine, tyrosine, tryptophan, arginine, lysine, glycine, histidine, aspartate, asparagine, glutamate, glutamine, proline and cysteine; (b) a truncation of (a), wherein said polypeptide is capable of inducing spontaneous nodule formation when expressed is a plant and wherein said plant is capable of spontaneous nodule formation.

The invention is further directed a method of producing a genetically modified plant according to the invention, characterised by introducing a gene cassette comprising said nucleotide sequence encoding said polypeptide and selecting a transgenic plant and its progeny expressing said polypeptide.

The invention further includes a genetically modified plant according to the invention produced according to a process of DNA mutagenesis and selecting a plant capable of spontaneous nodule formation, or by a method of transformation with a transgene encoding a mutant histidine kinase of the invention.

The invention further includes a seed of or a crop comprising the genetically modified plant of the invention, wherein said seed or crop is as defined in the claims.

### Brief description of the drawings

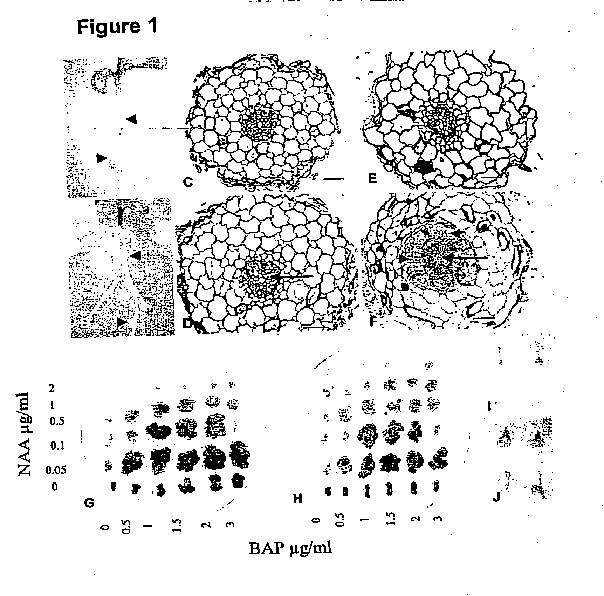

**Figure 1****. Phenotypic characterisation of the *snf2* mutant.**
   (**A**) wild type rhizobia induced root nodule (**B**) spontaneous root nodule on *snf2* root 5 weeks post germination. Arrowheads indicate nodules. Transverse section of (**C**) wild type and (**D**) *snf2* root at time zero and (**E**) wild type and (**F**) *snf2* root after 6 days of growth on B5 hormone-free medium. Arrowhead indicates dividing cells in the pericycle. Arrows indicate xylem cells. (**G**) and (**H**) callus growth from hypocotyls of wild type and *snf2* on different concentrations of auxin and cytokinin. The calli were photographed after 21 days of growth at 26°C. (**I**) and (**J**) root segments of wild type and *snf2* incubated for three weeks on hormone free media. Scale bars: (C); (D); (E); (F) 50 µm.

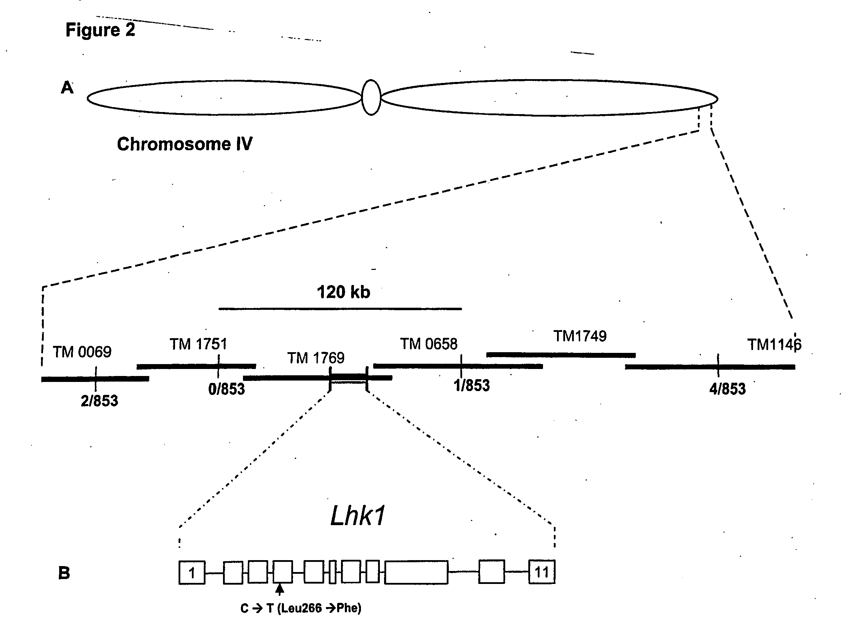

**Figure 2****. Map based cloning of the *Lhk1* gene**
   A: Cartoon of *Lotus japonicus* chromosome IV with expanded genetic map of *snf2* region (*Lhk1* locus) below, comprising 6 BAC/TAC clones (underlined in bold). Vertical lines indicate microsatellites or single nucleotide polymorphism markers. Number of recombinant plants obtained for a microsatellite (TM) marker is indicated in bold below the marker.
   B: Exon-intron structure of the *Lhk1* gene, where the 11 exons of *Lhk1* are indicated by open boxes. The C→T transition in exon 4 of the mutant *snf2* allele, (indicated with a black arrowhead) encodes a mutant LHK1 polypeptide comprising a substitution of F266 for L266.
**Figure 3****. Transformation of *snf2* allele into wild type hairy roots leads to controlled organogenesis and spontaneous nodulation phenotype.** *Lotus japonicus* wild type plants were transformed with *Agrobacterium rhizogenes* carrying the *snf2* gene. Spontaneous nodules on hairy roots are identified by white arrowheads. The main root is identified by a white arrow.
**Figure 4****. Structure of the *Lotus* LHK1 protein.**
   (**A**) Schematic representation of the LHK1 protein domains. (**B**) The amino acid sequence of LHK1 arranged in protein domains. The predicted extracellular receptor domain is given in italics and the CHASE domain within the extracellular receptor domain is underlined; the histidine kinase domain is given in bold and underlined; the His Kinase ATPase domain is given in bold; and the receiver domain is in bold and italics. The asterisk in the CHASE domain marks the position of the amino acid substitution in the *snf2* allele.

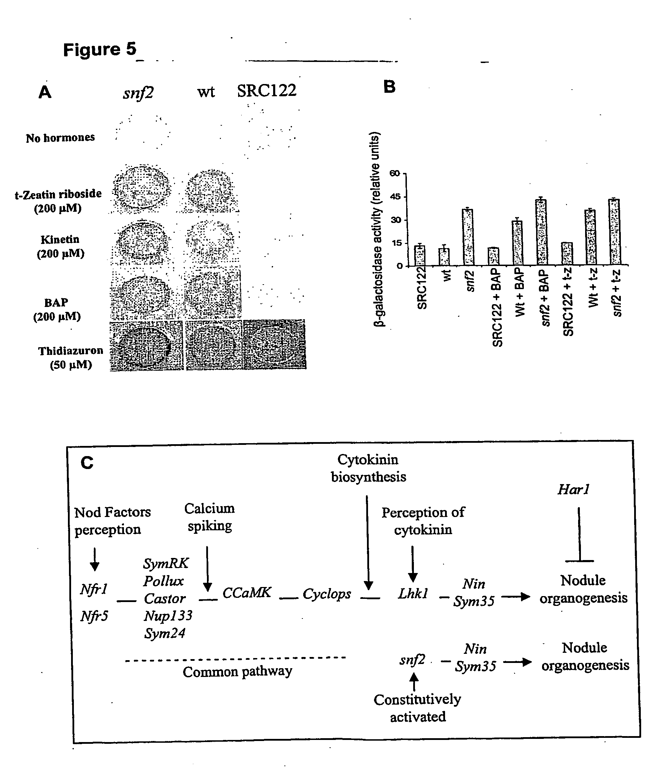

**Figure 5****. *In vivo* assays of receptor mediated cytokinin signalling.**
   (**A**) Plate assay of β-galactosidase activity expressed from a *cps::lacZ* reporter gene in *E*. *coli.* An *E*. *coli* SRC122 strain carrying the *cps::lacZ* reporter, transformed with a plasmid construct comprising the *snf2* cDNA or wild type cDNA, was grown on plates in the absence of cytokinin or in presence of four different cytokinins. The blue color shows β-galactosidase conversion of X-Gal substrate.
   (**B**) Cytokinin induction of β -galactosidase activity in liquid cultures of SRC122 *cps::lacZ* transformed with either the *snf2* cDNA or wild type cDNA. T-z: Trans-zeatin.
   (**C**) Working model for the functional role of *Lhk1* in nodulation. Recognition of a correctly decorated rhizobial Nod-factor by NFR1 and NFR5 induces signal transduction through the common pathway, including calcium spiking and interpretation of calcium oscillation by the CCaMK protein. A localised increase in cytokinin biosynthesis perceived by the LHK1 receptor then leads to cell dedifferentiation and activation of the cell cycle. *snf2* is constitutively active but still requires *Nin* and *Sym35* genes for nodule organogenesis.

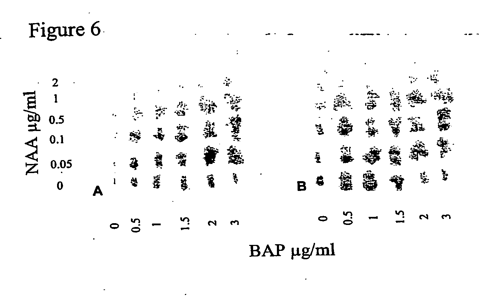

**Figure 6****. Effect of phytohormone treatment on callus growth of root explants.** Callus growth of wild type *L. japonicus* ecotype Gifu (**A**) and (**B**) *snf2* root segments on different concentrations of auxin and cytokinin. The calli were photographed after 21 days of growth at 26°C.

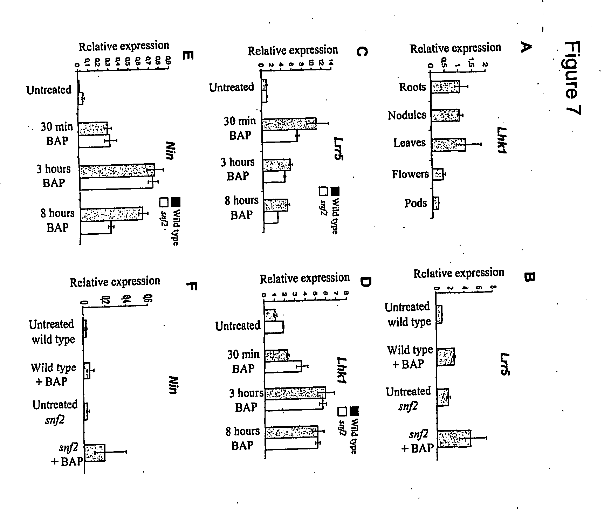

**Figure 7****. Expression of the *Lhk1* gene in organs and the *Lhk1, Lrr5* and *nin* in response to cytokinin.** (**A**) Expression of *Lhk1* in different organs. (**B**) Expression of *Lrr5* gene in wild type and *snf2* root explants incubated on B5 agar plates with or without 0.5 µg/ml of BAP for 10 days. (**C**), (**D**), (**E**) Expression of *Lrr5, Lhk1* and *Nin,* genes in intact wild type and *snf2* roots in response to cytokinin. (F) Expression of *Nin* gene in wild type and *snf2* root explants incubated on B5 agar plates with or without 0.5 µg/ml of BAP for 10 days.

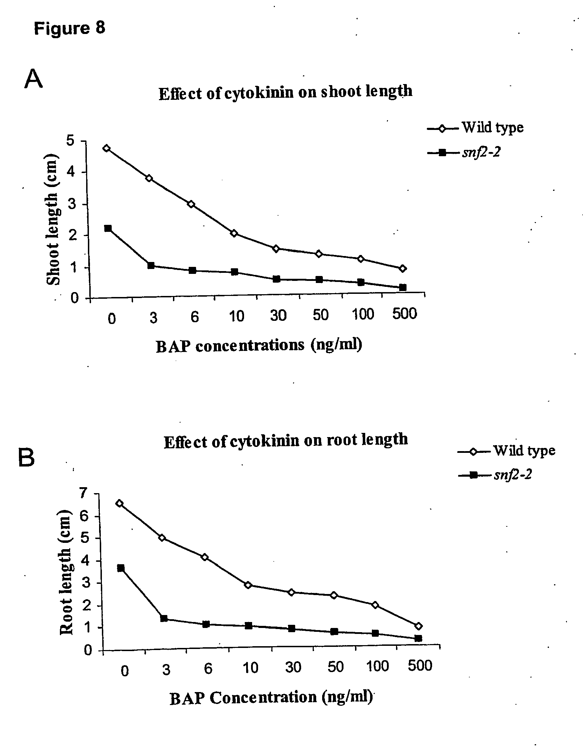

**Figure 8****: Effect of cytokinin on shoot and root length of *L. japonicus* wild type and *snf2-2* mutant.** Plants were grown on ¼ B&D containing different concentrations of BAP. Shoot and root length were measured after 3 weeks of growth. (**A**) shoot length (**B**) root length.

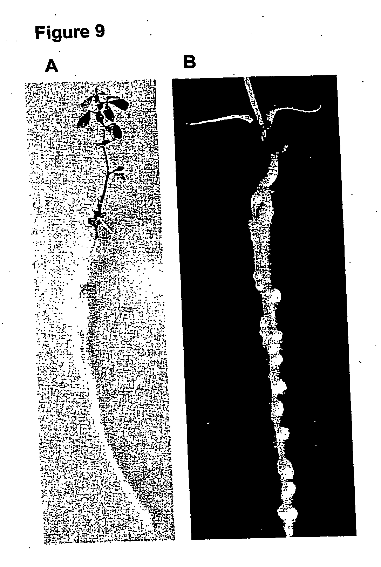

**Figure 9****. Nodulation phenotype of *snf2* and the double mutant *snf2 har1* in absence of rhizobia.** (A) *snf2* mutant and (B) *snf2 har1* double mutant 5 weeks post germination

### Detailed description

The present invention provides an isolated DNA molecule encoding a mutant histidine kinase polypeptide comprising an amino acid sequence selected from among: (a) SEQ ID NO: 6, 15 and 16, wherein the amino acid residue 266 [Phe] in SEQ ID NO: 6 and residue 266 [Xaa] in SEQ ID NO: 15 and residue 267 [Xaa] in SEQ ID NO: 16 is selected from among isoleucine, serine, threonine, valine, methionine, alanine, phenylalanine, tyrosine, tryptophan, arginine, lysine, glycine, histidine, aspartate, asparagine, glutamate, glutamine, proline and cysteine, and (b) a truncation of (a), whose expression in plants confers a spontaneous nodulation phenotype. More specifically the gene was isolated from *snf2* spontaneous nodulation mutants of *Lotus japonicus* that develop white rhizobia free nodules in absence of the *M. loti* microsymbiont, while wild type *Lotus* plants are only nodulated following induction with its cognate nod-factors or rhizobial symbiont (figure 1a, b). Detailed histological analyses of nodule sections demonstrate that *snf2* spontaneous nodules are genuine nodules with an ontogeny and physiology similar to rhizobial induced nodules. Thus, spontaneous root nodules formed on *snf2* mutants originate from cortical cells and have a peripheral vasculature characteristic for wild-type nodules, although they are devoid of infection threads and rhizobia. The *snf2* allele is monogenic dominant and inoculation of *snf2* mutants with *M. loti* results in development of normal nitrogen-fixing root nodules consistent with the presence of a gain-of-function mutation in this allele.

The *snf2* allele maps to a position on chromosome 4 approximately 1 cM from the end of the long arm (figure 2). The gene, corresponding to the *snf2* allele, encodes a mutant form of a *Lotus* Histidine Kinase (LHK1) that is a homolog of *Arabidopsis his*tidine *k*inase genes (*AHK*) encoding cytokinin receptor proteins. A comparison of the wild type *Lotus* histidine kinase gene (*Lhk1*) [SEQ ID NO:1] with the corresponding gene of the *snf2* allele [SEQ ID NO:4] reveals a single nucleotide transition (C to T) in the coding sequence of the *snf2* allele that results in the substitution of a conserved leucine [L266 in *Lhk1;* SEQ ID NO:3] by a phenylalanine (F266 in *snf2* allele of *Lhk1*;SEQ ID NO:6) in the encoded polypeptide. The isolated cDNAs correspond to a transcript of the wild type *Lhk1* gene [SEQ ID NO:2] and that of the snf2 allele [SEQ ID NO:5]. Alignment of the *Lhk1* genomic and cDNA sequences or the respective snf2 allele gene and cDNA sequences, defines a primary structure of *Lhk1* and its *snf2* allele as consisting of 11 exons (figure 2).

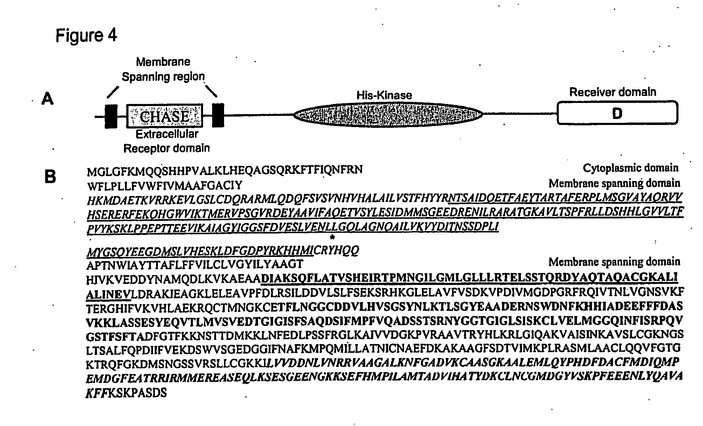

The isolated wild type and mutant cytokinin receptor proteins (LHK1) encoded by the *Lhk1* gene and its *snf* allele respectively, which comprise 993 amino acids with a predicted mass of 110 kD (figure 4). Both proteins comprise two membrane spanning segments at the N-terminus, located between amino acids 37 and 57 and between amino acids 328 and 357. Located between these segments are motifs characteristic of cyclases/histidine kinases associated sensory extracellular (CHASE) domain. This predicted extracellular domain is followed by a putative intracellular histidine kinase between amino acids 379 and 693 and a receiver domain between amino acids 852 and 985. These domains are characteristic for two-component regulatory systems operating through a phospho-relay. The phenylalanine 266 in the *snf2* allele, substituted for leucine 266, is localised in a conserved motif shared among the extracellular CHASE domains of histidine kinase receptors (figure 4). The functional properties of the histidine kinase (cytokinin receptor protein) encoded by the snf2 allele include cytokinin-independent activity, such that the snf2 allele, in contrast to the wild type *Lhk1* allele, can induce nodulation in the absence of cytokinin signalling.

Accordingly, the present invention provides an isolated mutant histidine kinase protein (mutant cytokinin receptor protein) capable of inducing spontaneous nodulation when expressed in a plant, comprising an amino acid sequence selected from among: (a) SEQ ID NO: 6, 15 and 16, wherein the amino acid residue 266 [Phe] in SEQ ID NO: 6 and residue 266 [Xaa] in SEQ ID NO: 15 and residue 267 [Xaa] in SEQ ID NO: 16, (said amino acid residue corresponding to L266 in wild type LHK1 histidine kinase encoded by the *Lotus japonicus Lhk1* gene) is substituted by an amino acid selected from isoleucine, serine, threonine, valine, methionine, alanine, phenylalanine, tyrosine, tryptophan, arginine, lysine, glycine, histidine, aspartate, asparagine, glutamate, glutamine, proline and cysteine, and (b) a truncation of (a). Alternatively described herein, the amino acid residue 266 [Phe] in SEQ ID NO: 6 and 266 [Xaa] in SEQ ID NO: 15 and residue 267 [Xaa] in SEQ ID NO: 16 (corresponding to L266 in wild type LHK1 histidine kinase (Xaa)) is conservatively substituted with an amino acid selected from among phenylalanine, tyrosine, tryptophan, histidine or proline. Said latter group may be extended to include either of the 3 following groups of amino acids (arginine, lysine, aspartate, glutamate) or (aspargine, glutamine, serine, threonine) or (cysteine, methionine, isoleucine, valine, glycine, alanine). Herein described are mutant histidine kinase homologues from a number of plants in which the amino acid residue corresponding to L266 in wild type LHK1 histidine kinase (based on alignment as shown in Table 2) is deleted or substituted with any amino acid other than leucine. More specifically mutant histidine kinase homologues from *Lotus japonicus* (mutant form of Lhk1; SEQ ID NO:6, 15), *M. truncatula* (mutant form of ABE94286; SEQ ID NO:16), *Arabidopsis* (mutant form of BAB33311; SEQ ID NO:9,17), rice (mutant form of XP_469566; SEQ ID NO:18), maize (mutant form of BAE80688; SEQ ID NO:12,19) and *Cucurbita maxima* (mutant form of CAF31355.1; SEQ ID NO:14,20) or orthologues or allelelic variants thereof, wherein Xaa is any amino acid other than leucine. The mutant histidine kinase may be one in which Xaa is selected from the group phenylalanine, tyrosine, tryptophan, histidine or proline, or alternatively Xaa is phenylalanine. The orthologue or allelic variant of the mutant histidine kinase herein described shares a percent sequence identity with said mutant histidine kinase, selected from the group consisting of at least 60, 65, 70, 75, 80, 85, 90, 95, and 98 percent, or truncation thereof, wherein said kinase has all of the functional properties of the mutant histidine kinase of the invention, such that when expressed in a plant it confers the ability to form spontaneous nodules. The orthologue/variant allele may be a mutant histidine kinase that is a cytokinin receptor protein that is cytokinin independent.

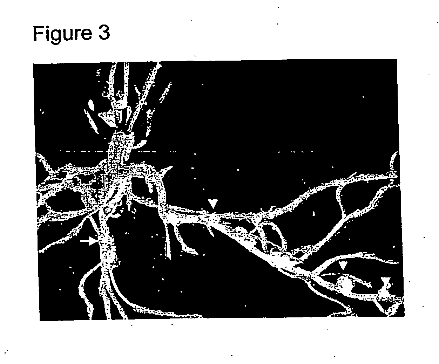

This single amino acid substitution or deletion encoded by the mutant *snf2* allele of the *Lhk1* gene or its homologues is sufficient to confer a spontaneous nodulation phenotype on the roots of transformed wild-type plants expressing a transgene comprising the gene corresponding to the mutant *snf* allele (Table 1 and figure 3). The *snf2* allele acts as a dominant gain-of-function gene, since spontaneous nodulation is seen in a wild type genetic background, and is absent in plants transformed with the wild type *Lhk1* gene. Hence, expression of a single copy of the mutant histidine kinase of the invention in a plant, as in the case of a heterozygous plant, is sufficient to confer the *snf2* spontaneous nodulation phentoype.

Also provided is a DNA molecule encoding said mutant histidine kinase homologue (wherein Xaa is phe) from *Lotus japonicus* (SEQ ID NO:4,5), *M*. *truncatula* (mutant form of Accession AC141922.19; SEQ ID NO:7), or fragments thereof encoding a full-length or truncated functional mutant histidine kinase capable of causing spontaneous nodulation when expressed in a plant.

The DNA sequence encoding the mutant histidine kinase protein of the invention may be operably linked to a promoter DNA sequence capable of driving expression of said histidine kinase in a plant, and to a 3' terminator sequence. The promoter can be a promoter directing expression of said histidine kinase in root tissues of a plant and/or in cells destined to become nodule primordia and mature into nodules. Suitable examples of a promoter and terminator include the promoter and terminator sequence of the corresponding wild type *Lhk1* gene. The promoter used to direct expression of the mutant histidine kinase may be a regulated (e.g. tissue or cell-type specific promoter) which includes the native promoter of the *Lhk1* gene or its homologues . An example of a heterologous constitutive promoter includes the 35SCaMV promoter (Acc.No:V00141, J02048). A transgene (gene cassette) comprising a DNA sequence encoding a mutant histidine kinase of the invention operably fused to a promoter sequence and optionally a terminator sequence can be constructed by recombinant DNA techniques.

A transgene comprising a DNA sequence encoding the mutant histidine kinase can be used to generate a plant expressing the mutant histidine kinase of the invention. The transgene can be stably integrated into the genome of a host plant by transformation techniques well know to one skilled in the art. Furthermore, binary vectors and *Agrobacterium tumefaciens*-based methods for the stable integration of transgenes into all major cereal plants are know, as described for example for rice (Hiei et al, 1994, The Plant J. 6; 271-282), and maize (Yuji et al, 1996, Nature Biotech. 14: 745-750). A DNA sequence encoding a mutant histidine kinase can also be introgressed into another plant by crossing with a genetically modified plant expressing the mutant histidine kinase.

The genetically modified plant of the invention, whether generated by mutagenesis or transformation with a transgene of the invention, can be used in a breeding program, in order to select plants with the ability to fix nitrogen, or enhanced nitrogen fixation ability, that have inherited the gene encoding the mutant histidine kinase. The invention thus encompasses a genetically modified plant, produced by transformation of a natural plant, that is capable of spontaneous nodulation. The expression of a gene encoding a mutant histidine kinase in a nitrogen-fixing plant, such as a member of the *Leguminoseae* (such as soybean, bean, pea, peanut, chickpea, cowpea, lentil, pigeonpea, alfalfa and clover), has particularly utility with respect to enhancing the nitrogen-fixing ability of said plant under one or more environmental growth conditions. The expression of a gene encoding a mutant histidine kinase in a crop plant that does not naturally fix nitrogen, such as a dicotyledenous plant or a monocotyledenous plant including a member of the cereals (such as wheat, rye, oats, barley, sorghum, millet, maize, *Poaceae* grass and rice), has particularly utility with respect to conferring the ability to fix nitrogen. Plants, as well as plant progeny, selected in such a breeding program may be cultivated for the purpose of harvesting a crop, where the crop may be the vegetative plant parts, e.g. leaf, stem or tuber, or reproductive parts, including flowers, seed, caryopsis, cob or fruit.

The examples given below demonstrate that a plant, e.g. *Lotus,* that is homozygous or heterozygous for a gene encoding the mutant histidine kinase polypeptide of the invention, forms spontaneous nodules that can be infected by nitrogen-fixing symbiotic bacteria e.g. *M. loti,* and which are capable of fixing nitrogen and supporting plant growth under nitrogen-limiting conditions. The formation of nitrogen-fixing nodules following inoculation with nitrogen-fixing bacteria in said spontaneously nodulating plants is not dependent on nod factor production by the infecting bacteria or nod factor perception by the infected plant. This indicates that only a subset of nodulation-related genes are required in order for nitrogen fixation to occur in a spontaneously nodulating plant expressing a mutant histidine kinase following inoculation with a rhizobium bacterium. The unique properties of the mutant histidine kinase of the invention may be exploited both to enhance nitrogen fixation in existing nitrogen fixing plants, as well as in establishing nitrogen fixation in non-nodulating plants.

### Example 1. Positional cloning and identification of the mutant snf-2 gene in Lotus japonicus

*Lotus* mutants (*snf2-1* and *snf2-2*) having a spontaneous nodulation phenotype, originates from an EMS screen of *Lotus japonicus* ecotype Gifu seeds. The mutant *snf-2* gene in *Lotus japonicus,* giving rise to spontaneous nodulation, has been localized on the long arm of chromosome IV, approximately 1 cM from the end, at a locus named *Lhk1.*

The location of the *snf2* gene was determined by fine mapping using microsatellite markers (TM markers) and single nucleotide polymorphic markers developed from BAC or TAC clones anchored to the genetic map of the *Lhk1* region. Mapping was performed on an F2 population established from a cross between *Lofus japonicus* ecotypes Miyakojima and MG-20. The fine map was used to build a physical TAC/BAC contig comprising six BAC/TAC clones from MG20 that were assembled to cover the *Lhk1* region between the two flanking markers TM1146 and TM0069 (Figure 2). Since *snf2* is a dominant mutation, wild-type F2 plants, rather than mutant plants, were scored for mapping purposes. Genotyping of 853 wild-type plants that did not develop root nodules spontaneously was used to identify markers delimiting the locus, corresponding to the *snf2* mutation, to a region of 120 kb. Fifteen genes were predicted within this region, one of which was predicted to encode a cytokinin receptor. The sequence of both the wild type [SEQ ID NO:1] and mutant *snf2* alleles [SEQ ID NO:4] of this candidate gene were determined, which revealed a C to T nucleotide substitution in both *snf2* alleles. The wild type gene was designated *L*otus *h*istidine kinase (*Lhk1*) gene encoding a LHK1 polypeptide [SEQ ID NO:3]. The single nucleotide transition (C to T) in an exon sequence of the mutant *snf2* allele, encodes a polypeptide having a phenylalanine (F266 in SEQ ID NO:6) in substitution of the conserved leucine (L266 in SEQ ID NO:3) in the polypeptide encoded by the wild type gene, and identifies *snf2* as an allele of a *L*otus *h*istidine *k*inase (*Lhk1*) gene.

### Example 2. Cloning and identification of the Lhk1 cDNA corresponding to the transcript of the wild type Lhk1 gene in Lotus japonicus

A full-length *Lhk1* cDNA (3568 bp) was isolated from a λ ZAPII cDNA library prepared from mRNA isolated from *M. loti* inoculated *Lotus japonicus* roots. The *Lhk1* cDNA was sequenced [SEQ ID NO:2], from which the transcription start site was determined to lie at least 137 nucleotides upstream of the start codon and the coding sequence was followed by a 3' untranslated region of approximately 445 nucleotides. Alignment of genomic and cDNA sequences defined a primary structure of *Lhk1* consisting of 11 exons (Figure 2B). The nucleotide sequence of the mutant *snf2* allele of the *Lhk1* cDNA is designated SEQ ID NO:5.

### Example 3: Expression of the snf2 allele in wild type Lotus roots confers a spontaneous nodulation phenotype.

Transgenic expression of the wild type or mutant *snf2* allele in wild type Lotus roots was performed in order to confirm *in planta* the genetic basis for the spontaneous nodulation phenotype. A genomic fragment of 12.7 kb from the BAC clone 1K18 was used to clone out the wild type *Lhk1* gene including a 2.2kb promoter region, and its *Snf2* allele comprising the Cut transition, which were then sub-cloned into the pIV10 plasmid. The constructs were integrated into *A. rhizogenes* strain AR12 by tri-parental mating. Transformation was performed as described by Stougaard (1995) Method Mol Biology 49: Plant Gene Transfer and Expression Protocols, p. 49-63, and included transformation with an empty vector control. Root nodulation, scored in the absence of rhizobial induction, was only detected in roots transformed with the *snf2* allele, while the wild type *Lhk1* gene failed to confer spontaneous nodulation on wild type roots (table 1, figure 3).

**Table 1**

| Induction of spontaneous nodules on wild type hairy roots transformed with wild type *Lhk1* gene or its *snf2* gain-of-function allele | | | |
|---|---|---|---|
| Transformation construct | % spontaneous nodulation* | nodule number/ plant* | nodule number/ nodulated plant* |
| empty vector | 0 (0/15) | 0 | 0 |
| Wild type *Lhk1*allele | 0 (0/41) | 0 | 0 |
| *snf2* allele | 37 (30/81) | 0.87 | 2.36 |

| | | | |
|---|---|---|---|
| * Transgenic roots were scored in absence of *M. loti.* | | | |

The ability of the *snf2* allele to confer spontaneous nodulation in transformed roots having a wild-type genetic background serves to confirm the dominant nature of the *snf2* allele. Furthermore it is surprisingly shown that a cytokinin independent histidine kinase (*snf2*), when under the control of a regulated promoter e.g. *Lhk1* gene promoter, confers the controlled organogenesis of root nodules, as against uncontrolled cell proliferation leading to massive nodule development. Controlled formation of spontaneous nodules, capable of infection and nitrogen fixation in plants transformed with the snf2 allele, enhances their capacity to fix nitrogen during cultivation. Since the only structural difference between the wild type *Lhk1* gene and its snf2 allele lies in a point mutation encoding a single amino acid substitution, these data demonstrate that spontaneous nodulation is the result of an L/F266 substitution in a LHK1 polypeptide.

### Example 3: Lotus LHK1 is a member of the cytokinin receptor family.

Annotation of the *Lhk1* cDNA clone reveals an open reading frame of 2979 nucleotides that is predicted to encode a cytokinin receptor protein (LHK1) consisting of 993 amino acids with a predicted mass of 110 kD (figure 4). At the N-terminus, two membrane spanning segments are located between amino acids 37 and 57 and between amino acids 328 and 357. Located between these segments are motifs characteristic for the cyclases/histidine kinases associated sensory extracellular (CHASE) domain. This predicted extracellular domain is followed by a putative intracellular histidine kinase between amino acids 379 and 693 and a receiver domain between amino acids 852 and 985. These domains are characteristic for two-component regulatory systems operating through a phospho-relay.

Comparative analysis defines the Lotus LHK1 protein as a member of the cytokinin receptor family which includes proteins from *Medicago truncatula, Arabidopsis,* rice and maize (table 2). The Lotus LHK1 protein shares an amino acid sequence identity of 83%, 68%, 58% and 49% respectively, with protein homologues from *M. truncatula* (ABE94286), *Arabidopsis* (BAB33311), rice (XP_469566) and maize (BAD01584). Among the three *Arabidopsis* cytokinin receptors, LHK1 is most similar to AHK4/(Cre1) that is important for normal root development and serves a function in perception of externally supplied cytokinin (Mahonen et al. (2000), Genes Dev. 4: 2938). The leucine 266 substituted by a phenylalanine 266 in the *snf2* allele, is localised in a conserved motif shared among the extracellular CHASE domains of histidine kinase receptors (figure 4).

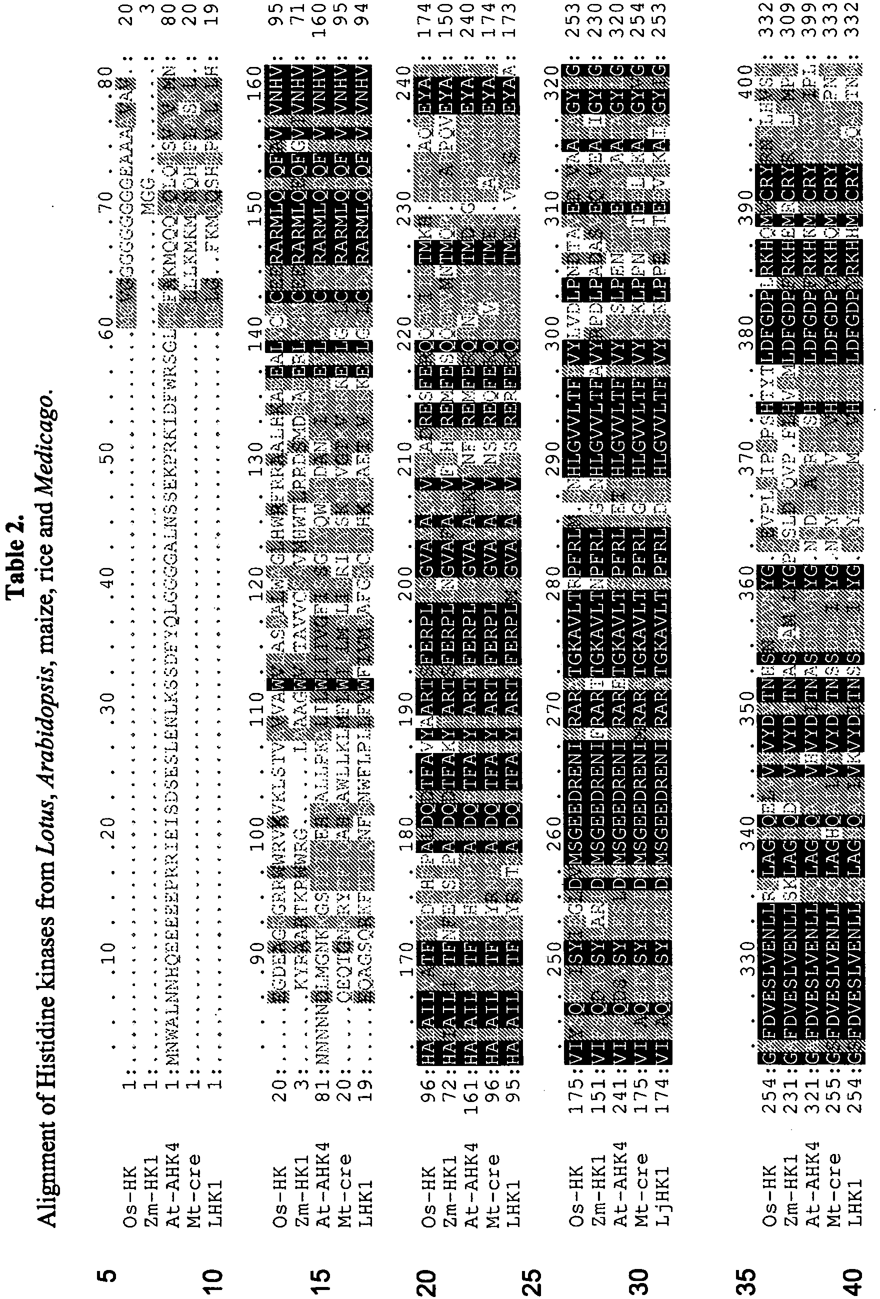

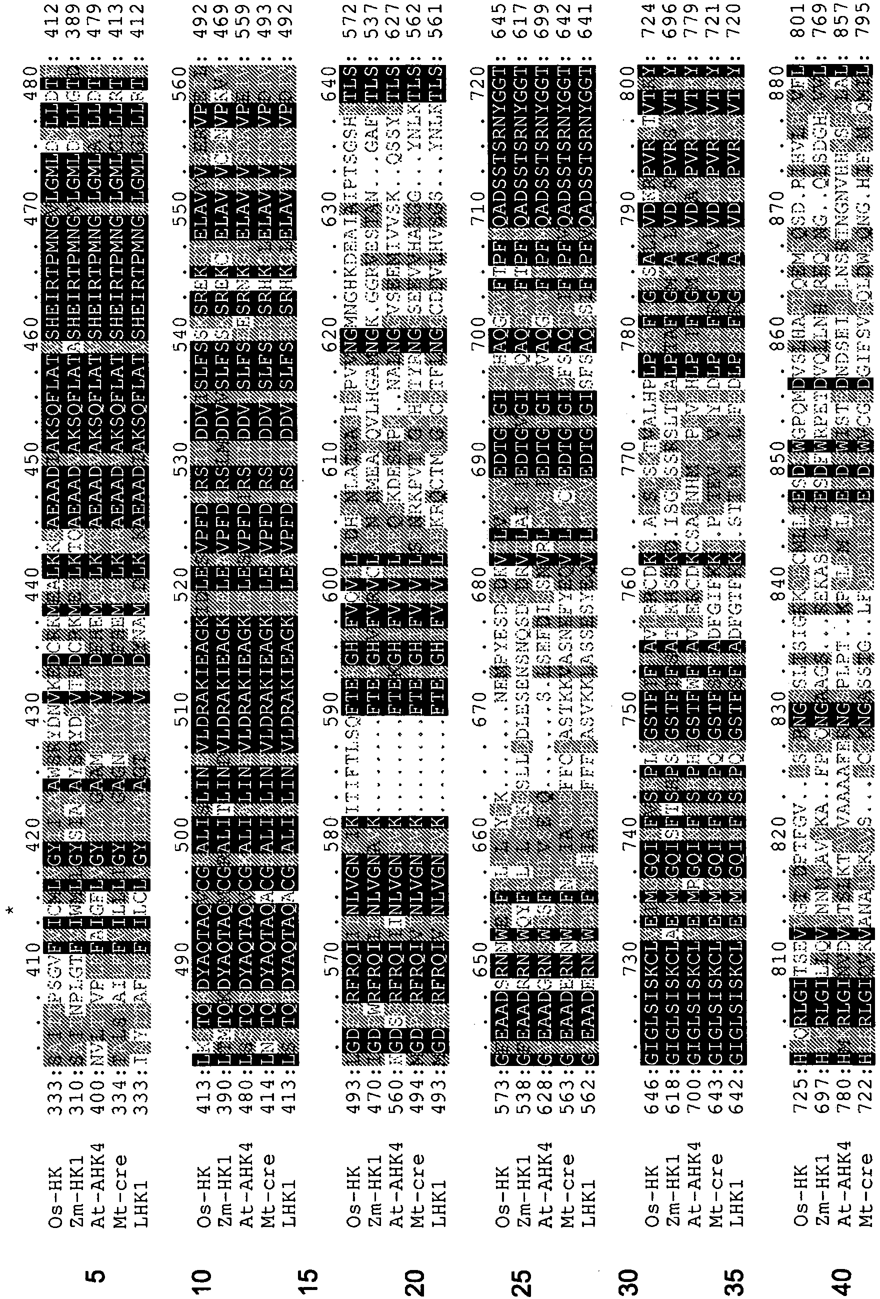

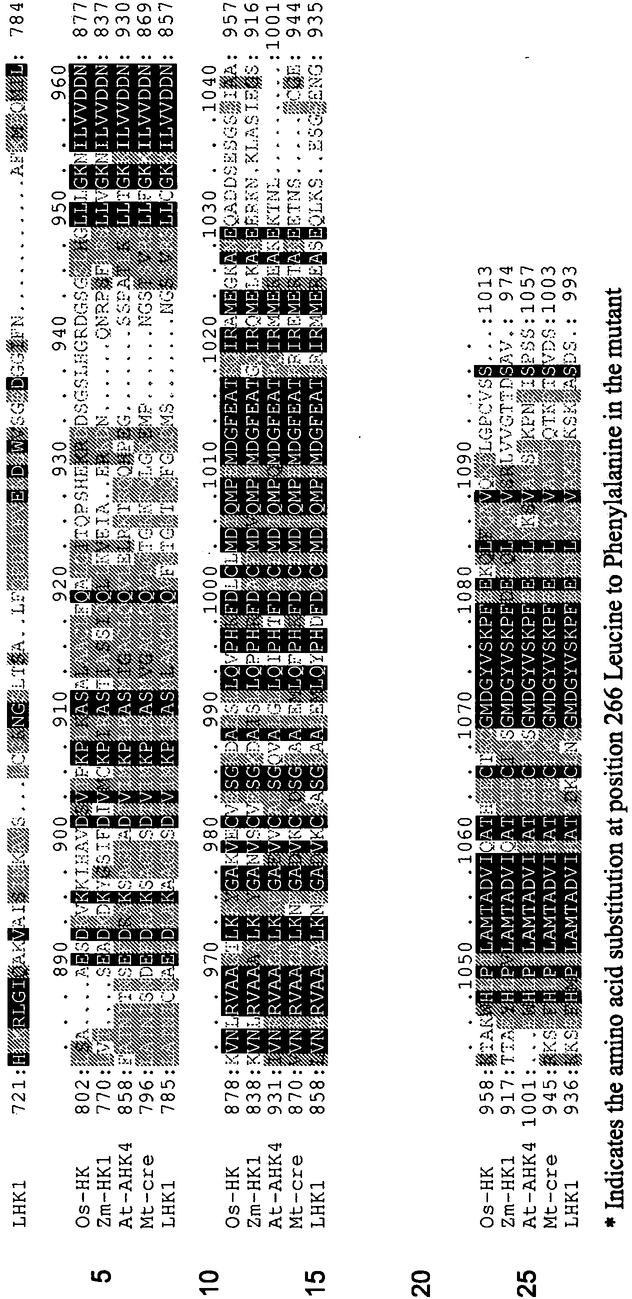

### Example 4. Cytokinin independent activity

The spontaneous nodulation phenotype conferred by the *snf2* allele is shown to be due to a cytokinin-independent function of the mutant LHK1 cytokinin receptor encoded by this allele, which is characterised by a UF266 substitution in the CHASE domain (figure 4). The *in vivo* activity of *Lotus* wild type and mutant LHK1 cytokinin receptors and their cytokinin response was demonstrated by means of a two-component phosphorelay assay developed in *E. coli* (Suzuki et al., (2001) Plant Cell Physiol. 42: 107). In this assay cytokinin induction can be determined without interference from endogenous cytokinin levels, cytokinin penetration and interfering metabolic pathways present in plants. The phosphorelay exploited in this assay is normally involved in regulating bacterial extracellular polysaccharide synthesis through activation of the *cps* operon in response to a sensor (RcsC), which has a domain structure similar to plant cytokinin receptors. Functional expression of a cytokinin receptor in an *E. coli* strain deleted of RcsC sensor allows cytokinin perception to be read out as β-galactosidase activity from the expression of a *cps::lacZ* fusion.

Accordingly the *Lhk1* cDNAs, corresponding to wild type and *snf2* allele were cloned into the pIN-III expression vector and transformed into the sensor-negative SRC122 (ΔRcsC) strain harbouring the *cps::lacZ* gene. The transformants were grown overnight in liquid Luria-agar and then spotted on LB plates, supplemented with and without t-zeatin, kinetin, BAP (200 µM) each and thidiazuron (50 µM). The LB media was further supplemented with X-Gal (5-bromo-4-chloro-3-indolyl-β-D-galactosidase), a β-galactosidase substrate. After 40 hours incubation at room temperature (25°C), the plates were photographed. *E coli* strain (SRC122) was taken as a negative control. Expression of the *snf2* allele (mutant LHK1 cytokinin receptor) is seen to induce β-galactosidase activity in absence of cytokinin (figure 5A). In contrast, wild type LHK1 induced β-galactosidase activity in a cytokinin-dependent fashion (figure 5A). The level of β-galactosidase activity induced by expression of the wild type and *snf2* alleles was quantitatively determined in the transformants after overnight growth at 37°C in LB medium according to the following protocol. Overnight cultures were diluted 1:1000 in 50 ml LB and further grown for 24 hours at 37°C. After the incubation period, 1 ml of culture was centrifuged for 10 min at 13000 rpm. The OD₆₀₀ of the remaining culture was measured. The pellet was resuspended into 1 ml 0.85% NaCl. 200 µl of the suspension were adjusted to 1 ml with Z buffer (Guarentee (1983) Methods Enzymol., 101: 183). Two drops 0.1 % SDS and 1 drop chloroform were added. The samples were vortexed and left at 30° C for 5 min. 200µl of *o*-Nitrophenyl-β-D-galactopyranoside ONPG (4 mg/ml) were then added to the sample, which was incubated further at 30°C. When the samples had obtained an appropriate yellow colour (corresponding to OD₄₂₀ = 0.300-0.600), the reaction is stopped by adding 500 µl of 1 M Na₂CO₃. The stop time was scored. The OD₄₂₀ was measured within 1 hour by spectrophotometry. The β-galactosidase activity was calculated as follow: Relative units = (0D₄₂₀*1000)/(OD₆₀₀*min*ml) (Guarentee, (1983) *supra*).

As shown in figure 5B, expression of the *snf2* allele of LHK1 in *E. coli* cells induced a three fold higher level of β-galactosidase activity than control, non-transformed *E*. *coli* cells and cells expressing wild type LHK1. Cytokinin addition results in a two fold induction of β-galactosidase activity in LHK1 expressing cells while *snf2* allele expressing cells respond with only a marginal increase in activity. These results demonstrate that LHK1 is a cytokinin receptor, while the mutant receptor encoded by the *snf2* allele is constitutively active, whose activity is comparable to the cytokinin-induced activity of the wild type receptor. While not wishing to be bound by theory, it is proposed that the extracellular CHASE domain, that normally binds cytokinin to activate the kinase (Kakimoto, (2001) Plant Cell Physiol. 42: 677; Anantharaman and Aravind, (2001) Trends Biochem Sci. 26: 579; Pas et al., (2004), FEBS Lett. 576: 287) is locked within an active conformation in the mutant (*snf2*) receptor. These properties of the mutant LHK1 would explain both the genetic dominant nature of the *snf2* allele and the phosphorelay assay results.

### Example 5. Impact of the snf2 allele on regulation of cell growth and differentiation

The spontaneous conversion of root cortical cells into root nodule initials seen in *snf2* mutants is probably due to constitutive activity in one or more steps of the pathway controlling cell differentiation. The *in vitro* tissue culture performance of *snf2* cells provides a means to assess the global impact of the *snf2* allele on cell differentiation processes and/or interference with normal phytohormone responses. Accordingly, hypocotyl and root explants (Figure 1 and 6) from 10 days old wild type and *snf2* plants (cultivated on ½ B5 salt medium) were grown on 36 ratios of naphtalene acetic acid (NAA) and 6-benzylaminopurine (BAP) for three weeks. The range of cytokinin and auxin concentrations employed included levels normally used for optimal *in vitro* culture of wild type explants. The explants were moved to a fresh media every week. Representative explants for each hormone combination were arranged on a Petri dish and photographed.

As shown in figure 1G, H and figure 6, there is little observable difference in callus growth from hypocototyl or root explants of plants with wild type and *snf2* alleles. Overall, the hormone dose response is similar. Only at high auxin concentrations (2 µg/ml NAA) did *snf2* explants survive slightly better than wild type explants. Conversely, on cytokinin (BAP) in absence of auxin, *snf2* explants developed less callus than wild type. When cultivated on tissue-culture plates without any phytohormones, green callus grew from the basal end of *snf2* root segments, in a manner reminiscent of a cytokinin-induced tissue response, and clearly different from wild type root segments (figure 1I and J). Co-segregation of this green callus phenotype and the *snf2* allele shows that this un-induced root callus formation was caused by the gain-of function cytokinin receptor encoded by the *snf2* allele.

Microscopy on thin sections of wild-type and *snf2* roots (figure1 C,D and E,F) show at least one extra cell layer in the xylem vessels of *snf2* roots. After 6 days of incubation on hormone-free media, additional pericycle cell layers originating from periclinal divisions were observed together with an increase in cell number of the *snf2* root vasculature while no changes were observed in wild type roots (figure 1 E, F).

### Example 6. Expression of Lhk1 gene in organs and Lhk1, Lrr5 and Nin genes in wild type and snf2 Lotus plants in response to cytokinin

Steady state levels of *Lhk1* and ARR transcripts were determined in roots and other plant organs by quantitative RT-PCR analysis according to the following protocol.

Wild type and *snf2* seeds were surface sterilised and germinated as described previously (Handberg, and Stougaard, (1992) Plant J. 2: 487). Plants were grown with 16 h light/8 h dark period at 21 °C. Total RNA was isolated from roots, nodules, leaves, flowers and pods of inoculated wild type *L. japonicus.* mRNA was extracted from roots of wild type and *snf2* plants grown for 2 weeks on ¼ B&D (Broughton and Dilworth, (1971) Biochem. J. 125: 1075) supplemented with 0.5µM potassium nitrate. Roots were treated with 10 µM BAP for three time points, 30 minutes, 3 hours and 8 hours. Untreated roots were taken as control. mRNA was also extracted from root segments of wild type and *snf2* plants grown for 10 days on hormone-free B5 salt medium and B5 salt medium supplemented with 0.5 µg/ml of BAP. Dynabeads mRNA direct kit (Invitrogen) was used for the extraction of mRNA.

First strand cDNA was prepared using reverse transcriptase (Fermentas). Quantitative PCR was performed on a Light Cycler (Roche Molecular Biochemicals) using Fast Start DNA master SYBR green kit (Roche) to amplify the target transcripts from 5 µl diluted cDNA. Four house keeping genes (Czechowski et al., (2005) Genome analysis 139: 5) were used to determine the relative expression of target genes (see below). For each treatment, normalized relative ratios of the target genes and the four independent house keeping genes have been calculated using the Relative Quantification Software (Quant) from Roche. The geometric mean (Vandesompele et al., (2002) Genome Biology 3:1) of the relative expression ratios for the three biological and technical replicates and the corresponding 95% intervals of confidence have been calculated as described previously (Radutoiu et al., (2003) Nature 425: 585).

The following primer pairs were used for quantitative PCR:
Independent house keeping gene primer sequences:
   **1. *PP2A*- TC9878- homolog of AT1G13320-protein phosphatase 2A**
      5'-GTAAATGCGTCTAAAGATAGGGTCC-3'
      5'-ACTAGACTGTAGTGCTTGAGAGGC-3'
   **2. UBC-CB828248 homolog of AT5G25760- ubiquitin-conjugating enzyme**
      5'-ATGTGCATTTTAAGACAGGG-3'
      5'-GAACGTAGAAGATTGCCTGAA-3'
   **3. TB2C- BI418560- tubulin beta chain**
      5'-GCTCACCACCCCAAGCTTTGG-3'
      5'-TGTCAATGGAGCAAACCCAACC-3'
   **4. ATP -AW719841-ATP synthase-**
      5'-AACACCACTCTCGATCATTTCTCTG-3'
      5'-CAATGTCGCCAAGGCCCATGGTG-3'
Target gene primer sequences:
   ***Lhk1***
      5'-AATTTGGTGAACCGAAGGGTCGCCG-3'
      5'-TCGACGAGTGGCCTCAAACCCATCC-3'
   ***snf2 allele of Lhk1***
      Snf2LC3Fwd: 5'-AGAGGTCTTAAAGCCATTGTG-3'
      Snf2LC6Rev: 5'-TATCAGGCTGAAATAATGCCG-3'
   **Nin-AJ239041.**
      5'-AGGAGCCCAAGTGAGTGCTA-3'
      5'-GCCATCAAGGTATATGACGAG-3'
   **ARR5- CB827384- homolog of AT3G48100-ARRS**
      5'-TCTTGACTCGAATTGATAGGTGC-3'
      5'-GATAGAGATGGCCTGCAACTACTG-3'

Transcript analysis shows *Lhk1* to be expressed at the highest level in roots, nodules and leaves, but transcripts were present in all organs tested (figure 7A).

Cytokinin-induced changes in cellular processes in plants are accompanied by increased expression of response regulators (ARR) genes belonging to the type-A class (Hutchison and Kieber, (2002) The plant Cell 14: 47). Ten genes encode ARRs in *Arabidopsis* and their transcripts have been found in all adult tissues. Type-A *ARR* genes are transcriptionally induced by cytokinin and *ARR4* and *ARR5* are rapidly induced primary response genes. Since the mutant receptor protein encoded by the *snf2* allele showed constitutive activity in the *E*. *coli* test system, the activation of a Lotus *ARR5* homolog (named *Lrr5*) was determined in *snf2* and wild-type roots (figure 7). A twofold higher level of *Lrr5* transcript was found in root explants of *snf2* mutants incubated on hormone-free B5 medium compared to wild type explants, while cytokinin [BAP] addition to the B5 medium increased the *Lrr5* transcript level two to three-fold in both *snf2* and wild-type explants (figure 7B). Direct cytokinin treatment of roots also increased the *Lrr5* transcript level in *snf2* and wild-type roots, but in this experiment, a difference in expression between untreated *snf2* and wild type roots was not detected (figure 7C).

*Lhk1* gene transcripts are also seen to increase rapidly in response to cytokinin treatment in both wild type and *snf2* mutants (Figure 7D), which is consistent with the cytokinin inducibility of its homologue in Arabidopsis *(AHK4* gene). The *Nin* gene in *Lotus,* which is required for the initiation of nodule primordial (Figure 5C), is also transcriptionally upregulated by cytokinin (figure 7E), but the transcript levels in untreated and treated *snf2* roots were not significantly different from that of wild type roots. Ectopic expression of *Nin* in root explants of *snf2* mutants incubated on hormone free B5 medium was not detected (Fig.2F).

In *Arabidopsis,* the *ARR* response pathway is desensitized after prolonged exposure to cytokinin (Rashotte et al., (2003), Plant Physiol. 132: 1998), which may explain the relatively small or lack of upregulation of *Lrr5* and *Nin* in *snf2* mutant roots.

### Example 7. The growth habit of Snf2 Lotus plant is sensitive to cytokinin

Although transcriptional changes in the *snf2 Lotus* plants are limited, plant growth was seen to be strongly affected by application of externally supplied cytokinin. Accordingly, wild type and *snf2* plants were grown on ½ B5 salt with and without increasing concentrations of BAP for 3 weeks. Shoot and root length of at least 60 plants for each treatment was measured. In line with the constitutive activity of the mutant cytokinin receptor encoded by the *snf2* allele, both shoot and root growth of *snf2* plants were hypersensitive to cytokinin compared to wild type (figure 8).

### Example 8. The snf2 root phenotype co-segregates with the snf2 mutation

The roots of *snf2* plants, grown on hormone-free ½ B5 salt media, are characterised by enhanced cell division in the pericycle and vascular tissue (figure 1 D,F). This growth pattern, which leads to a swollen root phenotype, is attributed to the expression of the *snf2* allele. To confirm that the root swelling phenotype indeed co-segregates with the spontaneous nodulation phenotype, the *snf2-2* mutant allele was backcrossed to the wild type Gifu ecotype and the F2 plants [50] were grown on ¼ B&D media for sufficient time to allow the scoring of spontaneous nodulation phenotype (- 5 weeks). The roots of the nodulating and non-nodulating plants were cultivated for 3 weeks on hormone-free media, and then scored for presence and absence of root swelling. Wild type and *snf2* plants were taken as controls. Only roots from F2 plants developing spontaneous nodules showed swelling of the root segments that was comparable to the swelling of the *snf2* root segments. This indicates that expression of the cytokinin independent mutant *Lhk1* protein in *Lotus* plants confers both the *snf2* root and shoot growth phenotype and the spontaneous nodulation phenotype.

### Example 9. snf2 and cytokinin signalling acts downstream of Nod factor induced signal transduction pathway.

The phenotype of *snf2* mutants suggests that cytokinin signalling is part of, or acts downstream, of the Nod-factor induced signal transduction pathway (figure 5C). To test this hypothesis, the *snf2* gene construct was transformed into mutant plants carrying mutations in genes constituting the common signal transduction pathway shared with mycorrhizal fungi or into mutant plants impaired in upstream or downstream genes.

Accordingly, seven single and double *L. japonicus* nodulation mutants were transformed using *Agrobacterium rhizogenes* carrying the *snf2* mutant gene construct or an empty vector as described previously (Stougaard, 1995 *supra).* All the mutants except *sym35* were genotyped by sequencing PCR products covering the mutation using the following primers:
***nfr1***
   Nfr1-1 Msel CAPS fw(56) 5'-CGCTGGTTTACCCATAAACGTGTTC-3'
   Nfr1-1 Msel CAPS rv(55) 5'-GGGCAAATGCATTTGTGCTGAG-3'
***nfr5***
   K2fwB 5'-CCAGCTAGGTGATAGCTACG-3'
   K2revC 5'-CCAGAAGATGAATGCTGCTTT-3'
   S5R rev5(64) 5'-GGTATTAGAACGCCCCCTGG-3'
***symRK (wild type)***
   symRK fw1 5'-CTGAGTTTGGACCCCTTTTG-3'
   symRK rev1 5'-ACGCCCTTATGAAAATGTGG-3'
***symRK (mutant gene)***
   Lore2 5' LTR out P 5'-GGAGCTCTGATACCAATGTTAGG-3'
   cac41.5F 5'-CGGCAATAGAGCGCTGGAGAGTTG-3'
***ccamk***
   LTsym15(60)fw 5'-TATGACACAGATAGATCAGGG-3'
   LTsym15(60)rev 5'-GAGAGCGGCTCAATGAATGT-3'
***nin***
   Resc fwd 5'-CTCAGAGCACGCTTCTTGGA-3'
   Resc rev 5'-ATCATGTGTGCAATCCATGATG-3'
***har1***
   #3fw2- 5'-CCTGAAATGCCTATTCGTTGAG-3'
   #3rev2 5'-CACAGCTTCTTCTGCATGCG-3'
***snf1***
   Ca2fwd 5'-TGGCTTGCATCCAAACGGC-3'
   Ca3-3rev 5'-ACTATTGTTGTCTCACTTTAGTG-3'
***snf2***
   C3fwd 5'-TGGGATAATTGGTTGCTTGACA-3'
   C3Brev 5'-TGACAATGTGAGTTCCAGCAG-3'

Transgenic hairy roots were then monitored for spontaneous nodulation in the absence of rhizobia.

**Table 3**

| Spontaneous nodulation in non-nodulating *Lotus* mutants transformed with the *snf2* gene. | |
|---|---|
| **Mutant*** | **% spontaneous nodulation** |
| *nfr1* | 43 (3/7) |
| *nfr5* | 41 (24/58) |
| *nfr1nfr5* | 28 (33/118) |
| *symrk* | 36 (31/87) |
| *ccamk* | 19 (18/94) |
| *nin* | 0 (0/112) |
| *sym35* | 0 (0/136) |

| | |
|---|---|
| * Hairy roots of transformed mutants were scored in the absence of *M*. *loti.* Roots of control mutant plants transformed with an empty vector did not develop spontaneous nodules. | |

Spontaneous nodulation observed in *nfr1-1, nfr5-2* and *nfr1-1 nfr5-2* Nod-factor receptor single and double mutants lacking the earliest electrophysiological responses to Nod-factor (Radutoiu *et al.,* (2003) *supra;* Madsen *et al.,* (2003) *supra),* demonstrates that *Lhk1* functions downstream of Nod-factor signal perception. The common pathway *symRK* mutants lacking Ca²⁺ spiking (Stracke et al., (2002) Nature, 417: 959; Niwa et al., (2001) Mol Plant Microbe Interact. 14: 848) and the *ccamk* mutants suggested to be unable to interpret Ca²⁺ spiking (Tirichine et al., (2006) Nature 441: 1153), also develop spontaneous root nodules in transgenic roots transformed with the *snf2* gene construct. In the *nin* and *sym35* mutants that are arrested before initiation of cell division induced through the common pathway, no spontaneous nodules were observed in transgenic roots showing that cytokinin signal perception acts upstream of cell division initiation, or operates in a parallel pathway (figure 5C). Further evidence for a central role of cytokinin and cytokinin perception in the Nod-factor induced signal transduction amplified through the common pathway comes from the additive effect of *snf1-1* and *snf2* mutations in double mutants. The *snf1-1* mutants synthesize a CCaMK protein impaired in autophosphorylation (Tirichine *et al.,* (2006) *supra)* and develop an average of seven spontaneous nodules. *snf2* mutants develop an average of three spontaneous nodules, while *snf1-1 snf2* double mutants exceed both and develop approximately seventeen spontaneous nodules. Conversion of cortical cells into nodule stem cells or the subsequent organ development seems therefore tightly controlled. This theory was tested by crossing the *snf2* allele into a hypernodulating *har1-1* genetic background (Krusell et. al., (2002) Nature 420: 422). In absence of rhizobia, homozygous *snf2 har1-1* double mutants developed an average of fourteen spontaneous nodules, while *snf2* mutants developed an average of three, and *har1-1* none (figure 9). These results indicate that only a few cells de-differentiate or that only few de-differentiated cells sustain cell divisions during the *snf2* nodule initiation process. The shoot controlled autoregulation of the root nodule number (Krusell et al., (2002) Nature 420: 422) is thus acting downstream of cytokinin signalling and cytokinin induced activation of root nodule founder cells (figure 5C). The data clearly demonstrate that cytokinin signalling is necessary and sufficient for the de-differentiation and cell proliferation leading to root nodule formation and that this developmental process can be triggered independently of cytokinin by expression of the dominant *snf2* allele. Surprisingly, the phenotype conferred by expression of the snf2 allele, expressed under the control of a regulated promoter (e.g. *Lhk1* gene promoter) is characterised by controlled organogenesis of root nodules, rather than uncontrolled, cytokinin-independent, cell proliferation.

### Example 10. Detection kit to distinguish snf2 mutant allele from wild type allele encoding LKH1 histidine kinase

A kit for the detection of the snf2 mutant allele useful for genotype screening purposes comprises at least:

Two alternative sets of dCAPS (derived cleaved amplified polymorphic sequence) primers are provided for detection of wild type versus mutation:

### Set one:

Snf2 Xmnl/Asp700 dCAPS fw (74)
5'-CACCAAAATTGCTTGGTTACCAGCAAGTTGACCGA
Snf2 Xmnl/Asp700 dCAPS rv(69) 5'-CCCTTCATGTGGCCCTTACCCAAC

The primers are suitable for use in a PCR test performed with genomic DNA as the template, where amplification is performed at 60°C with 30 seconds elongation time and 35-40 cycles. The 225 bp product is cut by Xmnl/Asp700 in the mutant, and not cut in wildtype. The cleaved PCR products can be identified following their separation on a 1% agarose gel.

### Set two:

The primers are suitable for use in a PCR test performed with genomic DNA as the template , where amplification is performed at 48°C with 30 seconds elongation time and 35-40 cycles. The 253 bp product is cut bu Rsal in the wildtype, but uncut in mutant.

The cleaved PCR products can be identified following their separation on a 1% agarose gel.

### Example 11. Amplification primers for cloning the snf2 cDNA coding sequence.

Primers to amplify the coding sequence from start to stop (cDNA) are:
- Snf2 cDNA fw: ATGGGTCTTGGGTTCAAGATGCA
- Snf2 cDNA rev: TCATGAGTCTGAAGCAGGCTTGG

PCR was performed with annealing at 58°C and 3 minute 20 seconds elongation time for 25 cycles with cDNA clone as template.

## Claims

1. A DNA molecule encoding a mutant histidine kinase polypeptide comprising an amino acid sequence selected from among:
a. SEQ ID NO: 6, 15 and 16, wherein the amino acid residue 266 [Phe] in SEQ ID NO: 6 and 266 [Xaa] in SEQ ID NO: 15 and residue 267 [Xaa] in SEQ ID NO: 16 is selected from among isoleucine, serine, threonine, valine, methionine, alanine, phenylalanine, tyrosine, tryptophan, arginine, lysine, glycine, histidine, aspartate, asparagine, glutamate, glutamine, proline and cysteine, and
b. a truncation of (a),
wherein said polypeptide is capable of inducing spontaneous nodule formation when expressed in a plant.

2. A mutant histidine kinase polypeptide encoded by the DNA molecule of claim 1, wherein said polypeptide comprises an amino acid sequence selected from among:
a. SEQ ID NO: 6, 15 and 16, wherein the amino acid residue 266 [Phe] in SEQ ID NO: 6 and residue 266 [Xaa] in SEQ ID NO: 15 and residue 267 [Xaa] in SEQ ID NO: 16 is selected from among isoleucine, serine, threonine, valine, methionine, alanine, phenylalanine, tyrosine, tryptophan, arginine, lysine, glycine, histidine, aspartate, asparagine, glutamate, glutamine, proline and cysteine, and
b. a truncation of (a),
wherein said polypeptide is capable of inducing spontaneous nodule formation when expressed in a plant.

3. A genetically modified plant **characterised by** having a nucleotide sequence encoding a mutant histidine kinase polypeptide comprising an amino acid sequence selected from among:
a. SEQ ID NO: 6, 15 and 16, wherein the amino acid residue 266 [Phe] in SEQ ID NO: 6 and residue 266 [Xaa] in SEQ ID NO: 15 and residue 267 [Xaa] in SEQ ID NO: 16 is selected from among isoleucine, serine, threonine, valine, methionine, alanine, phenylalanine, tyrosine, tryptophan, arginine, lysine, glycine, histidine, aspartate, asparagine, glutamate, glutamine, proline and cysteine, and
b. a truncation of (a),
wherein said polypeptide is capable of inducing spontaneous nodule formation when expressed in a plant and wherein said plant is capable of spontaneous nodule formation.

4. A genetically modified plant according to claim 3, wherein the mutant histidine kinase polypeptide is encoded by a nucleic acid molecule having a nucleic acid sequence selected from the group: SEQ ID NO: 4, 5 and 7, or the coding sequence thereof.

5. A genetically modified plant according to claim 3 or 4, further comprising a homologous or a heterologous promoter nucleotide sequence operably linked to the nucleotide sequence encoding the polypeptide.

6. A genetically modified plant according to claim 5, wherein said promoter is a regulated promoter.

7. A genetically modified plant according to any one of claims 3 - 6, wherein said plant is a monocotyledonous or a dicotyledonous plant.

8. A genetically modified plant according to claim 7, wherein said plant is selected from among rice, barley, maize, oats, rye, sorghum, wheat and *Poaceae* grass.

9. The genetically modified plant according to claim 3 or 4, obtainable by a process of DNA mutagenesis and selecting a plant capable of spontaneous nodule formation, **characterised by** having a nucleotide sequence encoding a mutant histidine kinase polypeptide comprising an amino acid sequence selected from among:
a. SEQ ID NO: 6, 15 and 16, wherein the amino acid residue 266 [Phe] in SEQ ID NO: 6 and residue 266 [Xaa] in SEQ ID NO: 15 and residue 267 [Xaa] in SEQ ID NO: 16 is selected from among isoleucine, serine, threonine, valine, methionine, alanine, phenylalanine, tyrosine, tryptophan, arginine, lysine, glycine, histidine, aspartate, asparagine, glutamate, glutamine, proline and cysteine, and
b. a truncation of (a), wherein said polypeptide is capable of inducing spontaneous nodule formation when expressed in a plant

10. A method of producing a genetically modified plant according to any one of claims 3 - 8, **characterised by** introducing into a plant by transformation a gene cassette comprising said nucleotide sequence encoding said polypeptide and selecting a transgenic plant and its progeny expressing said polypeptide.

11. The genetically modified plant according to any one of claims 3 - 8,
wherein the plant is transformed with and comprises a transgene having a nucleotide sequence encoding a mutant histidine kinase polypeptide comprising an amino acid sequence selected from among:
a. SEQ ID NO: 6, 15 and 16, wherein the amino acid residue 266 [Phe] in SEQ ID NO: 6 and residue 266 [Xaa] in SEQ ID NO: 15 and residue 267 [Xaa] in SEQ ID NO: 16 is selected from among isoleucine, serine, threonine, valine, methionine, alanine, phenylalanine, tyrosine, tryptophan, arginine, lysine, glycine, histidine, aspartate, asparagine, glutamate, glutamine, proline and cysteine, and
b. a truncation of (a),
wherein said polypeptide is capable of inducing spontaneous nodule formation when expressed in a plant.

12. Seed of the genetically modified plant according to any of claims 3 - 9, 11, **characterised by** having a nucleotide sequence encoding a mutant histidine kinase polypeptide comprising an amino acid sequence selected from among:
a. SEQ ID NO: 6, 15 and 16, wherein the amino acid residue 266 [Phe] in SEQ ID NO: 6 and residue 266 [Xaa] in SEQ ID NO: 15 and residue 267 [Xaa] in SEQ ID NO: 16 is selected from among isoleucine, serine, threonine, valine, methionine, alanine, phenylalanine, tyrosine, tryptophan, arginine, lysine, glycine, histidine, aspartate, asparagine, glutamate, glutamine, proline and cysteine, and
b. a truncation of (a),
wherein said polypeptide is capable of inducing spontaneous nodule formation when expressed in a plant.

13. A crop comprising a genetically modified plant according to any of claims 3 - 9, 11.

## Patentansprüche

1. DNA-Molekül, das ein mutantes Histidinkinase-Polypeptid codiert, umfassend eine Aminosäuresequenz, die aus Folgendem ausgewählt ist:
a. SEQ ID NO: 6, 15 und 16, wobei der Aminosäurerest 266 [Phe] in SEQ ID NO: 6 und 266 [Xaa] in SEQ ID NO: 15 und der Rest 267 [Xaa] in SEQ ID NO: 16 ausgewählt ist aus Isoleucin, Serin, Threonin, Valin, Methionin, Alanin, Phenylalanin, Tyrosin, Tryptophan, Arginin, Lysin, Glycin, Histidin, Aspartat, Asparagin, Glutamat, Glutamin, Prolin und Cystein, und
b. einer Verkürzung von (a),
wobei das Polypeptid dazu in der Lage ist, eine spontane Knöllchenbildung hervorzurufen, wenn es in einer Pflanze exprimiert ist.

2. Mutantes Histidinkinase-Polypeptid, codiert vom DNA-Molekül nach Anspruch 1, wobei das Polypeptid eine Aminosäuresequenz umfasst, die aus Folgendem ausgewählt ist:
a. SEQ ID NO: 6, 15 und 16, wobei der Aminosäurerest 266 [Phe] in SEQ ID NO: 6 und der Rest 266 [Xaa] in SEQ ID NO: 15 und der Rest 267 [Xaa] in SEQ ID NO: 16 ausgewählt ist aus Isoleucin, Serin, Threonin, Valin, Methionin, Alanin, Phenylalanin, Tyrosin, Tryptophan, Arginin, Lysin, Glycin, Histidin, Aspartat, Asparagin, Glutamat, Glutamin, Prolin und Cystein, und
b. einer Verkürzung von (a),
wobei das Polypeptid dazu in der Lage ist, eine spontane Knöllchenbildung hervorzurufen, wenn es in einer Pflanze exprimiert ist.

3. Genetisch modifizierte Pflanze, **dadurch gekennzeichnet, dass** sie eine Nukleotidsequenz aufweist, die ein mutantes Histidinkinase-Polypeptid codiert, umfassend eine Aminosäuresequenz, die aus Folgendem ausgewählt ist:
a. SEQ ID NO: 6, 15 und 16, wobei der Aminosäurerest 266 [Phe] in SEQ ID NO: 6 und der Rest 266 [Xaa] in SEQ ID NO: 15 und der Rest 267 [Xaa] in SEQ ID NO: 16 ausgewählt ist aus Isoleucin, Serin, Threonin, Valin, Methionin, Alanin, Phenylalanin, Tyrosin, Tryptophan, Arginin, Lysin, Glycin, Histidin, Aspartat, Asparagin, Glutamat, Glutamin, Prolin und Cystein, und
b. einer Verkürzung von (a),
wobei das Polypeptid dazu in der Lage ist, eine spontane Knöllchenbildung hervorzurufen, wenn es in einer Pflanze exprimiert ist, und wobei die Pflanze zu einer spontanen Knöllchenbildung in der Lage ist.

4. Genetisch modifizierte Pflanze nach Anspruch 3, wobei das mutante Histidinkinase-Polypeptid durch ein Nukleinsäuremolekül mit einer Nukleinsäuresequenz codiert ist, ausgewählt aus der Gruppe: SEQ ID NO: 4, 5 und 7 oder der Codiersequenz davon.

5. Genetisch modifizierte Pflanze nach Anspruch 3 oder 4, weiter umfassend eine homologe oder eine heterologe Promotornukleotidsequenz, die betriebsbereit mit der Nukleotidsequenz verbunden ist, die das Polypeptid codiert.

6. Genetisch modifizierte Pflanze nach Anspruch 5, wobei der Promotor ein regulierter Promotor ist.

7. Genetisch modifizierte Pflanze nach einem der Ansprüche 3 - 6, wobei die Pflanze eine monokotyle Pflanze oder eine dikotyle Pflanze ist.

8. Genetisch modifizierte Pflanze nach Anspruch 7, wobei die Pflanze ausgewählt ist aus Reis, Gerste, Mais, Hafer, Roggen, Sorghum, Weizen und *Poaceae-*Gras*.*

9. Genetisch modifizierte Pflanze nach Anspruch 3 oder 4, die durch einen Prozess der DNA-Mutagenese und die Auswahl einer Pflanze erhalten werden kann, die zu einer spontanen Knöllchenbildung in der Lage ist, **dadurch gekennzeichnet, dass** sie eine Nukleotidsequenz aufweist, die ein mutantes Histidinkinase-Poplypeptid codiert, umfassend eine Aminosäuresequenz, die aus Folgendem ausgewählt aus:
a. SEQ ID NO: 6, 15 und 16, wobei der Aminosäurerest 266 [Phe] in SEQ ID NO: 6 und der Rest 266 [Xaa] in SEQ ID NO: 15 und der Rest 267 [Xaa] in SEQ ID NO: 16 ausgewählt ist aus Isoleucin, Serin, Threonin, Valin, Methionin, Alanin, Phenylalanin, Tyrosin, Tryptophan, Arginin, Lysin, Glycin, Histidin, Aspartat, Asparagin, Glutamat, Glutamin, Prolin und Cystein, und
b. einer Verkürzung von (a),
wobei das Polypeptid dazu in der Lage ist, eine spontane Knöllchenbildung hervorzurufen, wenn es in einer Pflanze exprimiert ist.

10. Verfahren zur Herstellung einer genetisch modifizierten Pflanze nach einem der Ansprüche 3 - 8, **gekennzeichnet durch** die Einführung **durch** Transformation einer Genkassette in eine Pflanze, umfassend die Nukleotidsequenz, die das Polypeptid codiert, und die Auswahl einer transgenen Pflanze und ihrer Pflanzenlinien, die das Polypeptid exprimieren.

11. Genetisch modifizierte Pflanze nach einem der Ansprüche 3 - 8, wobei die Pflanze mit einem Transgen transformiert ist und dieses enthält, umfassend eine Nukleotidsequenz, die ein mutantes Histidinkinase-Polypeptid aufweist, umfassend eine Aminosäuresequenz, die aus Folgendem ausgewählt aus:
a. SEQ ID NO: 6, 15 und 16, wobei der Aminosäurerest 266 [Phe] in SEQ ID NO: 6 und der Rest 266 [Xaa] in SEQ ID NO: 15 und der Rest 267 [Xaa] in SEQ ID NO: 16 ausgewählt ist aus Isoleucin, Serin, Threonin, Valin, Methionin, Alanin, Phenylalanin, Tyrosin, Tryptophan, Arginin, Lysin, Glycin, Histidin, Aspartat, Asparagin, Glutamat, Glutamin, Prolin und Cystein, und
b. einer Verkürzung von (a),
wobei das Polypeptid dazu in der Lage ist, eine spontane Knöllchenbildung hervorzurufen, wenn es in einer Pflanze exprimiert ist.

12. Samen der genetisch modifizierten Pflanze nach einem der Ansprüche 3 - 9, 11, **dadurch gekennzeichnet, dass** sie eine Nukleotidsequenz aufweisen, die ein mutantes Histidinkinase-Polypeptid codiert, umfassend eine Aminosäuresequenz, die aus Folgendem ausgewählt ist:
a. SEQ ID NO: 6, 15 und 16, wobei der Aminosäurerest 266 [Phe] in SEQ ID NO: 6 und der Rest 266 [Xaa] in SEQ ID NO: 15 und der Rest 267 [Xaa] in SEQ ID NO: 16 ausgewählt ist aus Isoleucin, Serin, Threonin, Valin, Methionin, Alanin, Phenylalanin, Tyrosin, Tryptophan, Arginin, Lysin, Glycin, Histidin, Aspartat, Asparagin, Glutamat, Glutamin, Prolin und Cystein, und
b. einer Verkürzung von (a),
wobei das Polypeptid dazu in der Lage ist, eine spontane Knöllchenbildung hervorzurufen, wenn es in einer Pflanze exprimiert ist.

13. Feldfrucht, umfassend eine genetisch modifizierte Pflanze nach einem der Ansprüche 3-9,11.

## Revendications

1. Molécule d'ADN codant pour un polypeptide d'histidine kinase mutant, comprenant une séquence d'acides aminés choisie parmi :
a. les SEQ ID n° 6, 15 et 16, dans lesquelles le résidu d'acide aminé 266 [Phe] dans la SEQ ID n° 6 et le résidu 266 [Xaa] dans la SEQ ID n° 15 et le résidu 267 [Xaa] dans la SEQ ID n° 16 sont choisis parmi l'isoleucine, la sérine, la thréonine, la valine, la méthionine, l'alanine, la phénylalanine, la tyrosine, le tryptophane, l'arginine, la lysine, la glycine, l'histidine, l'aspartate, l'asparagine, le glutamate, la glutamine, la proline et la cystéine, et
b. une troncature des séquences décrites au point (a),
dans laquelle ledit polypeptide est en mesure d'induire une formation spontanée de nodules lorsqu'il est exprimé dans une plante.

2. Polypeptide d'histidine kinase mutant codé par la molécule d'ADN selon la revendication 1, dans lequel ledit polypeptide comprend une séquence d'acides aminés choisie parmi .
a. les SEQ ID n° 6, 15 et 16, dans lesquelles le résidu d'acide aminé 266 [Phe] dans la SEQ ID n° 6 et le résidu 266 [Xaa] dans la SEQ ID n° 15 et le résidu 267 [Xaa] dans la SEQ ID n° 16 sont choisis parmi l'isoleucine, la sérine, la thréonine, la valine, la méthionine, l'alanine, la phénylalanine, la tyrosine, le tryptophane, l'arginine, la lysine, la glycine, l'histidine, l'aspartate, l'asparagine, le glutamate, la glutamine, la proline et la cystéine, et
b. une troncature des séquences décrites au point (a),
dans lequel ledit polypeptide est en mesure d'induire une formation spontanée de nodules lorsqu'il est exprimé dans une plante.

3. Plante génétiquement modifiée, **caractérisée en ce qu'**elle possède une séquence nucléotidique codant pour un polypeptide d'histidine kinase mutant comprenant une séquence d'acides aminés choisie parmi :
a. les SEQ ID n° 6, 15 et 16, dans lesquelles le résidu d'acide aminé 266 [Phe] dans la SEQ ID n° 6 et le résidu 266 [Xaa] dans la SEQ ID n° 15 et le résidu 267 [Xaa] dans la SEQ ID n° 16 sont choisis parmi l'isoleucine, la sérine, la thréonine, la valine, la méthionine, l'alanine, la phénylalanine, la tyrosine, le tryptophane, l'arginine, la lysine, la glycine, l'histidine, l'aspartate, l'asparagine, le glutamate, la glutamine, la proline et la cystéine, et
b. une troncature des séquences décrites au point (a),
dans laquelle ledit polypeptide est en mesure d'induire la formation spontanée de nodules lorsqu'il est exprimé dans une plante et dans lequel ladite plante présente la capacité de former spontanément des nodules.

4. Plante génétiquement modifiée selon la revendication 3, dans laquelle le polypeptide d'histidine kinase mutant est codé par une molécule d'acide nucléique ayant une séquence d'acide nucléique choisie dans le groupe constitué des SEQ ID n° 4, 5 et 7 ou de la séquence codante de celui-ci.

5. Plante génétiquement modifiée selon la revendication 3 ou 4, comprenant en outre une séquence nucléotidique de promoteur homologue ou hétérologue reliée de manière fonctionnelle à la séquence nucléotidique codant pour le polypeptide.

6. Plante génétiquement modifiée selon la revendication 5, dans laquelle ledit promoteur est un promoteur régulé.

7. Plante génétiquement modifié selon l'une quelconque des revendications 3 à 6, dans laquelle ladite plante est une plante monocotylédone ou dicotylédone.

8. Plante génétiquement modifiée selon la revendication 7, dans laquelle ladite plante est choisie parmi le riz, l'orge, le maïs, l'avoine, le seigle, le sorgho, le blé et l'herbe de la famille des *Poaceae.*

9. Plante génétiquement modifiée selon la revendication 3 ou 4, pouvant être obtenue par un procédé de mutagenèse d'ADN et de sélection d'une plante capable de former des nodules de manière spontanée, **caractérisée en ce qu'**elle présente une séquence nucléotidique codant pour un polypeptide d'histidine kinase mutant comprenant une séquence d'acides aminés choisie parmi :
a. les SEQ ID n° 6, 15 et 16, dans lesquelles le résidu d'acide aminé 266 [Phe] dans la SEQ ID n° 6 et le résidu 266 [Xaa] dans la SEQ ID n° 15 et le résidu 267 [Xaa] dans la SEQ ID n° 16 sont choisis parmi l'isoleucine, la sérine, la thréonine, la valine, la méthionine, l'alanine, la phénylalanine, la tyrosine, le tryptophane, l'arginine, la lysine, la glycine, l'histidine, l'aspartate, l'asparagine, le glutamate, la glutamine, la proline et la cystéine, et
b. une troncature des séquences décrites au point (a), dans laquelle ledit polypeptide est en mesure d'induire la formation spontanée de nodules lorsqu'il est exprimé dans une plante.

10. Procédé de production d'une plante génétiquement modifiée selon l'une quelconque des revendications 3 à 8, **caractérisé en ce que** l'on introduit dans une plante, par transformation, une cassette de gène comprenant ladite séquence nucléotidique codant pour ledit polypeptide et **en ce que** l'on sélectionne une plante transgénique et sa descendance exprimant ledit polypeptide.

11. Plante génétiquement modifiée selon l'une quelconque des revendications 3 à 8,
dans laquelle la plante fait l'objet d'une transformation et comprend un transgène ayant une séquence nucléotidique codant pour un polypeptide d'histidine kinase mutant comprenant une séquence d'acides aminés choisie parmi :
a. les SEQ ID n° 6, 15 et 16, dans lesquelles le résidu d'acide aminé 266 [Phe] dans la SEQ ID n° 6 et le résidu 266 [Xaa] dans la SEQ ID n° 15 et le résidu 267 [Xaa] dans la SEQ ID n° 16 sont choisis parmi l'isoleucine, la sérine, la thréonine, la valine, la méthionine, l'alanine, la phénylalanine, la tyrosine, le tryptophane, l'arginine, la lysine, la glycine, l'histidine, l'aspartate, l'asparagine, le glutamate, la glutamine, la proline et la cystéine, et
b. une troncature des séquences décrites au point (a),
dans laquelle ledit polypeptide est capable d'induire la formation spontanée de nodules lorsqu'il est exprimé dans une plante.

12. Semence de la plante génétiquement modifiée selon l'une quelconque des revendications 3 à 9, et 11, **caractérisée en ce qu'**elle présente une séquence nucléotidique codant pour un polypeptide d'histidine kinase mutant comprenant une séquence d'acides aminés choisie parmi :
a. les SEQ ID n° 6, 15 et 16, dans lesquelles le résidu d'acide aminé 266 [Phe] dans la SEQ ID n° 6 et le résidu 266 [Xaa] dans la SEQ ID n° 15 et le résidu 267 [Xaa] dans la SEQ ID n° 16 sont choisis parmi l'isoleucine, la sérine, la thréonine, la valine, la méthionine, l'alanine, la phénylalanine, la tyrosine, le tryptophane, l'arginine, la lysine, la glycine, l'histidine, l'aspartate, l'asparagine, le glutamate, la glutamine, la proline et la cystéine, et
b. une troncature des séquences décrites au point (a),
dans laquelle ledit polypeptide est capable d'induire la formation spontanée de nodules lorsqu'il est exprimé dans une plante.

13. Culture comprenant une plante génétiquement modifiée selon l'une quelconque des revendications 3 à 9 et 11 .


### SEQUENCE LISTING

<110> Aarhus Universitet, Ledelsessekretariatet
   Tirichine, Leila
<120> A mutant histidine kinase that confers spontaneous nodulation in plants
<130> P80600597DK00
<160> 20
<170> PatentIn version 3.2
<210> 1
   <211> 12738
   <212> DNA
   <213> Lotus japonicus
<220>
   <221> promoter
   <222> (1)..(2359)
   <223> promoter + 5' UTR of transcript
<220>
   <221> exon
   <222> (2360)..(2704)
   <223> start of CDS in 1st exon
<220>
   <221> Intron
   <222> (2705)..(3024)
<220>
   <221> exon
   <222> (3025)..(3258)
<220>
   <221> Intron
   <222> (3259)..(3390)
<220>
   <221> exon
   <222> (3391)..(3560)
<220>
   <221> Intron
   <222> (3561)..(3834)
<220>
   <221> exon
   <222> (3835)..(4053)
<220>
   <221> Intron
   <222> (4054)..(4145)
<220>
   <221> exon
   <222> (4146)..(4332)
<220>
   <221> Intron
   <222> (4333)..(4420)
<220>
   <221> exon
   <222> (4421)..(4472)
<220>
   <221> Intron
   <222> (4473)..(4586)
<220>
   <221> exon
   <222> (4587)..(4816)
<220>
   <221> Intron
   <222> (4817)..(5037)
<220>
   <221> exon
   <222> (5038)..(5136)
<220>
   <221> Intron
   <222> (5137)..(5308)
<220>
   <221> exon
   <222> (5309)..(6024)
<220>
   <221> Intron
   <222> (6025)..(7042)
<220>
   <221> exon
   <222> (7043)..(7513)
<220>
   <221> Intron
   <222> (7514)..(8306)
<220>
   <221> exon
   <222> (8307)..(8564)
<220>
   <221> Intron
   <222> (8565)..(12738)
   <223> 3'UTR + 3 prime of transcript
<400> 1
   cccgggattg ttcatatccc catgtgtgtg tttggtttgg acacaagcaa gcttgccaca     60
   gtggtgttta caactcattc tgaattatat atatatatat atatatataa ctaatctcta    120
   tcaataattt agttagtttt atgggataat atgattcccg tcaaattagc ttgtaggtta    180
   aaaatccata tttatataat aaattagact tatctaaatc acttaagtaa atatgatgta 240
   aaatcacaat agcttaggta gtttaacaat gttttatcat gaatttaaaa aaaattagtt 300
   gtttcaaaat attattggac caaataagca aagaggagct aatccaatta taaacagcac 360
   atctttataa ttgttttcta ctcgatctaa tattggttcc ttctttcctt gagaaaaaaa 420
   gggttaaaaa tgcgatttgt tttgtaaaat taatttgtga cattaacaag agaaaactct 480
   acttttttaa tttttaagaa gttacttaaa tatagaaaga aagaagttac ttaaatatga 540
   aacttgtcta accaaagtca accgatcaaa gcgtttggtt agcaaagaga acttaaaaaa 600
   ataactaaat taactgtttg aacagtgatg taacagagaa atagaatggc ttacatatgg 660
   attatggagt taatggaagg cacactagtt ttagttcacg gcaacaagac agaattatcc 720
   tcctatattt taggaggttt tcttatttat ttttaatgta agacgtgtat ccttcttttg 780
   aggtaataat gttcgagctt gaaacattca cattatagtg tgactaattg cttaatatga 840
   atcaagcatg taccacttga aacactcact catcgtttat aatataatta ctttatagta 900
   ctataaagta tgtaattatt ttctcctttt taagtatctc aatcattttt ttgacccaaa 960
   tattatttta gagaagaaac atgtcttaag accaatacat caacatatat ggagagtagt 1020
   ttgacaacct ctcatgagct ttctaaagcc aaacaaatgt actcacataa aaaaattaaa 1080
   aatttacagc aagtgagtta taaagagtga ttaataaatg ttgaataacg atggagggta 1140
   gttatgaaat tccataaata aagcaaagga tgtttatggc aattgacatg ggaataagat 1200
   ccgcgcgttg tcgccgtgtc ataatcgctc agatttgtga tagcgagaga tttccattct 1260
   tttcctcttt tttcgaacga acgaacgaac gaacacagta gcagctgtat taggattcag 1320
   attgcatgat acattgatat tgatattgat attgatattg atattgttgt gggtcaagtc 1380
   tctactctac tatccaaagt aagcatatat atagagagag agagagagct tgggactagg 1440
   gaggatatta gcttatgtga ctgtgaagtt gaagggagac aagagcgtat ctggcaaaat 1500
   cctcaaaata aaatactagt actagtacag agaaaagaga ctaagagaga gagtgctgct 1560
   gcacatcaag acccattgtg atttgtgatt tgtgatttgt gatttgtgat ttgtgagtga 1620
   gttcattgta caggtattat tgtttgttgt ttctctcctc aaccaccctc taaagtctaa 1680
   tctaactcat tgggctctgt gcttagctgg ttgtgttttg tgtatggtga attagggggt 1740
   caatctctgg ttttcatcat tattatatta tatatgggaa taccgtgctc tcttctttgt 1800
   ccttcaccaa aactagtttc acgcctacac aacatgatta gagcctcttc atttttttaa 1860
   tctcatcctt taagtgtatt tctatttcta ttggctattg ggacaagggg aaggtggtgc 1920
   ttcttaggaa cttgagctgt tttccatctt ttgagaccca tgctttgtct ctctcatttt 1980
   taattctggg tctctttctt ctcttgtcct gattttttaa atgtgcttct tttttgcttc 2040
   ttacaaccac cctctaaacc attcatcatg cttggtttgc ttttgcttct cctttcacag   2100
   gtttcaatca cgcaaaacaa tgctgcaatg atgctgtact tggagtttct tctgtgaccc   2160
   cttttttcct tccttcaaca atcaacccac cagagaaaag tgtctcagat tttgagacta   2220
   ctttcaactt tcaaaacaaa gtggatggga tcttcatctt atataaccac acatcaatca   2280
   tttgtgctac ttctccaatt ttctttagag atgaaatgaa gagctaagca gacaagacaa   2340
   gtttatttgt ttgttgctg atg ggt ctt ggg ttc aag atg cag cag agc cac    2392
                        Met Gly Leu Gly Phe Lys Met Gln Gln Ser His
                        1               5                   10
   cac cct gtg gct ttg aag tta cat gag caa gct ggg agc cag aga aag     2440
   His Pro Val Ala Leu Lys Leu His Glu Gln Ala Gly Ser Gln Arg Lys
               15                  20                  25
   ttc act ttc att cag aac ttc aga aac tgg ttt cta ccc ctt ctg ttt     2488
   Phe Thr Phe Ile Gln Asn Phe Arg Asn Trp Phe Leu Pro Leu Leu Phe
           30                  35                  40
   gta tgg ttc att gtt atg gct gca ttt ggt gcc tgc atc tac cat aaa     2536
   Val Trp Phe Ile Val Met Ala Ala Phe Gly Ala Cys Ile Tyr His Lys
       45                  50                  55
   atg gat gct gaa act aaa gtc aga agg aaa gag gtg ctg ggt agc ctc     2584
   Met Asp Ala Glu Thr Lys Val Arg Arg Lys Glu Val Leu Gly Ser Leu
   60                  65                  70                  75
   tgt gat caa agg gct aga atg cta caa gac caa ttc agt gtc agt gtc     2632
   Cys Asp Gln Arg Ala Arg Met Leu Gln Asp Gln Phe Ser Val Ser Val
                   80                  85                  90
   aac cat gtc cat gcc ctt gcc atc ctt gtt tca acc ttc cat tac tac     2680
   Asn His Val His Ala Leu Ala Ile Leu Val Ser Thr Phe His Tyr Tyr
               95                  100                 105
   aga aat act tca gcc att gac cag gtttgtgctt gattttcctt tccttgaagc    2734
   Arg Asn Thr Ser Ala Ile Asp Gln
           110                 115
   attttttagt tggaggctca atttcttttt ctgatttgat tctggcctta aaaattagaa   2794
   tcaattgtag aaggatttcc aaacatgccc attttggaaa ttggtgcatc tgatagtatc   2854
   atgtttagat cagtttcttt ttcctcagaa ttgattttgg gcttaaaatc aattgtggaa   2914
   ggatattcat tagtaatttg gatattgttg catcatatgg ttctatctag ttacatcatt   2974
   tttttccact ctgattgcat gtatctttct cctgttcttt tccctatcag gaa acc      3030
                                                          Glu Thr
   ttt gca gaa tac acg gcc agg aca gca ttt gaa cgg cca tta atg agt     3078
   Phe Ala Glu Tyr Thr Ala Arg Thr Ala Phe Glu Arg Pro Leu Met Ser
           120                 125                 130
   ggg gtg gcc tat gca cag aga gtg gtt cac tca gag aga gaa aga ttt     3126
   Gly Val Ala Tyr Ala Gln Arg Val Val His Ser Glu Arg Glu Arg Phe
       135                 140                 145
   gag aag caa cat ggg tgg gtt ata aag aca atg gaa aga gtg cct tca      3174
   Glu Lys Gln His Gly Trp Val Ile Lys Thr Met Glu Arg Val Pro Ser
   150                 155                 160                 165
   ggg gtt agg gat gag tat gca gca gtg ata ttt gca cag gaa act gtc      3222
   Gly Val Arg Asp Glu Tyr Ala Ala Val Ile Phe Ala Gln Glu Thr Val
                   170                 175                 180
   tct tac ctt gaa tct att gat atg atg tct ggg gag gtaaatgtca           3268
   Ser Tyr Leu Glu Ser Ile Asp Met Met Ser Gly Glu
               185                 190
   acacttgtga attaattgta aaactcagaa gctactcaga gaagctcttc cccagaattg   3328
   gttctgcctt tagaataaat tgtacatgga tttgaccaca ttttctcatt tgcatgatgc   3388
   ag gag gac cga gag aac att ttg agg gct aga gcc act ggg aaa gct       3435
      Glu Asp Arg Glu Asn Ile Leu Arg Ala Arg Ala Thr Gly Lys Ala
          195                 200                 205
   gtt ctg act agc cct ttc aga ctg ctg gat tct cat cat ctt ggc gtg      3483
   Val Leu Thr Ser Pro Phe Arg Leu Leu Asp Ser His His Leu Gly Val
       210                 215                 220
   gtt cta aca ttt cct gtt tat aaa tct aag ctc cct cca gag cca acg      3531
   Val Leu Thr Phe Pro Val Tyr Lys Ser Lys Leu Pro Pro Glu Pro Thr
   225                 230                 235                 240
   acg gaa gag gtc att aaa gcc ata gca gg  gtatgtcctc atttcacttt        3580
   Thr Glu Glu Val Ile Lys Ala Ile Ala Gly
                   245
   tcttgccaaa accagacttc tatttggttg tgtttccgta ggctatgact gatatgtagt   3640
   ttcaactcag ttagactata atataaaccc ttcatgtggc ccttacccaa cagcttaagc   3700
   ttttgggata attggttgct tgacaaactc cttccgtaga aaacttggtt agctttggtt   3760
   ctatgtgggc tttatgtttt ccctgagctt atgtaatagc atgatgtgtt taatgtactt   3820
   tttaatggaa acag a tat att gga gga tcc ttt gat gtt gag tcc ctt gtg   3871
                     Tyr Ile Gly Gly Ser Phe Asp Val Glu Ser Leu Val
                                         255                 260
   gag aat tta ctt ggt caa ctt gct ggt aac caa gca att ttg gtg aag      3919
   Glu Asn Leu Leu Gly Gln Leu Ala Gly Asn Gln Ala Ile Leu Val Lys
           265                 270                 275
   gta tat gat ata aca aac tct agc gac ccc cta atc atg tat ggc agc      3967
   Val Tyr Asp Ile Thr Asn Ser Ser Asp Pro Leu Ile Met Tyr Gly Ser
       280                 285                 290
   caa tat gaa gag ggt gat atg tct ctt gtc cat gaa agt aag ctt gat      4015
   Gln Tyr Glu Glu Gly Asp Met Ser Leu Val His Glu Ser Lys Leu Asp
   295                 300                 305                 310
   ttt gga gat cca tac agg aaa cat cac atg atc tgt ag  gtgggtgctt       4063
   Phe Gly Asp Pro Tyr Arg Lys His His Met Ile Cys Arg
                   315                 320
   ctagttattg ttgtagtaca ttttctgagt ttcagtggtt tatcaattat cagcagattc   4123
   ttatgatcaa tttttttaac ag a tat cac caa cag gca cca aca aat tgg      4173
                              Tyr His Gln Gln Ala Pro Thr Asn Trp
                                  325                 330
   ata gca tat acc acg gca ttc cta ttc ttt gtg att ctt tgt tta gtg     4221
   Ile Ala Tyr Thr Thr Ala Phe Leu Phe Phe Val Ile Leu Cys Leu Val
           335                 340                 345
   ggt tac att tta tat gct gct gga act cac att gtc aag gta gaa gat     4269
   Gly Tyr Ile Leu Tyr Ala Ala Gly Thr His Ile Val Lys Val Glu Asp
       350                 355                 360
   gat tac aat gca atg cag gat tta aaa gtc aaa gca gaa gca gct gat     4317
   Asp Tyr Asn Ala Met Gln Asp Leu Lys Val Lys Ala Glu Ala Ala Asp
   365                 370                 375                 380
   att gcc aag tca cag gtacttttca tgacatgtta gcactgttcg ttatttcctt     4372
   Ile Ala Lys Ser Gln
                   385
   gaattgcata ctgatcacta gaaactgaaa atttgttatt aatgtcag ttt cta gct    4429
                                                        Phe Leu Ala
   acc gtc tct cat gaa att aga act ccc atg aat gga att tta g           4472
   Thr Val Ser His Glu Ile Arg Thr Pro Met Asn Gly Ile Leu
       390                 395                 400
   gtaactttaa gattctctct cgttctcttt ccactgaaaa gcaacacatg ctttcattcc   4532
   atacctgata ctttcccatt agtgatgcta tcgttaaact ccttgtcact gtag ga      4588
                                                               Gly
   atg ctt ggt ctg ctt tta cgc aca gaa ttg agt tca aca caa aga gac     4636
   Met Leu Gly Leu Leu Leu Arg Thr Glu Leu Ser Ser Thr Gln Arg Asp
       405                 410                 415
   tat gct cag act gct caa gca tgt ggg aag gca cta ata gca tta ata     4684
   Tyr Ala Gln Thr Ala Gln Ala Cys Gly Lys Ala Leu Ile Ala Leu Ile
   420                 425                 430                 435
   aat gag gtg ctt gac cga gct aaa att gaa gca ggc aaa tta gag cta     4732
   Asn Glu Val Leu Asp Arg Ala Lys Ile Glu Ala Gly Lys Leu Glu Leu
                   440                 445                 450
   gaa gca gtt cca ttt gac ctt cgt tcc ata ctt gac gat gtc ctt tct     4780
   Glu Ala Val Pro Phe Asp Leu Arg Ser Ile Leu Asp Asp Val Leu Ser
               455                 460                 465
   ctt ttt tct gag aag tca aga cac aaa ggc tta gag gtacgtttag          4826
   Leu Phe Ser Glu Lys Ser Arg His Lys Gly Leu Glu
           470                 475
   tcattgctaa atctgttgtg aagtcctgta caagtggcgt aatttcatag tcctatttcc   4886
   ttttcttcaa ttgatgatct tatttcatat tcctctcgtg tttctctctt tctatgttgc   4946
   catgtcgttg ggcggtgatg ttcctactct gatcccaaat tcctgatgtg caattattcc   5006
   gacttggact tcaatggttt gggaagtata g ctg gca gtg ttt gtt tct gac      5058
                                      Leu Ala Val Phe Val Ser Asp
                                      480                 485
   aaa gtt ccg gat ata gtt atg ggc gat cct ggg cga ttc aga caa ata     5106
   Lys Val Pro Asp Ile Val Met Gly Asp Pro Gly Arg Phe Arg Gln Ile
               490                 495                 500
   gtg aca aat ctt gtt gga aac tct gtt aag gttagtggaa ttttcaaact      5156
   Val Thr Asn Leu Val Gly Asn Ser Val Lys
           505                 510
   ttatttgcct aatgttgtgt gcaagttgtg tgttggaaat gcgtcctttt aacgttataa   5216
   aatcgtacaa gttcgtattc tccattgtat acaataactt attagcaaag tacttgttga   5276
   tatcattact gattaacttt aatatcttgc ag ttc act gag cga ggt cat ata     5329
                                       Phe Thr Glu Arg Gly His Ile
                                                   515
   ttt gtt aaa gtc cat tta gct gaa aaa aga cag tgc aca atg aat gga     5377
   Phe Val Lys Val His Leu Ala Glu Lys Arg Gln Cys Thr Met Asn Gly
   520                 525                 530                 535
   aaa tgt gag act ttt cta aat gga ggc tgt gat gat gtt ttg cat gta     5425
   Lys Cys Glu Thr Phe Leu Asn Gly Gly Cys Asp Asp Val Leu His Val
                   540                 545                 550
   tct ggc agt tat aat ttg aaa acc ctt agt gga tat gaa gcc gct gat     5473
   Ser Gly Ser Tyr Asn Leu Lys Thr Leu Ser Gly Tyr Glu Ala Ala Asp
               555                 560                 565
   gaa cgg aac agc tgg gat aat ttt aag cat cat att gct gac gaa gaa     5521
   Glu Arg Asn Ser Trp Asp Asn Phe Lys His His Ile Ala Asp Glu Glu
           570                 575                 580
   ttt ttc ttt gat gct tcg gtt aaa aag ttg gcc tct agt gaa tct tat     5569
   Phe Phe Phe Asp Ala Ser Val Lys Lys Leu Ala Ser Ser Glu Ser Tyr
       585                 590                 595
   gag caa gtc acc ttg atg gtc agc gtg gag gac act gga att ggg att     5617
   Glu Gln Val Thr Leu Met Val Ser Val Glu Asp Thr Gly Ile Gly Ile
   600                 605                 610                 615
   tct ttc tct gcc caa gat agt att ttc atg cct ttt gtg cag gct gac     5665
   Ser Phe Ser Ala Gln Asp Ser Ile Phe Met Pro Phe Val Gln Ala Asp
                   620                 625                 630
   agc tca acc tct cga aac tat ggg ggt acc ggg atc ggc ttg agt atc     5713
   Ser Ser Thr Ser Arg Asn Tyr Gly Gly Thr Gly Ile Gly Leu Ser Ile
               635                 640                 645
   agt aag tgc ttg gtt gaa ctg atg ggc ggt cag ata aac ttc ata agc     5761
   Ser Lys Cys Leu Val Glu Leu Met Gly Gly Gln Ile Asn Phe Ile Ser
           650                 655                 660
   cga ccc cag gtc ggg agc acg ttt tca ttc act gca gat ttc gga aca     5809
   Arg Pro Gln Val Gly Ser Thr Phe Ser Phe Thr Ala Asp Phe Gly Thr
       665                 670                 675
   ttt aag aaa aac tca aca act gac atg aag aaa ctt aac ttt gaa gat      5857
   Phe Lys Lys Asn Ser Thr Thr Asp Met Lys Lys Leu Asn Phe Glu Asp
   680                 685                 690                 695
   cta cct tct agt ttt aga ggt ctt aaa gcc att gtg gtt gat gga aaa      5905
   Leu Pro Ser Ser Phe Arg Gly Leu Lys Ala Ile Val Val Asp Gly Lys
                   700                 705                 710
   cct gtt aga gct gca gtg act aga tac cat ttg aag aga cta ggg ata      5953
   Pro Val Arg Ala Ala Val Thr Arg Tyr His Leu Lys Arg Leu Gly Ile
               715                 720                 725
   caa gct aaa gtt gca att agc atc aat aag gct gtt tct tta tgt ggg      6001
   Gln Ala Lys Val Ala Ile Ser Ile Asn Lys Ala Val Ser Leu Cys Gly
           730                 735                 740
   aaa aat ggt tct ttg acc tcg gc  gtaagtcttt aattaacctt tttggtttca      6054
   Lys Asn Gly Ser Leu Thr Ser Ala
       745                 750
   attatgtaga aatgtattga atgttatgat aatcagtagc attatcaact tttagtaatt     6114
   gttcttaaca tatgctaata gtcatatctt tctataatac tacaatactg tagccatata     6174
   atatctttcc tgtattggag tgagttttca aatgtttttc tgtgatattt tggaagttat     6234
   cttcagtttg agaactcatt tgtcattttt gcattttgtt attggatatt tggatggatc     6294
   tttacaaagg atgtgtggat tttgacttgt tatacacatt tcttctccat tttatattgt     6354
   ttgtgttatt ctttttactc ataaagaaat ttagaaactg cattgactgg ttctttttaa     6414
   ttacttacag atattgacat tgatattttt tgtaaatgct gtcttgacat ggtttaatta     6474
   cttacagact aggtttttct ttcctttcct aacatgcata tccatttact tttttgacca     6534
   accaacatcc tcatgagtca tgacatgttg atgatttata tggttgactt gagactattt     6594
   agacattaaa taaccgcaaa ttccatgttg tttgtgtgtt tggttccctg ttgggtaatc     6654
   tcagaatcaa ttatgataga gtaaaatcaa ttttggatga gatgtgtggg tgtcattttg     6714
   taaacctaaa cccaaaatca attctgctat aagctagaga gagtagttga acataatcaa     6774
   ttgtgagatt ttgcaagtgg attgcacaac attgccctat gaaaatcact ttttgttcac     6834
   aaaatttatc taaacataca taacttcatt ttcaaccttt actataatca attttacaat     6894
   aattaatttt acccaaaatc aattgtgaca atgagtttcc aaacacacac ttaaagacta     6954
   ccatttgcag aaaatatgtg atagaagact tatgtttatg tagtgtgttt cagttcattc     7014
   actgatttaa actactcgga ttttgcag a tta ttt cag cct gat att att ttt     7067
                                    Leu Phe Gln Pro Asp Ile Ile Phe
                                                    755
   gtt gag aag gac tct tgg gtt tct gga gag gat ggt ggt atc ttc aat      7115
   Val Glu Lys Asp Ser Trp Val Ser Gly Glu Asp Gly Gly Ile Phe Asn
   760                 765                 770                 775
   gcg ttt aag atg cct caa atg atc ctt ctt gca acc aat atc tgt aac      7163
   Ala Phe Lys Met Pro Gln Met Ile Leu Leu Ala Thr Asn Ile Cys Asn
                   780                 785                 790
   gct gaa ttt gat aaa gcc aaa gct gca ggt ttc agt gat aca gtg atc      7211
   Ala Glu Phe Asp Lys Ala Lys Ala Ala Gly Phe Ser Asp Thr Val Ile
               795                 800                 805
   atg aag cca ctg aga gct agt atg ctg gct gct tgt ctt cag caa gtt      7259
   Met Lys Pro Leu Arg Ala Ser Met Leu Ala Ala Cys Leu Gln Gln Val
           810                 815                 820
   ttc ggg act ggc aag acg agg cag ttt ggg aaa gac atg tcg aat ggt      7307
   Phe Gly Thr Gly Lys Thr Arg Gln Phe Gly Lys Asp Met Ser Asn Gly
       825                 830                 835
   tct tca gta cga agc ctt ctt tgc gga aag aaa atc tta gtg gtt gat      7355
   Ser Ser Val Arg Ser Leu Leu Cys Gly Lys Lys Ile Leu Val Val Asp
   840                 845                 850                 855
   gat aat ttg gtg aac cga agg gtc gcc gcc ggc gcg ttg aaa aac ttt      7403
   Asp Asn Leu Val Asn Arg Arg Val Ala Ala Gly Ala Leu Lys Asn Phe
                   860                 865                 870
   gga gct gat gtc aaa tgt gca gca agt ggc aaa gct gct ctt gaa atg      7451
   Gly Ala Asp Val Lys Cys Ala Ala Ser Gly Lys Ala Ala Leu Glu Met
               875                 880                 885
   ctt caa tat cct cac gat ttc gat gct tgc ttc atg gat att caa atg      7499
   Leu Gln Tyr Pro His Asp Phe Asp Ala Cys Phe Met Asp Ile Gln Met
           890                 895                 900
   cca gaa atg gat gg  gtatgcttac tggcactgac taatacatgt tttttgccaa      7553
   Pro Glu Met Asp Gly
       905
   cttaatatat tactctttca atattcgttg tgttattaga agatcatata gattaattta    7613
   taaattttct tttagcaaaa ccttatcaat taagtgtgta gaaaagtcag tctcacatta    7673
   tggtcaaata agtgttaggg caagcttcac ctcaaagcta gctatttggg tagatttagg    7733
   cctaacccga attctaagat ggtatcagag tctatcctag atctttttat tggaaaccac    7793
   ccgtatatga gcaactcgta gatattcatt cttgaaagtt gcacgctcca tatgtccatt    7853
   cctaggtgcg agagagaagt ctcactttga ctagagatat gattaaaaaa atatttataa    7913
   agggttgagc aatcctcacc tcagagctaa gcttttgggg taaagttagg cctaactcga    7973
   actctaataa agtgtttagc tggtgtgtca actgtcaata tgaaatcttt tgcaatttac    8033
   tatgcattca cttacctact ttattgaagc ttattgacaa tttgtgcaga agcatcatta    8093
   attaggaaca tgttagctat acaagttatg atgtttttgt atagcatatc atgttccaac    8153
   cttccaataa caaaatatgt ggttcaagtg tgagaatata taggttaaac aataaagtat    8213
   tgagttaaca gaaatctaaa cacacgctgt cactagctct tcatattgag acatgcatgg    8273
   gatttgacaa aacatctgaa taaatatttg cag g ttt gag gcc act cgt cga       8325
                                   Phe Glu Ala Thr Arg Arg
                                       910
   att cgg atg atg gaa aga gag gca agt gag cag ctg aaa agt gaa tct     8373
   Ile Arg Met Met Glu Arg Glu Ala Ser Glu Gln Leu Lys Ser Glu Ser
   915                 920                 925                 930
   ggt gaa gaa aat ggt aag aaa agt gag ttc cac atg cct ata ttg gcc     8421
   Gly Glu Glu Asn Gly Lys Lys Ser Glu Phe His Met Pro Ile Leu Ala
                   935                 940                 945
   atg aca gct gat gta atc cat gct aca tat gat aag tgc tta aat tgt     8469
   Met Thr Ala Asp Val Ile His Ala Thr Tyr Asp Lys Cys Leu Asn Cys
               950                 955                 960
   ggg atg gat gga tac gtc tca aag cct ttt gaa gaa gag aat ctc tat     8517
   Gly Met Asp Gly Tyr Val Ser Lys Pro Phe Glu Glu Glu Asn Leu Tyr
           965                 970                 975
   caa gca gtt gca aag ttc ttc aag tcc aag cct gct tca gac tca tg      8564
   Gln Ala Val Ala Lys Phe Phe Lys Ser Lys Pro Ala Ser Asp Ser
       980                 985                 990
   acactgctta ttctgcagaa caggtcaacc aacttttgat tgagaaacat ttagtgttag   8624
   catgtttgga tcaacttctc ccagcatcaa ttctgaaact cagaagctac tcatagaagc   8684
   ttctagccag aattgatttt ggcttcagaa gctactaatg gttttatgta gagagcaaaa   8744
   gttggtttcc aagatatgcg aggatccatg atgatacaca cgtctgaagt gatcatttct   8804
   aaccagttga agtttcactc gacgtgattt gaatccaagt aaatgcatac cacataatta   8864
   tccatcccca tcttgtgtac agattctccc aagggataag aaaatttatg taaattcaat   8924
   tttttctttt tgcatctcaa tacttccctg ttagaacttt ttccctatga ttatttccac   8984
   ttttcatttt caattatatt ttttgtaaaa ttggtctcca tcctataagg tttgcttagt   9044
   tttttttaat tcagatgaga aggttggtgc ttataatgtg tatacctttt tagcagtact   9104
   tgtgatataa atatttgtct tatttttaga acttaggaga taataagatc gtaggagtaa   9164
   atagcatttt tggagaatga tatcataatt ataatcatct acattctgca gttataaaaa   9224
   aaatcaatat taatgcatag caagtagctt taagaagtat gatctattta agtattgaat   9284
   tttttttcta aactaattac aatgtattat tgctgggata tgcgcttttt actcgataga   9344
   tatcatgttt ggattagttt ttcttagaat ccattttgga accgaaaaaa ttatcacata   9404
   atttattctt caaagttgat tttggcaatc aaggtaaaca ctcataaaag ctaaagggag   9464
   ttgtttctta ttattgtgat tttagctcat tgtggttttt caccgtgtat ataaacatgc   9524
   atttgatgtg acgttgactt gtgaagcacg tggctatcca ccagcaatct caagtaacac   9584
   ttgtcagcac tatgttgcta ttataagtga caaggcagtg accacacccc tctatatatg   9644
   cataaacttt tttattaaaa gtaacagaat caaaagggaa gttttctaca aattaattaa   9704
   tggaaaatta tcagaaccag tatggagctg ttcctgctag cagggctccg gttcagagcc 9764
   agagtgaaat attggaaacc accaatgttg gcggtgctac acaagtcata agaaatgatt 9824
   atgatggaag aaccactgct gcagatgcta ctgttgttgg tgagagcgat ctgcagcatc 9884
   accacaagaa aggaatatta gagaagatca aggagaagct tcctggaaca caccaccatc 9944
   aagatcacaa atagctagct agctttgttt agtttatttt gcttctatca ttataaatgt 10004
   aataataata gtatgtgtgt tgctacatgc atgtgtatgt atcgtttagt cagctagttt 10064
   gttaataact tgctttcaat tttgcttcct tacaagccta tttatgcaat gtatttggtc 10124
   atttcactat tggtttctaa tcaatctaaa ttagtacatg gtcctcttcc ttttagtgcc 10184
   ttttaattaa agagtaaatg ttgaatattg tttttagtca ttctatttac atttttattt 10244
   tttttgcttc atcggaaaat tatttacatt tttattaagt gtgtgtttag attttcgttt 10304
   ttcgttaaaa atttcataat taatttttta taacagtgat gtatgcagct aaggcatcct 10364
   aacagaccta tctttgtatg tatggcattc tgggcgggtc ggatttattt ttagccccat 10424
   aattccccac tactatacat aaaatgatac ctgctagtaa aataatttaa agtgtttcga 10484
   agtcatattg gtaaattgat ttcagttaaa aatcgaagtt tgttagctac taaagacggt 10544
   agcttctatg tcaaaatagt taattcctat attttataat tggatcattt tttctaaaat 10604
   ttattcaaac atctcaactc actttgacta taatcacttt ataattaatt ttgttgaatt 10664
   aaataaatta tattcaaatt aaatgtgata atacccactg aatgtgattg gaatgccatc 10724
   agggaaatat ggaacaaaga gagctgcatg tggaagggct tgttatgtct gcaaaacaga 10784
   caaagaagtt gtaatcacaa ttaggacaat tgccaaagat gtggggtcac attgacttgt 10844
   cagggaaagt ctatagatcc actgtgctct taaagctaga acaattaaca taaaatttct 10904
   gccaattaag gctttcactt cctatcccta tctgctgtat attttaagtt tgagtaaagg 10964
   gacagttgtg gtgatgttat aatcaactgc aggctgaggg aaaagttggg ggtcaagtgg 11024
   tgtctatata aacttttttg tcattattaa tctttaatct ttttggtttt gtaaaatgac 11084
   agcttttgtc ttgtgctgaa agttgactgt gctagctagt aaagtcatgc tggtttctta 11144
   attgattgtg gtgtttttat gcgacaactg tagacattgc aatgcaggca cacaagtcaa 11204
   gaggcaatta caaacaaaat acagctatta ataattgacc tactaactac taaggaggga 11264
   atttaaatgt gattttgaaa tcttatattg gatgtaaaga ggcaatactc atcagactgt 11324
   aaagtgacag aacaagtaga tggtaaacaa gtagaagtag aagcagcaac aaattgagta 11384
   aagaccatca atgtcaacac ttccttggag aagaatttca taagtgtcat gtgaaaataa 11444
   ctagacaaaa tgtagccaaa atgaagtatg aaactcagtt attgaaacga gtgacactga 11504
   ctagattcca agttattgaa ggaaccagca aaaggttaag tgacagtgaa gtgtgtgggt 11564
   tgaaagagga ttgaataaaa gctgaaccaa tggactgaat aggcaaactt gacaatttcc  11624
   cattagaatc tgttttaact tccttgtaca aaagtttctt cggtgaaatt tccagcattg  11684
   ttggtaacat gatgagtagc tcatgaatct ggaaaccaaa cagttttgtt tgttcaagct  11744
   gtagagaaga atgagaacta catgtgagca ttgttatgta ccaaaaaaac ctgtgagcat  11804
   tgttgctgca atcctggatc agtacgcata gcctgagcac aaagatgttg aattgaagta  11864
   gttgctctaa tatgttatga atattcaaga ttaaaacatt tttaagttat catgtgacaa  11924
   tcacataact attaaattat tttaatctta actattaatc attagatcta acgatcatat  11984
   tctcatataa aataattatt ctgatacaat aatatatata tatatatata tgtatgtagg  12044
   ggatctgagg atgtatcata tgagaaatgt gatattatgt gagaacatga gaataaatca  12104
   caaccgttag atttaatcaa cggaccagat taaaacaatt taatgctcac atgaccactt  12164
   aaaaatgtca tgtgactttc ttaaaaagtc aaatgacttc atgttttaat tttgaccgtt  12224
   catgattaga tttaacggtc atgatttatt ctcatgttct cacataaaaa catttttcat  12284
   ttttctgata tgtatatttg taacataaaa cacaacataa aatactatgt aaatcagcca  12344
   atttcaccaa atacaatatg atgtgaaaaa tataatatta ctattgatat tcactcaaat  12404
   gcacaatcgg ccctaaaaaa tggtcaattt ttctggttaa tatatttctt ttttaaaaca  12464
   tgagtattca tgcaatatat gatttcaaaa tatataattt ctttcaacat ttaaccaatt  12524
   tgcaccatgt gtaatatgta agagatcaaa aattatattg agttttattt atcttttaac  12584
   tgatatggga aacattttta tagataattt ttattcaagt tctaagttac ttcattttta  12644
   tttcggcgaa gttggtgctt ttttttcttt tcaaaccgtt tgaagatggt ttcctcatgg  12704
   attggagttt aatccatttt tctatcatga gctc                              12738
<210> 2
   <211> 3568
   <212> DNA
   <213> Lotus japonicus
<220>
   <221> 5'UTR
   <222> (1)..(137)
<220>
   <221> CDS
   <222> (138)..(3119)
<220>
   <221> 3'UTR
   <222> (3120)..(3568)
<400> 2
   ttcaactttc aaaacaaagt ggatgggatc ttcatcttat ataaccacac atcaatcatt      60
   tgtgctactt ctccaatttt ctttagagat gaaatgaaga gctaagcaga caagacaagt     120
   ttatttgttt gttgctg atg ggt ctt ggg ttc aag atg cag cag agc cac        170
                      Met Gly Leu Gly Phe Lys Met Gln Gln Ser His
                      1               5                   10
   cac cct gtg gct ttg aag tta cat gag caa gct ggg agc cag aga aag       218
   His Pro Val Ala Leu Lys Leu His Glu Gln Ala Gly Ser Gln Arg Lys
               15                  20                  25
   ttc act ttc att cag aac ttc aga aac tgg ttt cta ccc ctt ctg ttt       266
   Phe Thr Phe Ile Gln Asn Phe Arg Asn Trp Phe Leu Pro Leu Leu Phe
           30                  35                  40
   gta tgg ttc att gtt atg gct gca ttt ggt gcc tgc atc tac cat aaa       314
   Val Trp Phe Ile Val Met Ala Ala Phe Gly Ala Cys Ile Tyr His Lys
       45                  50                  55
   atg gat gct gaa act aaa gtc aga agg aaa gag gtg ctg ggt agc ctc       362
   Met Asp Ala Glu Thr Lys Val Arg Arg Lys Glu Val Leu Gly Ser Leu
   60                  65                  70                  75
   tgt gat caa agg gct aga atg cta caa gac caa ttc agt gtc agt gtc       410
   Cys Asp Gln Arg Ala Arg Met Leu Gln Asp Gln Phe Ser Val Ser Val
                   80                  85                  90
   aac cat gtc cat gcc ctt gcc atc ctt gtt tca acc ttc cat tac tac       458
   Asn His Val His Ala Leu Ala Ile Leu Val Ser Thr Phe His Tyr Tyr
               95                  100                 105
   aga aat act tca gcc att gac cag gaa acc ttt gca gaa tac acg gcc       506
   Arg Asn Thr Ser Ala Ile Asp Gln Glu Thr Phe Ala Glu Tyr Thr Ala
           110                 115                 120
   agg aca gca ttt gaa cgg cca tta atg agt ggg gtg gcc tat gca cag       554
   Arg Thr Ala Phe Glu Arg Pro Leu Met Ser Gly Val Ala Tyr Ala Gln
       125                 130                 135
   aga gtg gtt cac tca gag aga gaa aga ttt gag aag caa cat ggg tgg       602
   Arg Val Val His Ser Glu Arg Glu Arg Phe Glu Lys Gln His Gly Trp
   140                 145                 150                 155
   gtt ata aag aca atg gaa aga gtg cct tca ggg gtt agg gat gag tat       650
   Val Ile Lys Thr Met Glu Arg Val Pro Ser Gly Val Arg Asp Glu Tyr
                   160                 165                 170
   gca gca gtg ata ttt gca cag gaa act gtc tct tac ctt gaa tct att       698
   Ala Ala Val Ile Phe Ala Gln Glu Thr Val Ser Tyr Leu Glu Ser Ile
               175                 180                 185
   gat atg atg tct ggg gag gag gac cga gag aac att ttg agg gct aga       746
   Asp Met Met Ser Gly Glu Glu Asp Arg Glu Asn Ile Leu Arg Ala Arg
           190                 195                 200
   gcc act ggg aaa gct gtt ctg act agc cct ttc aga ctg ctg gat tct       794
   Ala Thr Gly Lys Ala Val Leu Thr Ser Pro Phe Arg Leu Leu Asp Ser
       205                 210                 215
   cat cat ctt ggc gtg gtt cta aca ttt cct gtt tat aaa tct aag ctc       842
   His His Leu Gly Val Val Leu Thr Phe Pro Val Tyr Lys Ser Lys Leu
   220                 225                 230                 235
   cct cca gag cca acg acg gaa gag gtc att aaa gcc ata gca gga tat      890
   Pro Pro Glu Pro Thr Thr Glu Glu Val Ile Lys Ala Ile Ala Gly Tyr
                   240                 245                 250
   att gga gga tcc ttt gat gtt gag tcc ctt gtg gag aat tta ctt ggt      938
   Ile Gly Gly Ser Phe Asp Val Glu Ser Leu Val Glu Asn Leu Leu Gly
               255                 260                 265
   caa ctt gct ggt aac caa gca att ttg gtg aag gta tat gat ata aca      986
   Gln Leu Ala Gly Asn Gln Ala Ile Leu Val Lys Val Tyr Asp Ile Thr
           270                 275                 280
   aac tct agc gac ccc cta atc atg tat ggc agc caa tat gaa gag ggt     1034
   Asn Ser Ser Asp Pro Leu Ile Met Tyr Gly Ser Gln Tyr Glu Glu Gly
       285                 290                 295
   gat atg tct ctt gtc cat gaa agt aag ctt gat ttt gga gat cca tac     1082
   Asp Met Ser Leu Val His Glu Ser Lys Leu Asp Phe Gly Asp Pro Tyr
   300                 305                 310                 315
   agg aaa cat cac atg atc tgt aga tat cac caa cag gca cca aca aat     1130
   Arg Lys His His Met Ile Cys Arg Tyr His Gln Gln Ala Pro Thr Asn
                   320                 325                 330
   tgg ata gca tat acc acg gca ttc cta ttc ttt gtg att ctt tgt tta     1178
   Trp Ile Ala Tyr Thr Thr Ala Phe Leu Phe Phe Val Ile Leu Cys Leu
               335                 340                 345
   gtg ggt tac att tta tat gct gct gga act cac att gtc aag gta gaa     1226
   Val Gly Tyr Ile Leu Tyr Ala Ala Gly Thr His Ile Val Lys Val Glu
           350                 355                 360
   gat gat tac aat gca atg cag gat tta aaa gtc aaa gca gaa gca gct     1274
   Asp Asp Tyr Asn Ala Met Gln Asp Leu Lys Val Lys Ala Glu Ala Ala
       365                 370                 375
   gat att gcc aag tca cag ttt cta gct acc gtc tct cat gaa att aga     1322
   Asp Ile Ala Lys Ser Gln Phe Leu Ala Thr Val Ser His Glu Ile Arg
   380                 385                 390                 395
   act ccc atg aat gga att tta gga atg ctt ggt ctg ctt tta cgc aca     1370
   Thr Pro Met Asn Gly Ile Leu Gly Met Leu Gly Leu Leu Leu Arg Thr
                   400                 405                 410
   gaa ttg agt tca aca caa aga gac tat gct cag act gct caa gca tgt     1418
   Glu Leu Ser Ser Thr Gln Arg Asp Tyr Ala Gln Thr Ala Gln Ala Cys
               415                 420                 425
   ggg aag gca cta ata gca tta ata aat gag gtg ctt gac cga gct aaa     1466
   Gly Lys Ala Leu Ile Ala Leu Ile Asn Glu Val Leu Asp Arg Ala Lys
           430                 435                 440
   att gaa gca ggc aaa tta gag cta gaa gca gtt cca ttt gac ctt cgt     1514
   Ile Glu Ala Gly Lys Leu Glu Leu Glu Ala Val Pro Phe Asp Leu Arg
       445                 450                 455
   tcc ata ctt gac gat gtc ctt tct ctt ttt tct gag aag tca aga cac     1562
   Ser Ile Leu Asp Asp Val Leu Ser Leu Phe Ser Glu Lys Ser Arg His
   460                 465                 470                 475
   aaa ggc tta gag ctg gca gtg ttt gtt tct gac aaa gtt ccg gat ata      1610
   Lys Gly Leu Glu Leu Ala Val Phe Val Ser Asp Lys Val Pro Asp Ile
                   480                 485                 490
   gtt atg ggc gat cct ggg cga ttc aga caa ata gtg aca aat ctt gtt      1658
   Val Met Gly Asp Pro Gly Arg Phe Arg Gln Ile Val Thr Asn Leu Val
               495                 500                 505
   gga aac tct gtt aag ttc act gag cga ggt cat ata ttt gtt aaa gtc      1706
   Gly Asn Ser Val Lys Phe Thr Glu Arg Gly His Ile Phe Val Lys Val
           510                 515                 520
   cat tta gct gaa aaa aga cag tgc aca atg aat gga aaa tgt gag act      1754
   His Leu Ala Glu Lys Arg Gln Cys Thr Met Asn Gly Lys Cys Glu Thr
       525                 530                 535
   ttt cta aat gga ggc tgt gat gat gtt ttg cat gta tct ggc agt tat      1802
   Phe Leu Asn Gly Gly Cys Asp Asp Val Leu His Val Ser Gly Ser Tyr
   540                 545                 550                 555
   aat ttg aaa acc ctt agt gga tat gaa gcc gct gat gaa cgg aac agc      1850
   Asn Leu Lys Thr Leu Ser Gly Tyr Glu Ala Ala Asp Glu Arg Asn Ser
                   560                 565                 570
   tgg gat aat ttt aag cat cat att gct gac gaa gaa ttt ttc ttt gat      1898
   Trp Asp Asn Phe Lys His His Ile Ala Asp Glu Glu Phe Phe Phe Asp
               575                 580                 585
   gct tcg gtt aaa aag ttg gcc tct agt gaa tct tat gag caa gtc acc      1946
   Ala Ser Val Lys Lys Leu Ala Ser Ser Glu Ser Tyr Glu Gln Val Thr
           590                 595                 600
   ttg atg gtc agc gtg gag gac act gga att ggg att tct ttc tct gcc      1994
   Leu Met Val Ser Val Glu Asp Thr Gly Ile Gly Ile Ser Phe Ser Ala
       605                 610                 615
   caa gat agt att ttc atg cct ttt gtg cag gct gac agc tca acc tct      2042
   Gln Asp Ser Ile Phe Met Pro Phe Val Gln Ala Asp Ser Ser Thr Ser
   620                 625                 630                 635
   cga aac tat ggg ggt acc ggg atc ggc ttg agt atc agt aag tgc ttg      2090
   Arg Asn Tyr Gly Gly Thr Gly Ile Gly Leu Ser Ile Ser Lys Cys Leu
                   640                 645                 650
   gtt gaa ctg atg ggc ggt cag ata aac ttc ata agc cga ccc cag gtc      2138
   Val Glu Leu Met Gly Gly Gln Ile Asn Phe Ile Ser Arg Pro Gln Val
               655                 660                 665
   ggg agc acg ttt tca ttc act gca gat ttc gga aca ttt aag aaa aac      2186
   Gly Ser Thr Phe Ser Phe Thr Ala Asp Phe Gly Thr Phe Lys Lys Asn
           670                 675                 680
   tca aca act gac atg aag aaa ctt aac ttt gaa gat cta cct tct agt      2234
   Ser Thr Thr Asp Met Lys Lys Leu Asn Phe Glu Asp Leu Pro Ser Ser
       685                 690                 695
   ttt aga ggt ctt aaa gcc att gtg gtt gat gga aaa cct gtt aga gct      2282
   Phe Arg Gly Leu Lys Ala Ile Val Val Asp Gly Lys Pro Val Arg Ala
   700                 705                 710                 715
   gca gtg act aga tac cat ttg aag aga cta ggg ata caa gct aaa gtt      2330
   Ala Val Thr Arg Tyr His Leu Lys Arg Leu Gly Ile Gln Ala Lys Val
                   720                 725                 730
   gca att agc atc aat aag gct gtt tct tta tgt ggg aaa aat ggt tct      2378
   Ala Ile Ser Ile Asn Lys Ala Val Ser Leu Cys Gly Lys Asn Gly Ser
               735                 740                 745
   ttg acc tcg gca tta ttt cag cct gat att att ttt gtt gag aag gac      2426
   Leu Thr Ser Ala Leu Phe Gln Pro Asp Ile Ile Phe Val Glu Lys Asp
           750                 755                 760
   tct tgg gtt tct gga gag gat ggt ggt atc ttc aat gcg ttt aag atg      2474
   Ser Trp Val Ser Gly Glu Asp Gly Gly Ile Phe Asn Ala Phe Lys Met
       765                 770                 775
   cct caa atg atc ctt ctt gca acc aat atc tgt aac gct gaa ttt gat      2522
   Pro Gln Met Ile Leu Leu Ala Thr Asn Ile Cys Asn Ala Glu Phe Asp
   780                 785                 790                 795
   aaa gcc aaa gct gca ggt ttc agt gat aca gtg atc atg aag cca ctg      2570
   Lys Ala Lys Ala Ala Gly Phe Ser Asp Thr Val Ile Met Lys Pro Leu
                   800                 805                 810
   aga gct agt atg ctg gct gct tgt ctt cag caa gtt ttc ggg act ggc      2618
   Arg Ala Ser Met Leu Ala Ala Cys Leu Gln Gln Val Phe Gly Thr Gly
               815                 820                 825
   aag acg agg cag ttt ggg aaa gac atg tcg aat ggt tct tca gta cga      2666
   Lys Thr Arg Gln Phe Gly Lys Asp Met Ser Asn Gly Ser Ser Val Arg
           830                 835                 840
   agc ctt ctt tgc gga aag aaa atc tta gtg gtt gat gat aat ttg gtg      2714
   Ser Leu Leu Cys Gly Lys Lys Ile Leu Val Val Asp Asp Asn Leu Val
       845                 850                 855
   aac cga agg gtc gcc gcc ggc gcg ttg aaa aac ttt gga gct gat gtc      2762
   Asn Arg Arg Val Ala Ala Gly Ala Leu Lys Asn Phe Gly Ala Asp Val
   860                 865                 870                 875
   aaa tgt gca gca agt ggc aaa gct gct ctt gaa atg ctt caa tat cct      2810
   Lys Cys Ala Ala Ser Gly Lys Ala Ala Leu Glu Met Leu Gln Tyr Pro
                   880                 885                 890
   cac gat ttc gat gct tgc ttc atg gat att caa atg cca gaa atg gat      2858
   His Asp Phe Asp Ala Cys Phe Met Asp Ile Gln Met Pro Glu Met Asp
               895                 900                 905
   ggg ttt gag gcc act cgt cga att cgg atg atg gaa aga gag gca agt      2906
   Gly Phe Glu Ala Thr Arg Arg Ile Arg Met Met Glu Arg Glu Ala Ser
           910                 915                 920
   gag cag ctg aaa agt gaa tct ggt gaa gaa aat ggt aag aaa agt gag      2954
   Glu Gln Leu Lys Ser Glu Ser Gly Glu Glu Asn Gly Lys Lys Ser Glu
       925                 930                 935
   ttc cac atg cct ata ttg gcc atg aca gct gat gta atc cat gct aca      3002
   Phe His Met Pro Ile Leu Ala Met Thr Ala Asp Val Ile His Ala Thr
   940                 945                 950                 955
   tat gat aag tgc tta aat tgt ggg atg gat gga tac gtc tca aag cct      3050
   Tyr Asp Lys Cys Leu Asn Cys Gly Met Asp Gly Tyr Val Ser Lys Pro
                   960                 965                 970
   ttt gaa gaa gag aat ctc tat caa gca gtt gca aag ttc ttc aag tcc      3098
   Phe Glu Glu Glu Asn Leu Tyr Gln Ala Val Ala Lys Phe Phe Lys Ser
               975                 980                 985
   aag cct gct tca gac tca tga cactgcttat tctgcagaac aggtcaacca         3149
   Lys Pro Ala Ser Asp Ser
           990
   acttttgatt gagaaacatt tagtgttagc atgtttggat caacttctcc cagcatcaat   3209
   tctgaaactc agaagctact catagaagct tctagccaga attgattttg gcttcagaag   3269
   ctactaatgg ttttatgtag agagcaaaag ttggtttcca agatatgcga ggatccatga   3329
   tgatacacac gtctgaagtg atcatttcta accagttgaa gtttcactcg acgtgatttg   3389
   aatccaagta aatgcatacc acataattat ccatccccat cttgtgtaca gattctccca   3449
   agggataaga aaatttatgt aaattcaatt tttttctttt gcatctcaat acttccctgt   3509
   tagaactttt tccctatgat tatttccact tttcattttc aattatattt tttgtaaaa    3568
<210> 3
   <211> 993
   <212> PRT
   <213> Lotus japonicus
<400> 3
   Met Gly Leu Gly Phe Lys Met Gln Gln Ser His His Pro Val Ala Leu
   1               5                   10                  15
   Lys Leu His Glu Gln Ala Gly Ser Gln Arg Lys Phe Thr Phe Ile Gln
               20                  25                  30
   Asn Phe Arg Asn Trp Phe Leu Pro Leu Leu Phe Val Trp Phe Ile Val
           35                  40                  45
   Met Ala Ala Phe Gly Ala Cys Ile Tyr His Lys Met Asp Ala Glu Thr
       50                  55                  60
   Lys Val Arg Arg Lys Glu Val Leu Gly Ser Leu Cys Asp Gln Arg Ala
   65                  70                  75                  80
   Arg Met Leu Gln Asp Gln Phe Ser Val Ser Val Asn His Val His Ala
                   85                  90                  95
   Leu Ala Ile Leu Val Ser Thr Phe His Tyr Tyr Arg Asn Thr Ser Ala
               100                 105                 110
   Ile Asp Gln Glu Thr Phe Ala Glu Tyr Thr Ala Arg Thr Ala Phe Glu
   115 120 125
   Arg Pro Leu Met Ser Gly Val Ala Tyr Ala Gln Arg Val Val His Ser
   130 135 140
   Glu Arg Glu Arg Phe Glu Lys Gln His Gly Trp Val Ile Lys Thr Met
   145 150 155 160
   Glu Arg Val Pro Ser Gly Val Arg Asp Glu Tyr Ala Ala Val Ile Phe
   165 170 175
   Ala Gln Glu Thr Val Ser Tyr Leu Glu Ser Ile Asp Met Met Ser Gly
   180 185 190
   Glu Glu Asp Arg Glu Asn Ile Leu Arg Ala Arg Ala Thr Gly Lys Ala
   195 200 205
   Val Leu Thr Ser Pro Phe Arg Leu Leu Asp Ser His His Leu Gly Val
   210 215 220
   Val Leu Thr Phe Pro Val Tyr Lys Ser Lys Leu Pro Pro Glu Pro Thr
   225 230 235 240
   Thr Glu Glu Val Ile Lys Ala Ile Ala Gly Tyr Ile Gly Gly Ser Phe
   245 250 255
   Asp Val Glu Ser Leu Val Glu Asn Leu Leu Gly Gln Leu Ala Gly Asn
   260 265 270
   Gln Ala Ile Leu Val Lys Val Tyr Asp Ile Thr Asn Ser Ser Asp Pro
   275 280 285
   Leu Ile Met Tyr Gly Ser Gln Tyr Glu Glu Gly Asp Met Ser Leu Val
   290 295 300
   His Glu Ser Lys Leu Asp Phe Gly Asp Pro Tyr Arg Lys His His Met
   305 310 315 320
   Ile Cys Arg Tyr His Gln Gln Ala Pro Thr Asn Trp Ile Ala Tyr Thr
   325 330 335
   Thr Ala Phe Leu Phe Phe Val Ile Leu Cys Leu Val Gly Tyr Ile Leu
   340 345 350
   Tyr Ala Ala Gly Thr His Ile Val Lys Val Glu Asp Asp Tyr Asn Ala
           355                 360                 365
   Met Gln Asp Leu Lys Val Lys Ala Glu Ala Ala Asp Ile Ala Lys Ser
       370                 375                 380
   Gln Phe Leu Ala Thr Val Ser His Glu Ile Arg Thr Pro Met Asn Gly
   385                 390                 395                 400
   Ile Leu Gly Met Leu Gly Leu Leu Leu Arg Thr Glu Leu Ser Ser Thr
                   405                 410                 415
   Gln Arg Asp Tyr Ala Gln Thr Ala Gln Ala Cys Gly Lys Ala Leu Ile
               420                 425                 430
   Ala Leu Ile Asn Glu Val Leu Asp Arg Ala Lys Ile Glu Ala Gly Lys
           435                 440                 445
   Leu Glu Leu Glu Ala Val Pro Phe Asp Leu Arg Ser Ile Leu Asp Asp
       450                 455                 460
   Val Leu Ser Leu Phe Ser Glu Lys Ser Arg His Lys Gly Leu Glu Leu
   465                 470                 475                 480
   Ala Val Phe Val Ser Asp Lys Val Pro Asp Ile Val Met Gly Asp Pro
                   485                 490                 495
   Gly Arg Phe Arg Gln Ile Val Thr Asn Leu Val Gly Asn Ser Val Lys
               500                 505                 510
   Phe Thr Glu Arg Gly His Ile Phe Val Lys Val His Leu Ala Glu Lys
           515                 520                 525
   Arg Gln Cys Thr Met Asn Gly Lys Cys Glu Thr Phe Leu Asn Gly Gly
       530                 535                 540
   Cys Asp Asp Val Leu His Val Ser Gly Ser Tyr Asn Leu Lys Thr Leu
   545                 550                 555                 560
   Ser Gly Tyr Glu Ala Ala Asp Glu Arg Asn Ser Trp Asp Asn Phe Lys
                   565                 570                 575
   His His Ile Ala Asp Glu Glu Phe Phe Phe Asp Ala Ser Val Lys Lys
               580                 585                 590
   Leu Ala Ser Ser Glu Ser Tyr Glu Gln Val Thr Leu Met Val Ser Val
           595                 600                 605
   Glu Asp Thr Gly Ile Gly Ile Ser Phe Ser Ala Gln Asp Ser Ile Phe
       610                 615                 620
   Met Pro Phe Val Gln Ala Asp Ser Ser Thr Ser Arg Asn Tyr Gly Gly
   625                 630                 635                 640
   Thr Gly Ile Gly Leu Ser Ile Ser Lys Cys Leu Val Glu Leu Met Gly
                   645                 650                 655
   Gly Gln Ile Asn Phe Ile Ser Arg Pro Gln Val Gly Ser Thr Phe Ser
               660                 665                 670
   Phe Thr Ala Asp Phe Gly Thr Phe Lys Lys Asn Ser Thr Thr Asp Met
           675                 680                 685
   Lys Lys Leu Asn Phe Glu Asp Leu Pro Ser Ser Phe Arg Gly Leu Lys
       690                 695                 700
   Ala Ile Val Val Asp Gly Lys Pro Val Arg Ala Ala Val Thr Arg Tyr
   705                 710                 715                 720
   His Leu Lys Arg Leu Gly Ile Gln Ala Lys Val Ala Ile Ser Ile Asn
                   725                 730                 735
   Lys Ala Val Ser Leu Cys Gly Lys Asn Gly Ser Leu Thr Ser Ala Leu
               740                 745                 750
   Phe Gln Pro Asp Ile Ile Phe Val Glu Lys Asp Ser Trp Val Ser Gly
           755                 760                 765
   Glu Asp Gly Gly Ile Phe Asn Ala Phe Lys Met Pro Gln Met Ile Leu
       770                 775                 780
   Leu Ala Thr Asn Ile Cys Asn Ala Glu Phe Asp Lys Ala Lys Ala Ala
   785                 790                 795                 800
   Gly Phe Ser Asp Thr Val Ile Met Lys Pro Leu Arg Ala Ser Met Leu
                   805                 810                 815
   Ala Ala Cys Leu Gln Gln Val Phe Gly Thr Gly Lys Thr Arg Gln Phe
               820                 825                 830
   Gly Lys Asp Met Ser Asn Gly Ser Ser Val Arg Ser Leu Leu Cys Gly
           835                 840                 845
   Lys Lys Ile Leu Val Val Asp Asp Asn Leu Val Asn Arg Arg Val Ala
       850                 855                 860
   Ala Gly Ala Leu Lys Asn Phe Gly Ala Asp Val Lys Cys Ala Ala Ser
   865                 870                 875                 880
   Gly Lys Ala Ala Leu Glu Met Leu Gln Tyr Pro His Asp Phe Asp Ala
                   885                 890                 895
   Cys Phe Met Asp Ile Gln Met Pro Glu Met Asp Gly Phe Glu Ala Thr
               900                 905                 910
   Arg Arg Ile Arg Met Met Glu Arg Glu Ala Ser Glu Gln Leu Lys Ser
           915                 920                 925
   Glu Ser Gly Glu Glu Asn Gly Lys Lys Ser Glu Phe His Met Pro Ile
       930                 935                 940
   Leu Ala Met Thr Ala Asp Val Ile His Ala Thr Tyr Asp Lys Cys Leu
   945                 950                 955                 960
   Asn Cys Gly Met Asp Gly Tyr Val Ser Lys Pro Phe Glu Glu Glu Asn
                   965                 970                 975
   Leu Tyr Gln Ala Val Ala Lys Phe Phe Lys Ser Lys Pro Ala Ser Asp
               980                 985                 990
   Ser
<210> 4
   <211> 12738
   <212> DNA
   <213> Lotus japonicus
<220>
   <221> promoter
   <222> (1)..(2359)
   <223> promoter + 5' UTR
<220>
   <221> exon
   <222> (2360)..(2704)
<220>
   <221> Intron
   <222> (2705)..(3024)
<220>
   <221> exon
   <222> (3025)..(3258)
<220>
   <221> Intron
   <222> (3259)..(3390)
<220>
   <221> exon
   <222> (3391)..(3560)
<220>
   <221> Intron
   <222> (3561)..(3834)
<220>
   <221> exon
   <222> (3835)..(4053)
   <223> C/T mutation in exon 4
<220>
   <221> Intron
   <222> (4054)..(4145)
<220>
   <221> exon
   <222> (4146)..(4332)
<220>
   <221> Intron
   <222> (4333)..(4420)
<220>
   <221> exon
   <222> (4421)..(4472)
<220>
   <221> Intron
   <222> (4473)..(4586)
<220>
   <221> exon
   <222> (4587) .. (4816)
<220>
   <221> Intron
   <222> (4817)..(5037)
<220>
   <221> exon
   <222> (5038)..(5136)
<220>
   <221> Intron
   <222> (5137)..(5308)
<220>
   <221> exon
   <222> (5309)..(6024)
<220>
   <221> Intron
   <222> (6025)..(7042)
<220>
   <221> exon
   <222> (7043)..(7513)
<220>
   <221> Intron
   <222> (7514)..(8306)
<220>
   <221> exon
   <222> (8307)..(8564)
<220>
   <221> Intron
   <222> (8565)..(12738)
   <223> 3' UTR + 3' intron
<400> 4
   cccgggattg ttcatatccc catgtgtgtg tttggtttgg acacaagcaa gcttgccaca     60
   gtggtgttta caactcattc tgaattatat atatatatat atatatataa ctaatctcta    120
   tcaataattt agttagtttt atgggataat atgattcccg tcaaattagc ttgtaggtta    180
   aaaatccata tttatataat aaattagact tatctaaatc acttaagtaa atatgatgta    240
   aaatcacaat agcttaggta gtttaacaat gttttatcat gaatttaaaa aaaattagtt    300
   gtttcaaaat attattggac caaataagca aagaggagct aatccaatta taaacagcac    360
   atctttataa ttgttttcta ctcgatctaa tattggttcc ttctttcctt gagaaaaaaa    420
   gggttaaaaa tgcgatttgt tttgtaaaat taatttgtga cattaacaag agaaaactct    480
   acttttttaa tttttaagaa gttacttaaa tatagaaaga aagaagttac ttaaatatga    540
   aacttgtcta accaaagtca accgatcaaa gcgtttggtt agcaaagaga acttaaaaaa    600
   ataactaaat taactgtttg aacagtgatg taacagagaa atagaatggc ttacatatgg    660
   attatggagt taatggaagg cacactagtt ttagttcacg gcaacaagac agaattatcc    720
   tcctatattt taggaggttt tcttatttat ttttaatgta agacgtgtat ccttcttttg    780
   aggtaataat gttcgagctt gaaacattca cattatagtg tgactaattg cttaatatga    840
   atcaagcatg taccacttga aacactcact catcgtttat aatataatta ctttatagta    900
   ctataaagta tgtaattatt ttctcctttt taagtatctc aatcattttt ttgacccaaa    960
   tattatttta gagaagaaac atgtcttaag accaatacat caacatatat ggagagtagt   1020
   ttgacaacct ctcatgagct ttctaaagcc aaacaaatgt actcacataa aaaaattaaa   1080
   aatttacagc aagtgagtta taaagagtga ttaataaatg ttgaataacg atggagggta   1140
   gttatgaaat tccataaata aagcaaagga tgtttatggc aattgacatg ggaataagat   1200
   ccgcgcgttg tcgccgtgtc ataatcgctc agatttgtga tagcgagaga tttccattct   1260
   tttcctcttt tttcgaacga acgaacgaac gaacacagta gcagctgtat taggattcag   1320
   attgcatgat acattgatat tgatattgat attgatattg atattgttgt gggtcaagtc   1380
   tctactctac tatccaaagt aagcatatat atagagagag agagagagct tgggactagg   1440
   gaggatatta gcttatgtga ctgtgaagtt gaagggagac aagagcgtat ctggcaaaat   1500
   cctcaaaata aaatactagt actagtacag agaaaagaga ctaagagaga gagtgctgct   1560
   gcacatcaag acccattgtg atttgtgatt tgtgatttgt gatttgtgat ttgtgagtga   1620
   gttcattgta caggtattat tgtttgttgt ttctctcctc aaccaccctc taaagtctaa   1680
   tctaactcat tgggctctgt gcttagctgg ttgtgttttg tgtatggtga attagggggt   1740
   caatctctgg ttttcatcat tattatatta tatatgggaa taccgtgctc tcttctttgt   1800
   ccttcaccaa aactagtttc acgcctacac aacatgatta gagcctcttc atttttttaa   1860
   tctcatcctt taagtgtatt tctatttcta ttggctattg ggacaagggg aaggtggtgc   1920
   ttcttaggaa cttgagctgt tttccatctt ttgagaccca tgctttgtct ctctcatttt   1980
   taattctggg tctctttctt ctcttgtcct gattttttaa atgtgcttct tttttgcttc   2040
   ttacaaccac cctctaaacc attcatcatg cttggtttgc ttttgcttct cctttcacag   2100
   gtttcaatca cgcaaaacaa tgctgcaatg atgctgtact tggagtttct tctgtgaccc   2160
   cttttttcct tccttcaaca atcaacccac cagagaaaag tgtctcagat tttgagacta   2220
   ctttcaactt tcaaaacaaa gtggatggga tcttcatctt atataaccac acatcaatca   2280
   tttgtgctac ttctccaatt ttctttagag atgaaatgaa gagctaagca gacaagacaa   2340
   gtttatttgt ttgttgctg atg ggt ctt ggg ttc aag atg cag cag agc cac    2392
                       Met Gly Leu Gly Phe Lys Met Gln Gln Ser His
                       1               5                   10
   cac cct gtg gct ttg aag tta cat gag caa gct ggg agc cag aga aag     2440
   His Pro Val Ala Leu Lys Leu His Glu Gln Ala Gly Ser Gln Arg Lys
               15                  20                  25
   ttc act ttc att cag aac ttc aga aac tgg ttt cta ccc ctt ctg ttt     2488
   Phe Thr Phe Ile Gln Asn Phe Arg Asn Trp Phe Leu Pro Leu Leu Phe
           30                  35                  40
   gta tgg ttc att gtt atg gct gca ttt ggt gcc tgc atc tac cat aaa     2536
   Val Trp Phe Ile Val Met Ala Ala Phe Gly Ala Cys Ile Tyr His Lys
       45                  50                  55
   atg gat gct gaa act aaa gtc aga agg aaa gag gtg ctg ggt agc ctc     2584
   Met Asp Ala Glu Thr Lys Val Arg Arg Lys Glu Val Leu Gly Ser Leu
   60                  65                  70                  75
   tgt gat caa agg gct aga atg cta caa gac caa ttc agt gtc agt gtc     2632
   Cys Asp Gln Arg Ala Arg Met Leu Gln Asp Gln Phe Ser Val Ser Val
                   80                  85                  90
   aac cat gtc cat gcc ctt gcc atc ctt gtt tca acc ttc cat tac tac     2680
   Asn His Val His Ala Leu Ala Ile Leu Val Ser Thr Phe His Tyr Tyr
               95                  100                 105
   aga aat act tca gcc att gac cag gtttgtgctt gattttcctt tccttgaagc    2734
   Arg Asn Thr Ser Ala Ile Asp Gln
           110                 115
   attttttagt tggaggctca atttcttttt ctgatttgat tctggcctta aaaattagaa    2794
   tcaattgtag aaggatttcc aaacatgccc attttggaaa ttggtgcatc tgatagtatc   2854
   atgtttagat cagtttcttt ttcctcagaa ttgattttgg gcttaaaatc aattgtggaa   2914
   ggatattcat tagtaatttg gatattgttg catcatatgg ttctatctag ttacatcatt   2974
   tttttccact ctgattgcat gtatctttct cctgttcttt tccctatcag gaa acc      3030
                                                          Glu Thr
   ttt gca gaa tac acg gcc agg aca gca ttt gaa cgg cca tta atg agt     3078
   Phe Ala Glu Tyr Thr Ala Arg Thr Ala Phe Glu Arg Pro Leu Met Ser
           120                 125                 130
   ggg gtg gcc tat gca cag aga gtg gtt cac tca gag aga gaa aga ttt     3126
   Gly Val Ala Tyr Ala Gln Arg Val Val His Ser Glu Arg Glu Arg Phe
       135                 140                 145
   gag aag caa cat ggg tgg gtt ata aag aca atg gaa aga gtg cct tca     3174
   Glu Lys Gln His Gly Trp Val Ile Lys Thr Met Glu Arg Val Pro Ser
   150                 155                 160                 165
   ggg gtt agg gat gag tat gca gca gtg ata ttt gca cag gaa act gtc     3222
   Gly Val Arg Asp Glu Tyr Ala Ala Val Ile Phe Ala Gln Glu Thr Val
                   170                 175                 180
   tct tac ctt gaa tct att gat atg atg tct ggg gag gtaaatgtca          3268
   Ser Tyr Leu Glu Ser Ile Asp Met Met Ser Gly Glu
               185                 190
   acacttgtga attaattgta aaactcagaa gctactcaga gaagctcttc cccagaattg   3328
   gttctgcctt tagaataaat tgtacatgga tttgaccaca ttttctcatt tgcatgatgc   3388
   ag gag gac cga gag aac att ttg agg gct aga gcc act ggg aaa gct      3435
      Glu Asp Arg Glu Asn Ile Leu Arg Ala Arg Ala Thr Gly Lys Ala
          195                 200                 205
   gtt ctg act agc cct ttc aga ctg ctg gat tct cat cat ctt ggc gtg     3483
   Val Leu Thr Ser Pro Phe Arg Leu Leu Asp Ser His His Leu Gly Val
       210                 215                 220
   gtt cta aca ttt cct gtt tat aaa tct aag ctc cct cca gag cca acg     3531
   Val Leu Thr Phe Pro Val Tyr Lys Ser Lys Leu Pro Pro Glu Pro Thr
   225                 230                 235                 240
   acg gaa gag gtc att aaa gcc ata gca gg  gtatgtcctc atttcacttt       3580
   Thr Glu Glu Val Ile Lys Ala Ile Ala Gly
                   245
   tcttgccaaa accagacttc tatttggttg tgtttccgta ggctatgact gatatgtagt   3640
   ttcaactcag ttagactata atataaaccc ttcatgtggc ccttacccaa cagcttaagc   3700
   ttttgggata attggttgct tgacaaactc cttccgtaga aaacttggtt agctttggtt   3760
   ctatgtgggc tttatgtttt ccctgagctt atgtaatagc atgatgtgtt taatgtactt   3820
   tttaatggaa acag a tat att gga gga tcc ttt gat gtt gag tcc ctt gtg   3871
                     Tyr Ile Gly Gly Ser Phe Asp Val Glu Ser Leu Val
                                             255                 260
   gag aat tta ttt ggt caa ctt gct ggt aac caa gca att ttg gtg aag     3919
   Glu Asn Leu Phe Gly Gln Leu Ala Gly Asn Gln Ala Ile Leu Val Lys
           265                 270                 275
   gta tat gat ata aca aac tct agc gac ccc cta atc atg tat ggc agc     3967
   Val Tyr Asp Ile Thr Asn Ser Ser Asp Pro Leu Ile Met Tyr Gly Ser
       280                 285                 290
   caa tat gaa gag ggt gat atg tct ctt gtc cat gaa agt aag ctt gat     4015
   Gln Tyr Glu Glu Gly Asp Met Ser Leu Val His Glu Ser Lys Leu Asp
   295                 300                 305                 310
   ttt gga gat cca tac agg aaa cat cac atg atc tgt ag  gtgggtgctt      4063
   Phe Gly Asp Pro Tyr Arg Lys His His Met Ile Cys Arg
                   315                 320
   ctagttattg ttgtagtaca ttttctgagt ttcagtggtt tatcaattat cagcagattc   4123
   ttatgatcaa tttttttaac ag a tat cac caa cag gca cca aca aat tgg      4173
                              Tyr His Gln Gln Ala Pro Thr Asn Trp
                                  325                 330
   ata gca tat acc acg gca ttc cta ttc ttt gtg att ctt tgt tta gtg     4221
   Ile Ala Tyr Thr Thr Ala Phe Leu Phe Phe Val Ile Leu Cys Leu Val
           335                 340                 345
   ggt tac att tta tat gct gct gga act cac att gtc aag gta gaa gat     4269
   Gly Tyr Ile Leu Tyr Ala Ala Gly Thr His Ile Val Lys Val Glu Asp
       350                 355                 360
   gat tac aat gca atg cag gat tta aaa gtc aaa gca gaa gca gct gat     4317
   Asp Tyr Asn Ala Met Gln Asp Leu Lys Val Lys Ala Glu Ala Ala Asp
   365                 370                 375                 380
   att gcc aag tca cag gtacttttca tgacatgtta gcactgttcg ttatttcctt     4372
   Ile Ala Lys Ser Gln
                   385
   gaattgcata ctgatcacta gaaactgaaa atttgttatt aatgtcag ttt cta gct    4429
                                                        Phe Leu Ala
   acc gtc tct cat gaa att aga act ccc atg aat gga att tta g           4472
   Thr Val Ser His Glu Ile Arg Thr Pro Met Asn Gly Ile Leu
       390                 395                 400
   gtaactttaa gattctctct cgttctcttt ccactgaaaa gcaacacatg ctttcattcc   4532
   atacctgata ctttcccatt agtgatgcta tcgttaaact ccttgtcact gtag ga       4588
                                                              Gly
   atg ctt ggt ctg ctt tta cgc aca gaa ttg agt tca aca caa aga gac      4636
   Met Leu Gly Leu Leu Leu Arg Thr Glu Leu Ser Ser Thr Gln Arg Asp
       405                 410                 415
   tat gct cag act gct caa gca tgt ggg aag gca cta ata gca tta ata      4684
   Tyr Ala Gln Thr Ala Gln Ala Cys Gly Lys Ala Leu Ile Ala Leu Ile
   420                 425                 430                 435
   aat gag gtg ctt gac cga gct aaa att gaa gca ggc aaa tta gag cta      4732
   Asn Glu Val Leu Asp Arg Ala Lys Ile Glu Ala Gly Lys Leu Glu Leu
                   440                 445                 450
   gaa gca gtt cca ttt gac ctt cgt tcc ata ctt gac gat gtc ctt tct      4780
   Glu Ala Val Pro Phe Asp Leu Arg Ser Ile Leu Asp Asp Val Leu Ser
               455                 460                 465
   ctt ttt tct gag aag tca aga cac aaa ggc tta gag gtacgtttag           4826
   Leu Phe Ser Glu Lys Ser Arg His Lys Gly Leu Glu
           470                 475
   tcattgctaa atctgttgtg aagtcctgta caagtggcgt aatttcatag tcctatttcc    4886
   ttttcttcaa ttgatgatct tatttcatat tcctctcgtg tttctctctt tctatgttgc    4946
   catgtcgttg ggcggtgatg ttcctactct gatcccaaat tcctgatgtg caattattcc    5006
   gacttggact tcaatggttt gggaagtata g ctg gca gtg ttt gtt tct gac       5058
                                      Leu Ala Val Phe Val Ser Asp
                                      480                 485
   aaa gtt ccg gat ata gtt atg ggc gat cct ggg cga ttc aga caa ata      5106
   Lys Val Pro Asp Ile Val Met Gly Asp Pro Gly Arg Phe Arg Gln Ile
               490                 495                 500
   gtg aca aat ctt gtt gga aac tct gtt aag gttagtggaa ttttcaaact        5156
   Val Thr Asn Leu Val Gly Asn Ser Val Lys
           505                 510
   ttatttgcct aatgttgtgt gcaagttgtg tgttggaaat gcgtcctttt aacgttataa    5216
   aatcgtacaa gttcgtattc tccattgtat acaataactt attagcaaag tacttgttga    5276
   tatcattact gattaacttt aatatcttgc ag ttc act gag cga ggt cat ata      5329
                                       Phe Thr Glu Arg Gly His Ile
                                                   515
   ttt gtt aaa gtc cat tta gct gaa aaa aga cag tgc aca atg aat gga      5377
   Phe Val Lys Val His Leu Ala Glu Lys Arg Gln Cys Thr Met Asn Gly
   520                 525                 530                 535
   aaa tgt gag act ttt cta aat gga ggc tgt gat gat gtt ttg cat gta      5425
   Lys Cys Glu Thr Phe Leu Asn Gly Gly Cys Asp Asp Val Leu His Val
                   540                 545                 550
   tct ggc agt tat aat ttg aaa acc ctt agt gga tat gaa gcc gct gat      5473
   Ser Gly Ser Tyr Asn Leu Lys Thr Leu Ser Gly Tyr Glu Ala Ala Asp
               555                 560                 565
   gaa cgg aac agc tgg gat aat ttt aag cat cat att gct gac gaa gaa      5521
   Glu Arg Asn Ser Trp Asp Asn Phe Lys His His Ile Ala Asp Glu Glu
           570                 575                 580
   ttt ttc ttt gat gct tcg gtt aaa aag ttg gcc tct agt gaa tct tat      5569
   Phe Phe Phe Asp Ala Ser Val Lys Lys Leu Ala Ser Ser Glu Ser Tyr
       585                 590                 595
   gag caa gtc acc ttg atg gtc agc gtg gag gac act gga att ggg att      5617
   Glu Gln Val Thr Leu Met Val Ser Val Glu Asp Thr Gly Ile Gly Ile
   600                 605                 610                 615
   tct ttc tct gcc caa gat agt att ttc atg cct ttt gtg cag gct gac      5665
   Ser Phe Ser Ala Gln Asp Ser Ile Phe Met Pro Phe Val Gln Ala Asp
                   620                 625                 630
   agc tca acc tct cga aac tat ggg ggt acc ggg atc ggc ttg agt atc      5713
   Ser Ser Thr Ser Arg Asn Tyr Gly Gly Thr Gly Ile Gly Leu Ser Ile
               635                 640                 645
   agt aag tgc ttg gtt gaa ctg atg ggc ggt cag ata aac ttc ata agc      5761
   Ser Lys Cys Leu Val Glu Leu Met Gly Gly Gln Ile Asn Phe Ile Ser
           650                 655                 660
   cga ccc cag gtc ggg agc acg ttt tca ttc act gca gat ttc gga aca      5809
   Arg Pro Gln Val Gly Ser Thr Phe Ser Phe Thr Ala Asp Phe Gly Thr
       665                 670                 675
   ttt aag aaa aac tca aca act gac atg aag aaa ctt aac ttt gaa gat      5857
   Phe Lys Lys Asn Ser Thr Thr Asp Met Lys Lys Leu Asn Phe Glu Asp
   680                 685                 690                 695
   cta cct tct agt ttt aga ggt ctt aaa gcc att gtg gtt gat gga aaa      5905
   Leu Pro Ser Ser Phe Arg Gly Leu Lys Ala Ile Val Val Asp Gly Lys
                   700                 705                 710
   cct gtt aga gct gca gtg act aga tac cat ttg aag aga cta ggg ata      5953
   Pro Val Arg Ala Ala Val Thr Arg Tyr His Leu Lys Arg Leu Gly Ile
               715                 720                 725
   caa gct aaa gtt gca att agc atc aat aag gct gtt tct tta tgt ggg      6001
   Gln Ala Lys Val Ala Ile Ser Ile Asn Lys Ala Val Ser Leu Cys Gly
           730                 735                 740
   aaa aat ggt tct ttg acc tcg gc  gtaagtcttt aattaacctt tttggtttca     6054
   Lys Asn Gly Ser Leu Thr Ser Ala
       745                 750
   attatgtaga aatgtattga atgttatgat aatcagtagc attatcaact tttagtaatt   6114
   gttcttaaca tatgctaata gtcatatctt tctataatac tacaatactg tagccatata   6174
   atatctttcc tgtattggag tgagttttca aatgtttttc tgtgatattt tggaagttat   6234
   cttcagtttg agaactcatt tgtcattttt gcattttgtt attggatatt tggatggatc   6294
   tttacaaagg atgtgtggat tttgacttgt tatacacatt tcttctccat tttatattgt   6354
   ttgtgttatt ctttttactc ataaagaaat ttagaaactg cattgactgg ttctttttaa   6414
   ttacttacag atattgacat tgatattttt tgtaaatgct gtcttgacat ggtttaatta   6474
   cttacagact aggtttttct ttccttttct aacatgcata tccatttact tttttgacca   6534
   accaacatcc tcatgagtca tgacatgttg atgatttata tggttgactt gagactattt   6594
   agacattaaa taaccgcaaa ttccatgttg tttgtgtgtt tggttccctg ttgggtaatc   6654
   tcagaatcaa ttatgataga gtaaaatcaa ttttggatga gatgtgtggg tgtcattttg   6714
   taaacctaaa cccaaaatca attctgctat aagctagaga gagtagttga acataatcaa   6774
   ttgtgagatt ttgcaagtgg attgcacaac attgccctat gaaaatcact ttttgttcac   6834
   aaaatttatc taaacataca taacttcatt ttcaaccttt actataatca attttacaat   6894
   aattaatttt acccaaaatc aattgtgaca atgagtttcc aaacacacac ttaaagacta   6954
   ccatttgcag aaaatatgtg atagaagact tatgtttatg tagtgtgttt cagttcattc   7014
   actgatttaa actactcgga ttttgcag a tta ttt cag cct gat att att ttt    7067
                                    Leu Phe Gln Pro Asp Ile Ile Phe
                                                    755
   gtt gag aag gac tct tgg gtt tct gga gag gat ggt ggt atc ttc aat     7115
   Val Glu Lys Asp Ser Trp Val Ser Gly Glu Asp Gly Gly Ile Phe Asn
   760                 765                 770                 775
   gcg ttt aag atg cct caa atg atc ctt ctt gca acc aat atc tgt aac     7163
   Ala Phe Lys Met Pro Gln Met Ile Leu Leu Ala Thr Asn Ile Cys Asn
                   780                 785                 790
   gct gaa ttt gat aaa gcc aaa gct gca ggt ttc agt gat aca gtg atc     7211
   Ala Glu Phe Asp Lys Ala Lys Ala Ala Gly Phe Ser Asp Thr Val Ile
               795                 800                 805
   atg aag cca ctg aga gct agt atg ctg gct gct tgt ctt cag caa gtt     7259
   Met Lys Pro Leu Arg Ala Ser Met Leu Ala Ala Cys Leu Gln Gln Val
           810                 815                 820
   ttc ggg act ggc aag acg agg cag ttt ggg aaa gac atg tcg aat ggt     7307
   Phe Gly Thr Gly Lys Thr Arg Gln Phe Gly Lys Asp Met Ser Asn Gly
       825                 830                 835
   tct tca gta cga agc ctt ctt tgc gga aag aaa atc tta gtg gtt gat     7355
   Ser Ser Val Arg Ser Leu Leu Cys Gly Lys Lys Ile Leu Val Val Asp
   840                 845                 850                 855
   gat aat ttg gtg aac cga agg gtc gcc gcc ggc gcg ttg aaa aac ttt     7403
   Asp Asn Leu Val Asn Arg Arg Val Ala Ala Gly Ala Leu Lys Asn Phe
                   860                 865                 870
   gga gct gat gtc aaa tgt gca gca agt ggc aaa gct gct ctt gaa atg     7451
   Gly Ala Asp Val Lys Cys Ala Ala Ser Gly Lys Ala Ala Leu Glu Met
               875                 880                 885
   ctt caa tat cct cac gat ttc gat gct tgc ttc atg gat att caa atg     7499
   Leu Gln Tyr Pro His Asp Phe Asp Ala Cys Phe Met Asp Ile Gln Met
           890                 895                 900
   cca gaa atg gat gg  gtatgcttac tggcactgac taatacatgt tttttgccaa     7553
   Pro Glu Met Asp Gly
       905
   cttaatatat tactctttca atattcgttg tgttattaga agatcatata gattaattta   7613
   taaattttct tttagcaaaa ccttatcaat taagtgtgta gaaaagtcag tctcacatta   7673
   tggtcaaata agtgttaggg caagcttcac ctcaaagcta gctatttggg tagatttagg   7733
   cctaacccga attctaagat ggtatcagag tctatcctag atctttttat tggaaaccac   7793
   ccgtatatga gcaactcgta gatattcatt cttgaaagtt gcacgctcca tatgtccatt   7853
   cctaggtgcg agagagaagt ctcactttga ctagagatat gattaaaaaa atatttataa   7913
   agggttgagc aatcctcacc tcagagctaa gcttttgggg taaagttagg cctaactcga   7973
   actctaataa agtgtttagc tggtgtgtca actgtcaata tgaaatcttt tgcaatttac   8033
   tatgcattca cttacctact ttattgaagc ttattgacaa tttgtgcaga agcatcatta   8093
   attaggaaca tgttagctat acaagttatg atgtttttgt atagcatatc atgttccaac   8153
   cttccaataa caaaatatgt ggttcaagtg tgagaatata taggttaaac aataaagtat   8213
   tgagttaaca gaaatctaaa cacacgctgt cactagctct tcatattgag acatgcatgg   8273
   gatttgacaa aacatctgaa taaatatttg cag g ttt gag gcc act cgt cga      8325
                                          Phe Glu Ala Thr Arg Arg
                                              910
   att cgg atg atg gaa aga gag gca agt gag cag ctg aaa agt gaa tct     8373
   Ile Arg Met Met Glu Arg Glu Ala Ser Glu Gln Leu Lys Ser Glu Ser
   915                 920                 925                 930
   ggt gaa gaa aat ggt aag aaa agt gag ttc cac atg cct ata ttg gcc     8421
   Gly Glu Glu Asn Gly Lys Lys Ser Glu Phe His Met Pro Ile Leu Ala
                   935                 940                 945
   atg aca gct gat gta atc cat gct aca tat gat aag tgc tta aat tgt     8469
   Met Thr Ala Asp Val Ile His Ala Thr Tyr Asp Lys Cys Leu Asn Cys
               950                 955                 960
   ggg atg gat gga tac gtc tca aag cct ttt gaa gaa gag aat ctc tat     8517
   Gly Met Asp Gly Tyr Val Ser Lys Pro Phe Glu Glu Glu Asn Leu Tyr
           965                 970                 975
   caa gca gtt gca aag ttc ttc aag tcc aag cct gct tca gac tca tg      8564
   Gln Ala Val Ala Lys Phe Phe Lys Ser Lys Pro Ala Ser Asp Ser
       980                 985                 990
   acactgctta ttctgcagaa caggtcaacc aacttttgat tgagaaacat ttagtgttag   8624
   catgtttgga tcaacttctc ccagcatcaa ttctgaaact cagaagctac tcatagaagc   8684
   ttctagccag aattgatttt ggcttcagaa gctactaatg gttttatgta gagagcaaaa   8744
   gttggtttcc aagatatgcg aggatccatg atgatacaca cgtctgaagt gatcatttct   8804
   aaccagttga agtttcactc gacgtgattt gaatccaagt aaatgcatac cacataatta   8864
   tccatcccca tcttgtgtac agattctccc aagggataag aaaatttatg taaattcaat   8924
   ttttttcttt tgcatctcaa tacttccctg ttagaacttt ttccctatga ttatttccac   8984
   ttttcatttt caattatatt ttttgtaaaa ttggtctcca tcctataagg tttgcttagt   9044
   tttttttaat tcagatgaga aggttggtgc ttataatgtg tatacctttt tagcagtact   9104
   tgtgatataa atatttgtct tatttttaga acttaggaga taataagatc gtaggagtaa   9164
   atagcatttt tggagaatga tatcataatt ataatcatct acattctgca gttataaaaa   9224
   aaatcaatat taatgcatag caagtagctt taagaagtat gatctattta agtattgaat   9284
   tttttttcta aactaattac aatgtattat tgctgggata tgcgcttttt actcgataga   9344
   tatcatgttt ggattagttt ttcttagaat ccattttgga accgaaaaaa ttatcacata   9404
   atttattctt caaagttgat tttggcaatc aaggtaaaca ctcataaaag ctaaagggag   9464
   ttgtttctta ttattgtgat tttagctcat tgtggttttt caccgtgtat ataaacatgc   9524
   atttgatgtg acgttgactt gtgaagcacg tggctatcca ccagcaatct caagtaacac   9584
   ttgtcagcac tatgttgcta ttataagtga caaggcagtg accacacccc tctatatatg   9644
   cataaacttt tttattaaaa gtaacagaat caaaagggaa gttttctaca aattaattaa   9704
   tggaaaatta tcagaaccag tatggagctg ttcctgctag cagggctccg gttcagagcc   9764
   agagtgaaat attggaaacc accaatgttg gcggtgctac acaagtcata agaaatgatt   9824
   atgatggaag aaccactgct gcagatgcta ctgttgttgg tgagagcgat ctgcagcatc   9884
   accacaagaa aggaatatta gagaagatca aggagaagct tcctggaaca caccaccatc   9944
   aagatcacaa atagctagct agctttgttt agtttatttt gcttctatca ttataaatgt  10004
   aataataata gtatgtgtgt tgctacatgc atgtgtatgt atcgtttagt cagctagttt  10064
   gttaataact tgctttcaat tttgcttcct tacaagccta tttatgcaat gtatttggtc  10124
   atttcactat tggtttctaa tcaatctaaa ttagtacatg gtcctcttcc ttttagtgcc  10184
   ttttaattaa agagtaaatg ttgaatattg tttttagtca ttctatttac atttttattt  10244
   tttttgcttc atcggaaaat tatttacatt tttattaagt gtgtgtttag attttcgttt  10304
   ttcgttaaaa atttcataat taatttttta taacagtgat gtatgcagct aaggcatcct  10364
   aacagaccta tctttgtatg tatggcattc tgggcgggtc ggatttattt ttagccccat  10424
   aattccccac tactatacat aaaatgatac ctgctagtaa aataatttaa agtgtttcga  10484
   agtcatattg gtaaattgat ttcagttaaa aatcgaagtt tgttagctac taaagacggt  10544
   agcttctatg tcaaaatagt taattcctat attttataat tggatcattt tttctaaaat  10604
   ttattcaaac atctcaactc actttgacta taatcacttt ataattaatt ttgttgaatt  10664
   aaataaatta tattcaaatt aaatgtgata atacccactg aatgtgattg gaatgccatc  10724
   agggaaatat ggaacaaaga gagctgcatg tggaagggct tgttatgtct gcaaaacaga  10784
   caaagaagtt gtaatcacaa ttaggacaat tgccaaagat gtggggtcac attgacttgt  10844
   cagggaaagt ctatagatcc actgtgctct taaagctaga acaattaaca taaaatttct  10904
   gccaattaag gctttcactt cctatcccta tctgctgtat attttaagtt tgagtaaagg  10964
   gacagttgtg gtgatgttat aatcaactgc aggctgaggg aaaagttggg ggtcaagtgg  11024
   tgtctatata aacttttttg tcattattaa tctttaatct ttttggtttt gtaaaatgac  11084
   agcttttgtc ttgtgctgaa agttgactgt gctagctagt aaagtcatgc tggtttctta  11144
   attgattgtg gtgtttttat gcgacaactg tagacattgc aatgcaggca cacaagtcaa  11204
   gaggcaatta caaacaaaat acagctatta ataattgacc tactaactac taaggaggga  11264
   atttaaatgt gattttgaaa tcttatattg gatgtaaaga ggcaatactc atcagactgt  11324
   aaagtgacag aacaagtaga tggtaaacaa gtagaagtag aagcagcaac aaattgagta  11384
   aagaccatca atgtcaacac ttccttggag aagaatttca taagtgtcat gtgaaaataa  11444
   ctagacaaaa tgtagccaaa atgaagtatg aaactcagtt attgaaacga gtgacactga  11504
   ctagattcca agttattgaa ggaaccagca aaaggttaag tgacagtgaa gtgtgtgggt  11564
   tgaaagagga ttgaataaaa gctgaaccaa tggactgaat aggcaaactt gacaatttcc  11624
   cattagaatc tgttttaact tccttgtaca aaagtttctt cggtgaaatt tccagcattg  11684
   ttggtaacat gatgagtagc tcatgaatct ggaaaccaaa cagttttgtt tgttcaagct  11744
   gtagagaaga atgagaacta catgtgagca ttgttatgta ccaaaaaaac ctgtgagcat  11804
   tgttgctgca atcctggatc agtacgcata gcctgagcac aaagatgttg aattgaagta  11864
   gttgctctaa tatgttatga atattcaaga ttaaaacatt tttaagttat catgtgacaa  11924
   tcacataact attaaattat tttaatctta actattaatc attagatcta acgatcatat  11984
   tctcatataa aataattatt ctgatacaat aatatatata tatatatata tgtatgtagg  12044
   ggatctgagg atgtatcata tgagaaatgt gatattatgt gagaacatga gaataaatca  12104
   caaccgttag atttaatcaa cggaccagat taaaacaatt taatgctcac atgaccactt  12164
   aaaaatgtca tgtgactttc ttaaaaagtc aaatgacttc atgttttaat tttgaccgtt  12224
   catgattaga tttaacggtc atgatttatt ctcatgttct cacataaaaa catttttcat  12284
   ttttctgata tgtatatttg taacataaaa cacaacataa aatactatgt aaatcagcca  12344
   atttcaccaa atacaatatg atgtgaaaaa tataatatta ctattgatat tcactcaaat  12404
   gcacaatcgg ccctaaaaaa tggtcaattt ttctggttaa tatatttctt ttttaaaaca  12464
   tgagtattca tgcaatatat gatttcaaaa tatataattt ctttcaacat ttaaccaatt  12524
   tgcaccatgt gtaatatgta agagatcaaa aattatattg agttttattt atcttttaac 12584
   tgatatggga aacattttta tagataattt ttattcaagt tctaagttac ttcattttta 12644
   tttcggcgaa gttggtgctt ttttttcttt tcaaaccgtt tgaagatggt ttcctcatgg 12704
   attggagttt aatccatttt tctatcatga gctc 12738
<210> 5
   <211> 3568
   <212> DNA
   <213> Lotus japonicus
<220>
   <221> 5'UTR
   <222> (1)..(137)
<220>
   <221> CDS
   <222> (138)..(3119)
<220>
   <221> 3'UTR
   <222> (3120)..(3568)
<400> 5
   ttcaactttc aaaacaaagt ggatgggatc ttcatcttat ataaccacac atcaatcatt       60
   tgtgctactt ctccaatttt ctttagagat gaaatgaaga gctaagcaga caagacaagt      120
   ttatttgttt gttgctg atg ggt ctt ggg ttc aag atg cag cag agc cac         170
                      Met Gly Leu Gly Phe Lys Met Gln Gln Ser His
                      1               5                   10
   cac cct gtg gct ttg aag tta cat gag caa gct ggg agc cag aga aag        218
   His Pro Val Ala Leu Lys Leu His Glu Gln Ala Gly Ser Gln Arg Lys
               15                  20                  25
   ttc act ttc att cag aac ttc aga aac tgg ttt cta ccc ctt ctg ttt        266
   Phe Thr Phe Ile Gln Asn Phe Arg Asn Trp Phe Leu Pro Leu Leu Phe
           30                  35                  40
   gta tgg ttc att gtt atg gct gca ttt ggt gcc tgc atc tac cat aaa        314
   Val Trp Phe Ile Val Met Ala Ala Phe Gly Ala Cys Ile Tyr His Lys
       45                  50                  55
   atg gat gct gaa act aaa gtc aga agg aaa gag gtg ctg ggt agc ctc        362
   Met Asp Ala Glu Thr Lys Val Arg Arg Lys Glu Val Leu Gly Ser Leu
   60                  65                  70                  75
   tgt gat caa agg gct aga atg cta caa gac caa ttc agt gtc agt gtc        410
   Cys Asp Gln Arg Ala Arg Met Leu Gln Asp Gln Phe Ser Val Ser Val
                   80                  85                  90
   aac cat gtc cat gcc ctt gcc atc ctt gtt tca acc ttc cat tac tac        458
   Asn His Val His Ala Leu Ala Ile Leu Val Ser Thr Phe His Tyr Tyr
               95                  100                 105
   aga aat act tca gcc att gac cag gaa acc ttt gca gaa tac acg gcc        506
   Arg Asn Thr Ser Ala Ile Asp Gln Glu Thr Phe Ala Glu Tyr Thr Ala
           110                 115                 120
   agg aca gca ttt gaa cgg cca tta atg agt ggg gtg gcc tat gca cag       554
   Arg Thr Ala Phe Glu Arg Pro Leu Met Ser Gly Val Ala Tyr Ala Gln
       125                 130                 135
   aga gtg gtt cac tca gag aga gaa aga ttt gag aag caa cat ggg tgg       602
   Arg Val Val His Ser Glu Arg Glu Arg Phe Glu Lys Gln His Gly Trp
   140                 145                 150                 155
   gtt ata aag aca atg gaa aga gtg cct tca ggg gtt agg gat gag tat       650
   Val Ile Lys Thr Met Glu Arg Val Pro Ser Gly Val Arg Asp Glu Tyr
                   160                 165                 170
   gca gca gtg ata ttt gca cag gaa act gtc tct tac ctt gaa tct att       698
   Ala Ala Val Ile Phe Ala Gln Glu Thr Val Ser Tyr Leu Glu Ser Ile
               175                 180                 185
   gat atg atg tct ggg gag gag gac cga gag aac att ttg agg gct aga       746
   Asp Met Met Ser Gly Glu Glu Asp Arg Glu Asn Ile Leu Arg Ala Arg
           190                 195                 200
   gcc act ggg aaa gct gtt ctg act agc cct ttc aga ctg ctg gat tct       794
   Ala Thr Gly Lys Ala Val Leu Thr Ser Pro Phe Arg Leu Leu Asp Ser
       205                 210                 215
   cat cat ctt ggc gtg gtt cta aca ttt cct gtt tat aaa tct aag ctc       842
   His His Leu Gly Val Val Leu Thr Phe Pro Val Tyr Lys Ser Lys Leu
   220                 225                 230                 235
   cct cca gag cca acg acg gaa gag gtc att aaa gcc ata gca gga tat       890
   Pro Pro Glu Pro Thr Thr Glu Glu Val Ile Lys Ala Ile Ala Gly Tyr
                   240                 245                 250
   att gga gga tcc ttt gat gtt gag tcc ctt gtg gag aat tta ttt ggt       938
   Ile Gly Gly Ser Phe Asp Val Glu Ser Leu Val Glu Asn Leu Phe Gly
               255                 260                 265
   caa ctt gct ggt aac caa gca att ttg gtg aag gta tat gat ata aca       986
   Gln Leu Ala Gly Asn Gln Ala Ile Leu Val Lys Val Tyr Asp Ile Thr
           270                 275                 280
   aac tct agc gac ccc cta atc atg tat ggc agc caa tat gaa gag ggt      1034
   Asn Ser Ser Asp Pro Leu Ile Met Tyr Gly Ser Gln Tyr Glu Glu Gly
       285                 290                 295
   gat atg tct ctt gtc cat gaa agt aag ctt gat ttt gga gat cca tac      1082
   Asp Met Ser Leu Val His Glu Ser Lys Leu Asp Phe Gly Asp Pro Tyr
   300                 305                 310                 315
   agg aaa cat cac atg atc tgt aga tat cac caa cag gca cca aca aat      1130
   Arg Lys His His Met Ile Cys Arg Tyr His Gln Gln Ala Pro Thr Asn
                   320                 325                 330
   tgg ata gca tat acc acg gca ttc cta ttc ttt gtg att ctt tgt tta      1178
   Trp Ile Ala Tyr Thr Thr Ala Phe Leu Phe Phe Val Ile Leu Cys Leu
               335                 340                 345
   gtg ggt tac att tta tat gct gct gga act cac att gtc aag gta gaa      1226
   Val Gly Tyr Ile Leu Tyr Ala Ala Gly Thr His Ile Val Lys Val Glu
           350                 355                 360
   gat gat tac aat gca atg cag gat tta aaa gtc aaa gca gaa gca gct      1274
   Asp Asp Tyr Asn Ala Met Gln Asp Leu Lys Val Lys Ala Glu Ala Ala
       365                 370                 375
   gat att gcc aag tca cag ttt cta gct acc gtc tct cat gaa att aga      1322
   Asp Ile Ala Lys Ser Gln Phe Leu Ala Thr Val Ser His Glu Ile Arg
   380                 385                 390                 395
   act ccc atg aat gga att tta gga atg ctt ggt ctg ctt tta cgc aca      1370
   Thr Pro Met Asn Gly Ile Leu Gly Met Leu Gly Leu Leu Leu Arg Thr
                   400                 405                 410
   gaa ttg agt tca aca caa aga gac tat gct cag act gct caa gca tgt      1418
   Glu Leu Ser Ser Thr Gln Arg Asp Tyr Ala Gln Thr Ala Gln Ala Cys
               415                 420                 425
   ggg aag gca cta ata gca tta ata aat gag gtg ctt gac cga gct aaa      1466
   Gly Lys Ala Leu Ile Ala Leu Ile Asn Glu Val Leu Asp Arg Ala Lys
           430                 435                 440
   att gaa gca ggc aaa tta gag cta gaa gca gtt cca ttt gac ctt cgt      1514
   Ile Glu Ala Gly Lys Leu Glu Leu Glu Ala Val Pro Phe Asp Leu Arg
       445                 450                 455
   tcc ata ctt gac gat gtc ctt tct ctt ttt tct gag aag tca aga cac      1562
   Ser Ile Leu Asp Asp Val Leu Ser Leu Phe Ser Glu Lys Ser Arg His
   460                 465                 470                 475
   aaa ggc tta gag ctg gca gtg ttt gtt tct gac aaa gtt ccg gat ata      1610
   Lys Gly Leu Glu Leu Ala Val Phe Val Ser Asp Lys Val Pro Asp Ile
                   480                 485                 490
   gtt atg ggc gat cct ggg cga ttc aga caa ata gtg aca aat ctt gtt      1658
   Val Met Gly Asp Pro Gly Arg Phe Arg Gln Ile Val Thr Asn Leu Val
               495                 500                 505
   gga aac tct gtt aag ttc act gag cga ggt cat ata ttt gtt aaa gtc      1706
   Gly Asn Ser Val Lys Phe Thr Glu Arg Gly His Ile Phe Val Lys Val
           510                 515                 520
   cat tta gct gaa aaa aga cag tgc aca atg aat gga aaa tgt gag act      1754
   His Leu Ala Glu Lys Arg Gln Cys Thr Met Asn Gly Lys Cys Glu Thr
       525                 530                 535
   ttt cta aat gga ggc tgt gat gat gtt ttg cat gta tct ggc agt tat      1802
   Phe Leu Asn Gly Gly Cys Asp Asp Val Leu His Val Ser Gly Ser Tyr
   540                 545                 550                 555
   aat ttg aaa acc ctt agt gga tat gaa gcc gct gat gaa cgg aac agc      1850
   Asn Leu Lys Thr Leu Ser Gly Tyr Glu Ala Ala Asp Glu Arg Asn Ser
                   560                 565                 570
   tgg gat aat ttt aag cat cat att gct gac gaa gaa ttt ttc ttt gat      1898
   Trp Asp Asn Phe Lys His His Ile Ala Asp Glu Glu Phe Phe Phe Asp
               575                 580                 585
   gct tcg gtt aaa aag ttg gcc tct agt gaa tct tat gag caa gtc acc      1946
   Ala Ser Val Lys Lys Leu Ala Ser Ser Glu Ser Tyr Glu Gln Val Thr
           590                 595                 600
   ttg atg gtc agc gtg gag gac act gga att ggg att tct ttc tct gcc      1994
   Leu Met Val Ser Val Glu Asp Thr Gly Ile Gly Ile Ser Phe Ser Ala
       605                 610                 615
   caa gat agt att ttc atg cct ttt gtg cag gct gac agc tca acc tct      2042
   Gln Asp Ser Ile Phe Met Pro Phe Val Gln Ala Asp Ser Ser Thr Ser
   620                 625                 630                 635
   cga aac tat ggg ggt acc ggg atc ggc ttg agt atc agt aag tgc ttg      2090
   Arg Asn Tyr Gly Gly Thr Gly Ile Gly Leu Ser Ile Ser Lys Cys Leu
                   640                 645                 650
   gtt gaa ctg atg ggc ggt cag ata aac ttc ata agc cga ccc cag gtc      2138
   Val Glu Leu Met Gly Gly Gln Ile Asn Phe Ile Ser Arg Pro Gln Val
               655                 660                 665
   ggg agc acg ttt tca ttc act gca gat ttc gga aca ttt aag aaa aac      2186
   Gly Ser Thr Phe Ser Phe Thr Ala Asp Phe Gly Thr Phe Lys Lys Asn
           670                 675                 680
   tca aca act gac atg aag aaa ctt aac ttt gaa gat cta cct tct agt      2234
   Ser Thr Thr Asp Met Lys Lys Leu Asn Phe Glu Asp Leu Pro Ser Ser
       685                 690                 695
   ttt aga ggt ctt aaa gcc att gtg gtt gat gga aaa cct gtt aga gct      2282
   Phe Arg Gly Leu Lys Ala Ile Val Val Asp Gly Lys Pro Val Arg Ala
   700                 705                 710                 715
   gca gtg act aga tac cat ttg aag aga cta ggg ata caa gct aaa gtt      2330
   Ala Val Thr Arg Tyr His Leu Lys Arg Leu Gly Ile Gln Ala Lys Val
                   720                 725                 730
   gca att agc atc aat aag gct gtt tct tta tgt ggg aaa aat ggt tct      2378
   Ala Ile Ser Ile Asn Lys Ala Val Ser Leu Cys Gly Lys Asn Gly Ser
               735                 740                 745
   ttg acc tcg gca tta ttt cag cct gat att att ttt gtt gag aag gac      2426
   Leu Thr Ser Ala Leu Phe Gln Pro Asp Ile Ile Phe Val Glu Lys Asp
           750                 755                 760
   tct tgg gtt tct gga gag gat ggt ggt atc ttc aat gcg ttt aag atg      2474
   Ser Trp Val Ser Gly Glu Asp Gly Gly Ile Phe Asn Ala Phe Lys Met
       765                 770                 775
   cct caa atg atc ctt ctt gca acc aat atc tgt aac gct gaa ttt gat      2522
   Pro Gln Met Ile Leu Leu Ala Thr Asn Ile Cys Asn Ala Glu Phe Asp
   780                 785                 790                 795
   aaa gcc aaa gct gca ggt ttc agt gat aca gtg atc atg aag cca ctg      2570
   Lys Ala Lys Ala Ala Gly Phe Ser Asp Thr Val Ile Met Lys Pro Leu
                   800                 805                 810
   aga gct agt atg ctg gct gct tgt ctt cag caa gtt ttc ggg act ggc      2618
   Arg Ala Ser Met Leu Ala Ala Cys Leu Gln Gln Val Phe Gly Thr Gly
               815                 820                 825
   aag acg agg cag ttt ggg aaa gac atg tcg aat ggt tct tca gta cga      2666
   Lys Thr Arg Gln Phe Gly Lys Asp Met Ser Asn Gly Ser Ser Val Arg
           830                 835                 840
   agc ctt ctt tgc gga aag aaa atc tta gtg gtt gat gat aat ttg gtg      2714
   Ser Leu Leu Cys Gly Lys Lys Ile Leu Val Val Asp Asp Asn Leu Val
       845                 850                 855
   aac cga agg gtc gcc gcc ggc gcg ttg aaa aac ttt gga gct gat gtc      2762
   Asn Arg Arg Val Ala Ala Gly Ala Leu Lys Asn Phe Gly Ala Asp Val
   860                 865                 870                 875
   aaa tgt gca gca agt ggc aaa gct gct ctt gaa atg ctt caa tat cct      2810
   Lys Cys Ala Ala Ser Gly Lys Ala Ala Leu Glu Met Leu Gln Tyr Pro
                   880                 885                 890
   cac gat ttc gat gct tgc ttc atg gat att caa atg cca gaa atg gat      2858
   His Asp Phe Asp Ala Cys Phe Met Asp Ile Gln Met Pro Glu Met Asp
               895                 900                 905
   ggg ttt gag gcc act cgt cga att cgg atg atg gaa aga gag gca agt      2906
   Gly Phe Glu Ala Thr Arg Arg Ile Arg Met Met Glu Arg Glu Ala Ser
           910                 915                 920
   gag cag ctg aaa agt gaa tct ggt gaa gaa aat ggt aag aaa agt gag      2954
   Glu Gln Leu Lys Ser Glu Ser Gly Glu Glu Asn Gly Lys Lys Ser Glu
       925                 930                 935
   ttc cac atg cct ata ttg gcc atg aca gct gat gta atc cat gct aca      3002
   Phe His Met Pro Ile Leu Ala Met Thr Ala Asp Val Ile His Ala Thr
   940                 945                 950                 955
   tat gat aag tgc tta aat tgt ggg atg gat gga tac gtc tca aag cct      3050
   Tyr Asp Lys Cys Leu Asn Cys Gly Met Asp Gly Tyr Val Ser Lys Pro
                   960                 965                 970
   ttt gaa gaa gag aat ctc tat caa gca gtt gca aag ttc ttc aag tcc      3098
   Phe Glu Glu Glu Asn Leu Tyr Gln Ala Val Ala Lys Phe Phe Lys Ser
               975                 980                 985
   aag cct gct tca gac tca tga cactgcttat tctgcagaac aggtcaacca         3149
   Lys Pro Ala Ser Asp Ser
           990
   acttttgatt gagaaacatt tagtgttagc atgtttggat caacttctcc cagcatcaat   3209
   tctgaaactc agaagctact catagaagct tctagccaga attgattttg gcttcagaag   3269
   ctactaatgg ttttatgtag agagcaaaag ttggtttcca agatatgcga ggatccatga   3329
   tgatacacac gtctgaagtg atcatttcta accagttgaa gtttcactcg acgtgatttg   3389
   aatccaagta aatgcatacc acataattat ccatccccat cttgtgtaca gattctccca   3449
   agggataaga aaatttatgt aaattcaatt tttttctttt gcatctcaat acttccctgt   3509
   tagaactttt tccctatgat tatttccact tttcattttc aattatattt tttgtaaaa    3568
<210> 6
   <211> 993
   <212> PRT
   <213> Lotus japonicus
<400> 6
   Met Gly Leu Gly Phe Lys Met Gln Gln Ser His His Pro Val Ala Leu
   1               5                   10                  15
   Lys Leu His Glu Gln Ala Gly Ser Gln Arg Lys Phe Thr Phe Ile Gln
               20                  25                  30
   Asn Phe Arg Asn Trp Phe Leu Pro Leu Leu Phe Val Trp Phe Ile Val
           35                  40                  45
   Met Ala Ala Phe Gly Ala Cys Ile Tyr His Lys Met Asp Ala Glu Thr
       50                  55                  60
   Lys Val Arg Arg Lys Glu Val Leu Gly Ser Leu Cys Asp Gln Arg Ala
   65                  70                  75                  80
   Arg Met Leu Gln Asp Gln Phe Ser Val Ser Val Asn His Val His Ala
                   85                  90                  95
   Leu Ala Ile Leu Val Ser Thr Phe His Tyr Tyr Arg Asn Thr Ser Ala
               100                 105                 110
   Ile Asp Gln Glu Thr Phe Ala Glu Tyr Thr Ala Arg Thr Ala Phe Glu
           115                 120                 125
   Arg Pro Leu Met Ser Gly Val Ala Tyr Ala Gln Arg Val Val His Ser
       130                 135                 140
   Glu Arg Glu Arg Phe Glu Lys Gln His Gly Trp Val Ile Lys Thr Met
   145                 150                 155                 160
   Glu Arg Val Pro Ser Gly Val Arg Asp Glu Tyr Ala Ala Val Ile Phe
                   165                 170                 175
   Ala Gln Glu Thr Val Ser Tyr Leu Glu Ser Ile Asp Met Met Ser Gly
               180                 185                 190
   Glu Glu Asp Arg Glu Asn Ile Leu Arg Ala Arg Ala Thr Gly Lys Ala
           195                 200                 205
   Val Leu Thr Ser Pro Phe Arg Leu Leu Asp Ser His His Leu Gly Val
       210                 215                 220
   Val Leu Thr Phe Pro Val Tyr Lys Ser Lys Leu Pro Pro Glu Pro Thr
   225                 230                 235                 240
   Thr Glu Glu Val Ile Lys Ala Ile Ala Gly Tyr Ile Gly Gly Ser Phe
                   245                 250                 255
   Asp Val Glu Ser Leu Val Glu Asn Leu Phe Gly Gln Leu Ala Gly Asn
               260                 265                 270
   Gln Ala Ile Leu Val Lys Val Tyr Asp Ile Thr Asn Ser Ser Asp Pro
           275                 280                 285
   Leu Ile Met Tyr Gly Ser Gln Tyr Glu Glu Gly Asp Met Ser Leu Val
       290                 295                 300
   His Glu Ser Lys Leu Asp Phe Gly Asp Pro Tyr Arg Lys His His Met
   305                 310                 315                 320
   Ile Cys Arg Tyr His Gln Gln Ala Pro Thr Asn Trp Ile Ala Tyr Thr
                   325                 330                 335
   Thr Ala Phe Leu Phe Phe Val Ile Leu Cys Leu Val Gly Tyr Ile Leu
               340                 345                 350
   Tyr Ala Ala Gly Thr His Ile Val Lys Val Glu Asp Asp Tyr Asn Ala
           355                 360                 365
   Met Gln Asp Leu Lys Val Lys Ala Glu Ala Ala Asp Ile Ala Lys Ser
       370                 375                 380
   Gln Phe Leu Ala Thr Val Ser His Glu Ile Arg Thr Pro Met Asn Gly
   385                 390                 395                 400
   Ile Leu Gly Met Leu Gly Leu Leu Leu Arg Thr Glu Leu Ser Ser Thr
                   405                 410                 415
   Gln Arg Asp Tyr Ala Gln Thr Ala Gln Ala Cys Gly Lys Ala Leu Ile
               420                 425                 430
   Ala Leu Ile Asn Glu Val Leu Asp Arg Ala Lys Ile Glu Ala Gly Lys
           435                 440                 445
   Leu Glu Leu Glu Ala Val Pro Phe Asp Leu Arg Ser Ile Leu Asp Asp
       450                 455                 460
   Val Leu Ser Leu Phe Ser Glu Lys Ser Arg His Lys Gly Leu Glu Leu
   465                 470                 475                 480
   Ala Val Phe Val Ser Asp Lys Val Pro Asp Ile Val Met Gly Asp Pro
   485 490 495
   Gly Arg Phe Arg Gln Ile Val Thr Asn Leu Val Gly Asn Ser Val Lys
   500 505 510
   Phe Thr Glu Arg Gly His Ile Phe Val Lys Val His Leu Ala Glu Lys
   515 520 525
   Arg Gln Cys Thr Met Asn Gly Lys Cys Glu Thr Phe Leu Asn Gly Gly
   530 535 540
   Cys Asp Asp Val Leu His Val Ser Gly Ser Tyr Asn Leu Lys Thr Leu
   545 550 555 560
   Ser Gly Tyr Glu Ala Ala Asp Glu Arg Asn Ser Trp Asp Asn Phe Lys
   565 570 575
   His His Ile Ala Asp Glu Glu Phe Phe Phe Asp Ala Ser Val Lys Lys
   580 585 590
   Leu Ala Ser Ser Glu Ser Tyr Glu Gln Val Thr Leu Met Val Ser Val
   595 600 605
   Glu Asp Thr Gly Ile Gly Ile Ser Phe Ser Ala Gln Asp Ser Ile Phe
   610 615 620
   Met Pro Phe Val Gln Ala Asp Ser Ser Thr Ser Arg Asn Tyr Gly Gly
   625 630 635 640
   Thr Gly Ile Gly Leu Ser Ile Ser Lys Cys Leu Val Glu Leu Met Gly
   645 650 655
   Gly Gln Ile Asn Phe Ile Ser Arg Pro Gln Val Gly Ser Thr Phe Ser
   660 665 670
   Phe Thr Ala Asp Phe Gly Thr Phe Lys Lys Asn Ser Thr Thr Asp Met
   675 680 685
   Lys Lys Leu Asn Phe Glu Asp Leu Pro Ser Ser Phe Arg Gly Leu Lys
   690 695 700
   Ala Ile Val Val Asp Gly Lys Pro Val Arg Ala Ala Val Thr Arg Tyr
   705 710 715 720
   His Leu Lys Arg Leu Gly Ile Gln Ala Lys Val Ala Ile Ser Ile Asn
   725 730 735
   Lys Ala Val Ser Leu Cys Gly Lys Asn Gly Ser Leu Thr Ser Ala Leu
   740 745 750
   Phe Gln Pro Asp Ile Ile Phe Val Glu Lys Asp Ser Trp Val Ser Gly
   755 760 765
   Glu Asp Gly Gly Ile Phe Asn Ala Phe Lys Met Pro Gln Met Ile Leu
   770 775 780
   Leu Ala Thr Asn Ile Cys Asn Ala Glu Phe Asp Lys Ala Lys Ala Ala
   785 790 795 800
   Gly Phe Ser Asp Thr Val Ile Met Lys Pro Leu Arg Ala Ser Met Leu
   805 810 815
   Ala Ala Cys Leu Gln Gln Val Phe Gly Thr Gly Lys Thr Arg Gln Phe
   820 825 830
   Gly Lys Asp Met Ser Asn Gly Ser Ser Val Arg Ser Leu Leu Cys Gly
   835 840 845
   Lys Lys Ile Leu Val Val Asp Asp Asn Leu Val Asn Arg Arg Val Ala
   850 855 860
   Ala Gly Ala Leu Lys Asn Phe Gly Ala Asp Val Lys Cys Ala Ala Ser
   865 870 875 880
   Gly Lys Ala Ala Leu Glu Met Leu Gln Tyr Pro His Asp Phe Asp Ala
   885 890 895
   Cys Phe Met Asp Ile Gln Met Pro Glu Met Asp Gly Phe Glu Ala Thr
   900 905 910
   Arg Arg Ile Arg Met Met Glu Arg Glu Ala Ser Glu Gln Leu Lys Ser
   915 920 925
   Glu Ser Gly Glu Glu Asn Gly Lys Lys Ser Glu Phe His Met Pro Ile
   930 935 940
   Leu Ala Met Thr Ala Asp Val Ile His Ala Thr Tyr Asp Lys Cys Leu
   945 950 955 960
   Asn Cys Gly Met Asp Gly Tyr Val Ser Lys Pro Phe Glu Glu Glu Asn
   965 970 975
   Leu Tyr Gln Ala Val Ala Lys Phe Phe Lys Ser Lys Pro Ala Ser Asp
   980 985 990
   Ser
<210> 7
   <211> 4743
   <212> DNA
   <213> Medicago truncatula
<220>
   <221> exon
   <222> (1) .. (348)
<220>
   <221> Intron
   <222> (349)..(462)
<220>
   <221> exon
   <222> (463)..(696)
<220>
   <221> Intron
   <222> (697)..(799)
<220>
   <221> exon
   <222> (800)..(969)
<220>
   <221> Intron
   <222> (970)..(1261)
<220>
   <221> exon
   <222> (1262)..(1480)
   <223> mutated codon encoding L/F substitution
<220>
   <221> Intron
   <222> (1481)..(1574)
<220>
   <221> exon
   <222> (1575)..(1761)
<220>
   <221> Intron
   <222> (1762)..(1857)
<220>
   <221> exon
   <222> (1858)..(1909)
<220>
   <221> Intron
   <222> (1910)..(2018)
<220>
   <221> exon
   <222> (2019)..(2248)
<220>
   <221> Intron
   <222> (2249)..(2383)
<220>
   <221> exon
   <222> (2384)..(2482)
<220>
   <221> Intron
   <222> (2483)..(2624)
<220>
   <221> exon
   <222> (2625)..(3340)
<220>
   <221> Intron
   <222> (3341)..(3597)
<220>
   <221> exon
   <222> (3598)..(4101)
<220>
   <221> Intron
   <222> (4102)..(4490)
<220>
   <221> exon
   <222> (4491)..(4743)
<400> 7
<210> 8
   <211> 3694
   <212> DNA
   <213> Arabidopsis thaliana
<220>
   <221> 5'UTR
   <222> (1)..(68)
<220>
   <221> CDS
   <222> (69)..(3311)
<220>
   <221> 3'UTR
   <222> (3312)..(3694)
<220>
   <221> misc_feature
   <222> (3594)..(3594)
   <223> n is a, c, g, or t
<220>
   <221> misc_feature
   <222> (3631)..(3631)
   <223> n is a, c, g, or t
<400> 8
   aaaaaatctc actaaaacaa aagaagaaga aagaagaaag aaaatggaat acctacattt      60
   ttgaagtg atg aga aga gat ttt gtg tat aat aat aat gca atg ttc aat      110
            Met Arg Arg Asp Phe Val Tyr Asn Asn Asn Ala Met Phe Asn
            1               5                   10
   cct ctc aca act cat tac agc tca gat atg aac tgg gca ctc aac aat      158
   Pro Leu Thr Thr His Tyr Ser Ser Asp Met Asn Trp Ala Leu Asn Asn
   15                  20                  25                  30
   cat caa gaa gaa gaa gaa gag cca cga aga att gaa att tct gat tcc      206
   His Gln Glu Glu Glu Glu Glu Pro Arg Arg Ile Glu Ile Ser Asp Ser
                   35                  40                  45
   gag tca cta gaa aac ttg aaa agc agc gat ttt tat caa ctg ggt ggt      254
   Glu Ser Leu Glu Asn Leu Lys Ser Ser Asp Phe Tyr Gln Leu Gly Gly
               50                  55                  60
   ggt ggt gct ctg aat tcg tca gaa aag ccg aga aag atc gat ttt tgg      302
   Gly Gly Ala Leu Asn Ser Ser Glu Lys Pro Arg Lys Ile Asp Phe Trp
           65                  70                  75
   cgt tcg ggg ttg atg ggt ttt gcg aag atg cag cag cag caa cag ctt      350
   Arg Ser Gly Leu Met Gly Phe Ala Lys Met Gln Gln Gln Gln Gln Leu
       80                  85                  90
   cag cat tca gtg gcg gtg aag atg aac aat aat aat aat aac gat cta      398
   Gln His Ser Val Ala Val Lys Met Asn Asn Asn Asn Asn Asn Asp Leu
   95                  100                 105                 110
   atg ggt aat aaa aaa ggg tca act ttc ata caa gaa cat cga gca ttg      446
   Met Gly Asn Lys Lys Gly Ser Thr Phe Ile Gln Glu His Arg Ala Leu
                   115                 120                 125
   tta cca aaa gct ttg att ctg tgg atc atc att gtt ggg ttt ata agc      494
   Leu Pro Lys Ala Leu Ile Leu Trp Ile Ile Ile Val Gly Phe Ile Ser
               130                 135                 140
   agt ggg att tat cag tgg atg gat gat gct aat aag att aga agg gaa      542
   Ser Gly Ile Tyr Gln Trp Met Asp Asp Ala Asn Lys Ile Arg Arg Glu
           145                 150                 155
   gag gtt ttg gtc agc atg tgt gat caa aga gct aga atg ttg cag gat      590
   Glu Val Leu Val Ser Met Cys Asp Gln Arg Ala Arg Met Leu Gln Asp
       160                 165                 170
   caa ttt agt gtt agt gtt aat cat gtt cat gct ttg gct att ctc gtc      638
   Gln Phe Ser Val Ser Val Asn His Val His Ala Leu Ala Ile Leu Val
   175                 180                 185                 190
   tcc act ttt cat tac cac aag aac cct tct gca att gat cag gag aca      686
   Ser Thr Phe His Tyr His Lys Asn Pro Ser Ala Ile Asp Gln Glu Thr
                   195                 200                 205
   ttt gcg gag tac acg gca aga aca gca ttt gag aga ccg ttg cta agt      734
   Phe Ala Glu Tyr Thr Ala Arg Thr Ala Phe Glu Arg Pro Leu Leu Ser
               210                 215                 220
   gga gtg gct tat gct gaa aaa gtt gtg aat ttt gag agg gag atg ttt      782
   Gly Val Ala Tyr Ala Glu Lys Val Val Asn Phe Glu Arg Glu Met Phe
           225                 230                 235
   gag cgg cag cac aat tgg gtt ata aag aca atg gat aga gga gag cct      830
   Glu Arg Gln His Asn Trp Val Ile Lys Thr Met Asp Arg Gly Glu Pro
       240                 245                 250
   tca ccg gtt agg gat gag tat gct cct gtt ata ttc tct caa gat agt      878
   Ser Pro Val Arg Asp Glu Tyr Ala Pro Val Ile Phe Ser Gln Asp Ser
   255                 260                 265                 270
   gtc tct tac ctt gag tca ctc gat atg atg tca ggc gag gag gat cgt      926
   Val Ser Tyr Leu Glu Ser Leu Asp Met Met Ser Gly Glu Glu Asp Arg
                   275                 280                 285
   gag aat att ttg cga gct aga gaa acc gga aaa gct gtc ttg act agc      974
   Glu Asn Ile Leu Arg Ala Arg Glu Thr Gly Lys Ala Val Leu Thr Ser
               290                 295                 300
   cct ttt agg ttg ttg gaa act cac cat ctc gga gtt gtg ttg aca ttc     1022
   Pro Phe Arg Leu Leu Glu Thr His His Leu Gly Val Val Leu Thr Phe
           305                 310                 315
   cct gtc tac aag tct tct ctt cct gaa aat ccg act gtc gaa gag cgt     1070
   Pro Val Tyr Lys Ser Ser Leu Pro Glu Asn Pro Thr Val Glu Glu Arg
       320                 325                 330
   att gca gcc act gca ggg tac ctt ggt ggt gcg ttt gat gtg gag tct     1118
   Ile Ala Ala Thr Ala Gly Tyr Leu Gly Gly Ala Phe Asp Val Glu Ser
   335                 340                 345                 350
   cta gtc gag aat tta ttt ggt cag ctt gct ggt aac caa gca ata gtt      1166
   Leu Val Glu Asn Leu Phe Gly Gln Leu Ala Gly Asn Gln Ala Ile Val
                   355                 360                 365
   gtg cat gtg tat gat atc acc aat gca tca gat cca ctt gtc atg tat      1214
   Val His Val Tyr Asp Ile Thr Asn Ala Ser Asp Pro Leu Val Met Tyr
               370                 375                 380
   ggt aat caa gat gaa gaa gcc gac aga tct ctc tct cat gag agc aag      1262
   Gly Asn Gln Asp Glu Glu Ala Asp Arg Ser Leu Ser His Glu Ser Lys
           385                 390                 395
   ctc gat ttt gga gac ccc ttc agg aaa cat aag atg ata tgc agg tac      1310
   Leu Asp Phe Gly Asp Pro Phe Arg Lys His Lys Met Ile Cys Arg Tyr
       400                 405                 410
   cac caa aag gca cca ata cca ttg aat gtg ctc aca act gtg cca ttg      1358
   His Gln Lys Ala Pro Ile Pro Leu Asn Val Leu Thr Thr Val Pro Leu
   415                 420                 425                 430
   ttc ttt gcg att ggt ttc ttg gtg ggt tat ata ctg tat ggt gca gct      1406
   Phe Phe Ala Ile Gly Phe Leu Val Gly Tyr Ile Leu Tyr Gly Ala Ala
                   435                 440                 445
   atg cac ata gta aaa gtc gaa gat gat ttc cat gaa atg caa gag ctt      1454
   Met His Ile Val Lys Val Glu Asp Asp Phe His Glu Met Gln Glu Leu
               450                 455                 460
   aaa gtg cga gca gaa gct gct gat gtc gct aaa tcg cag ttt ctt gct      1502
   Lys Val Arg Ala Glu Ala Ala Asp Val Ala Lys Ser Gln Phe Leu Ala
           465                 470                 475
   acc gtg tct cac gag atc agg aca cca atg aat ggc att ctc gga atg      1550
   Thr Val Ser His Glu Ile Arg Thr Pro Met Asn Gly Ile Leu Gly Met
       480                 485                 490
   ctt gct atg ctc cta gat aca gaa cta agc tcg aca cag aga gat tac      1598
   Leu Ala Met Leu Leu Asp Thr Glu Leu Ser Ser Thr Gln Arg Asp Tyr
   495                 500                 505                 510
   gct caa acc gct caa gta tgt ggt aaa gct ttg att gca ttg ata aat      1646
   Ala Gln Thr Ala Gln Val Cys Gly Lys Ala Leu Ile Ala Leu Ile Asn
                   515                 520                 525
   gag gtt ctt gat cgc gcc aag att gaa gct gga aag ctg gag ttg gaa      1694
   Glu Val Leu Asp Arg Ala Lys Ile Glu Ala Gly Lys Leu Glu Leu Glu
               530                 535                 540
   tca gta cca ttt gat atc cgt tca ata ttg gat gat gtc ctt tct cta      1742
   Ser Val Pro Phe Asp Ile Arg Ser Ile Leu Asp Asp Val Leu Ser Leu
           545                 550                 555
   ttc tct gag gag tca agg aac aaa ggc att gag ctc gcg gtt ttc gtt      1790
   Phe Ser Glu Glu Ser Arg Asn Lys Gly Ile Glu Leu Ala Val Phe Val
       560                 565                 570
   tca gac aaa gta cca gag ata gtc aaa gga gat tca ggg aga ttt aga      1838
   Ser Asp Lys Val Pro Glu Ile Val Lys Gly Asp Ser Gly Arg Phe Arg
   575                 580                 585                 590
   cag ata atc ata aac ctt gtt gga aat tcg gtt aaa ttc aca gag aaa      1886
   Gln Ile Ile Ile Asn Leu Val Gly Asn Ser Val Lys Phe Thr Glu Lys
                   595                 600                 605
   gga cat atc ttt gtt aaa gtc cat ctt gcg gaa caa tca aaa gat gaa      1934
   Gly His Ile Phe Val Lys Val His Leu Ala Glu Gln Ser Lys Asp Glu
               610                 615                 620
   tct gaa ccg aaa aat gca ttg aat ggt gga gtg tct gaa gaa atg atc      1982
   Ser Glu Pro Lys Asn Ala Leu Asn Gly Gly Val Ser Glu Glu Met Ile
           625                 630                 635
   gtt gtt tcc aaa cag tca agt tac aac aca ttg agc ggt tac gaa gct      2030
   Val Val Ser Lys Gln Ser Ser Tyr Asn Thr Leu Ser Gly Tyr Glu Ala
       640                 645                 650
   gct gat ggt cgg aat agc tgg gat tca ttc aag cat ttg gtc tct gag      2078
   Ala Asp Gly Arg Asn Ser Trp Asp Ser Phe Lys His Leu Val Ser Glu
   655                 660                 665                 670
   gag cag tca tta tcg gag ttt gat att tct agc aat gtt agg ctt atg      2126
   Glu Gln Ser Leu Ser Glu Phe Asp Ile Ser Ser Asn Val Arg Leu Met
                   675                 680                 685
   gtt tca atc gaa gac acg ggt att gga atc cct tta gtt gca caa ggc      2174
   Val Ser Ile Glu Asp Thr Gly Ile Gly Ile Pro Leu Val Ala Gln Gly
               690                 695                 700
   cgt gtg ttt atg ccg ttt atg caa gca gat agc tcg act tca aga aac      2222
   Arg Val Phe Met Pro Phe Met Gln Ala Asp Ser Ser Thr Ser Arg Asn
           705                 710                 715
   tat gga ggt act ggt att ggt ttg agt ata agc aag tgt ctt gtt gaa      2270
   Tyr Gly Gly Thr Gly Ile Gly Leu Ser Ile Ser Lys Cys Leu Val Glu
       720                 725                 730
   ctt atg cgt ggt cag ata aat ttc ata agc cgg cct cat att gga agc      2318
   Leu Met Arg Gly Gln Ile Asn Phe Ile Ser Arg Pro His Ile Gly Ser
   735                 740                 745                 750
   acg ttc tgg ttc acg gct gtt tta gag aaa tgc gat aaa tgc agt gcg      2366
   Thr Phe Trp Phe Thr Ala Val Leu Glu Lys Cys Asp Lys Cys Ser Ala
                   755                 760                 765
   att aac cat atg aag aaa cct aat gtg gaa cac ttg cct tct act ttt      2414
   Ile Asn His Met Lys Lys Pro Asn Val Glu His Leu Pro Ser Thr Phe
               770                 775                 780
   aaa gga atg aaa gct ata gtt gtt gat gct aag cct gtt aga gct gct      2462
   Lys Gly Met Lys Ala Ile Val Val Asp Ala Lys Pro Val Arg Ala Ala
           785                 790                 795
   gtg act aga tac cat atg aaa aga ctc gga atc aat gtt gat gtc gtg      2510
   Val Thr Arg Tyr His Met Lys Arg Leu Gly Ile Asn Val Asp Val Val
       800                 805                 810
   aca agt ctc aaa acc gct gtt gtt gca gct gct gcg ttt gaa aga aac      2558
   Thr Ser Leu Lys Thr Ala Val Val Ala Ala Ala Ala Phe Glu Arg Asn
   815                 820                 825                 830
   ggt tct cct ctc cca aca aaa ccg caa ctt gat atg atc tta gta gag     2606
   Gly Ser Pro Leu Pro Thr Lys Pro Gln Leu Asp Met Ile Leu Val Glu
                   835                 840                 845
   aaa gat tca tgg att tca act gaa gat aat gac tca gag att cgt tta     2654
   Lys Asp Ser Trp Ile Ser Thr Glu Asp Asn Asp Ser Glu Ile Arg Leu
               850                 855                 860
   ttg aat tca aga acc aac gga aac gtt cat cac aag tct ccg aaa cta     2702
   Leu Asn Ser Arg Thr Asn Gly Asn Val His His Lys Ser Pro Lys Leu
           865                 870                 875
   gct cta ttc gca aca aac atc aca aat tcg gag ttc gac aga gct aaa     2750
   Ala Leu Phe Ala Thr Asn Ile Thr Asn Ser Glu Phe Asp Arg Ala Lys
       880                 885                 890
   tcc gca gga ttt gca gat acg gta ata atg aaa ccg tta aga gca agc     2798
   Ser Ala Gly Phe Ala Asp Thr Val Ile Met Lys Pro Leu Arg Ala Ser
   895                 900                 905                 910
   atg att ggg gcg tgt ctg caa caa gtt ctc gag ctg aga aaa aca aga     2846
   Met Ile Gly Ala Cys Leu Gln Gln Val Leu Glu Leu Arg Lys Thr Arg
                   915                 920                 925
   caa caa cat cca gaa gga tca tca ccc gca act ctc aag agc ttg ctt     2894
   Gln Gln His Pro Glu Gly Ser Ser Pro Ala Thr Leu Lys Ser Leu Leu
               930                 935                 940
   aca ggg aag aag att ctt gtg gtt gat gat aat ata gtt aac agg aga     2942
   Thr Gly Lys Lys Ile Leu Val Val Asp Asp Asn Ile Val Asn Arg Arg
           945                 950                 955
   gta gct gca gga gct ctc aag aaa ttt gga gca gaa gtg gtt tgt gca     2990
   Val Ala Ala Gly Ala Leu Lys Lys Phe Gly Ala Glu Val Val Cys Ala
       960                 965                 970
   gag agt ggt caa gtt gct ttg ggt ttg ctt cag att cca cac act ttc     3038
   Glu Ser Gly Gln Val Ala Leu Gly Leu Leu Gln Ile Pro His Thr Phe
   975                 980                 985                 990
   gat gct tgc ttc atg gat att caa atg cca  cag atg gac gga ttt  gaa   3086
   Asp Ala Cys Phe Met Asp Ile Gln Met Pro  Gln Met Asp Gly Phe  Glu
                   995                 1000                1005
   gca act cgt cag  ata aga atg atg gag  aag gaa gct aaa gag  aag      3131
   Ala Thr Arg Gln  Ile Arg Met Met Glu  Lys Glu Ala Lys Glu  Lys
               1010                 1015                 1020
   acg aat ctc gaa  tgg cat tta ccg att  cta gcg atg act gcg  gat      3176
   Thr Asn Leu Glu  Trp His Leu Pro Ile  Leu Ala Met Thr Ala  Asp
               1025                 1030                 1035
   gtg ata cac gcg  acc tac gag gaa tgt  ctg aaa agt ggg atg  gat      3221
   Val Ile His Ala  Thr Tyr Glu Glu Cys  Leu Lys Ser Gly Met  Asp
               1040                 1045                 1050
   ggt tac gtc tcc  aaa cct ttt gaa gaa  gag aat ctc tat aaa  tcc      3266
   Gly Tyr Val Ser  Lys Pro Phe Glu Glu  Glu Asn Leu Tyr Lys  Ser
               1055                 1060                 1065
   gtt gcc aaa tca  ttc aaa cct aat cct  atc tca cct tcg tcg  taa      3311
   Val Ala Lys Ser  Phe Lys Pro Asn Pro  Ile Ser Pro Ser Ser
             1070                1075                1080
   tccaatcttc cggcgagttt ttttctctcc gcagccggaa gaatggactg cttctgctga   3371
   ttgattggga taaatatgca ttttggtttc tgtacatata gtaggttcac aatctagaga   3431
   ttttgaaggt tttttttaat ttctcactta agtaatgtag cttgccatga ctagtgtatg   3491
   ttgttaaacg acgacgtcta agatggttca gtgttgatct tagcgtaagt attaatccca   3551
   tgggaatcgg ttgtactgta tcagatttgg ttagtcgttt aancattgta atgttctaat   3611
   aatcactttt tcatatatan cctcatgtta taacatgaga cgagaccatt ttgattaaaa   3671
   aaaaaaaaaa aaaaaaaaaa aaa                                           3694
<210> 9
   <211> 1080
   <212> PRT
   <213> Arabidopsis thaliana
<400> 9
   Met Arg Arg Asp Phe Val Tyr Asn Asn Asn Ala Met Phe Asn Pro Leu
   1               5                   10                  15
   Thr Thr His Tyr Ser Ser Asp Met Asn Trp Ala Leu Asn Asn His Gln
               20                  25                  30
   Glu Glu Glu Glu Glu Pro Arg Arg Ile Glu Ile Ser Asp Ser Glu Ser
           35                  40                  45
   Leu Glu Asn Leu Lys Ser Ser Asp Phe Tyr Gln Leu Gly Gly Gly Gly
       50                  55                  60
   Ala Leu Asn Ser Ser Glu Lys Pro Arg Lys Ile Asp Phe Trp Arg Ser
   65                  70                  75                  80
   Gly Leu Met Gly Phe Ala Lys Met Gln Gln Gln Gln Gln Leu Gln His
                   85                  90                  95
   Ser Val Ala Val Lys Met Asn Asn Asn Asn Asn Asn Asp Leu Met Gly
               100                 105                 110
   Asn Lys Lys Gly Ser Thr Phe Ile Gln Glu His Arg Ala Leu Leu Pro
           115                 120                 125
   Lys Ala Leu Ile Leu Trp Ile Ile Ile Val Gly Phe Ile Ser Ser Gly
       130                 135                 140
   Ile Tyr Gln Trp Met Asp Asp Ala Asn Lys Ile Arg Arg Glu Glu Val
   145                 150                 155                 160
   Leu Val Ser Met Cys Asp Gln Arg Ala Arg Met Leu Gln Asp Gln Phe
                   165                 170                 175
   Ser Val Ser Val Asn His Val His Ala Leu Ala Ile Leu Val Ser Thr
               180                 185                 190
   Phe His Tyr His Lys Asn Pro Ser Ala Ile Asp Gln Glu Thr Phe Ala
           195                 200                 205
   Glu Tyr Thr Ala Arg Thr Ala Phe Glu Arg Pro Leu Leu Ser Gly Val
       210                 215                 220
   Ala Tyr Ala Glu Lys Val Val Asn Phe Glu Arg Glu Met Phe Glu Arg
   225                 230                 235                 240
   Gln His Asn Trp Val Ile Lys Thr Met Asp Arg Gly Glu Pro Ser Pro
                   245                 250                 255
   Val Arg Asp Glu Tyr Ala Pro Val Ile Phe Ser Gln Asp Ser Val Ser
               260                 265                 270
   Tyr Leu Glu Ser Leu Asp Met Met Ser Gly Glu Glu Asp Arg Glu Asn
           275                 280                 285
   Ile Leu Arg Ala Arg Glu Thr Gly Lys Ala Val Leu Thr Ser Pro Phe
       290                 295                 300
   Arg Leu Leu Glu Thr His His Leu Gly Val Val Leu Thr Phe Pro Val
   305                 310                 315                 320
   Tyr Lys Ser Ser Leu Pro Glu Asn Pro Thr Val Glu Glu Arg Ile Ala
                   325                 330                 335
   Ala Thr Ala Gly Tyr Leu Gly Gly Ala Phe Asp Val Glu Ser Leu Val
               340                 345                 350
   Glu Asn Leu Phe Gly Gln Leu Ala Gly Asn Gln Ala Ile Val Val His
           355                 360                 365
   Val Tyr Asp Ile Thr Asn Ala Ser Asp Pro Leu Val Met Tyr Gly Asn
       370                 375                 380
   Gln Asp Glu Glu Ala Asp Arg Ser Leu Ser His Glu Ser Lys Leu Asp
   385                 390                 395                 400
   Phe Gly Asp Pro Phe Arg Lys His Lys Met Ile Cys Arg Tyr His Gln
                   405                 410                 415
   Lys Ala Pro Ile Pro Leu Asn Val Leu Thr Thr Val Pro Leu Phe Phe
               420                 425                 430
   Ala Ile Gly Phe Leu Val Gly Tyr Ile Leu Tyr Gly Ala Ala Met His
           435                 440                 445
   Ile Val Lys Val Glu Asp Asp Phe His Glu Met Gln Glu Leu Lys Val
       450                 455                 460
   Arg Ala Glu Ala Ala Asp Val Ala Lys Ser Gln Phe Leu Ala Thr Val
   465                 470                 475                 480
   Ser His Glu Ile Arg Thr Pro Met Asn Gly Ile Leu Gly Met Leu Ala
                   485                 490                 495
   Met Leu Leu Asp Thr Glu Leu Ser Ser Thr Gln Arg Asp Tyr Ala Gln
               500                 505                 510
   Thr Ala Gln Val Cys Gly Lys Ala Leu Ile Ala Leu Ile Asn Glu Val
           515                 520                 525
   Leu Asp Arg Ala Lys Ile Glu Ala Gly Lys Leu Glu Leu Glu Ser Val
       530                 535                 540
   Pro Phe Asp Ile Arg Ser Ile Leu Asp Asp Val Leu Ser Leu Phe Ser
   545                 550                 555                 560
   Glu Glu Ser Arg Asn Lys Gly Ile Glu Leu Ala Val Phe Val Ser Asp
                   565                 570                 575
   Lys Val Pro Glu Ile Val Lys Gly Asp Ser Gly Arg Phe Arg Gln Ile
               580                 585                 590
   Ile Ile Asn Leu Val Gly Asn Ser Val Lys Phe Thr Glu Lys Gly His
           595                 600                 605
   Ile Phe Val Lys Val His Leu Ala Glu Gln Ser Lys Asp Glu Ser Glu
       610                 615                 620
   Pro Lys Asn Ala Leu Asn Gly Gly Val Ser Glu Glu Met Ile Val Val
   625                 630                 635                 640
   Ser Lys Gln Ser Ser Tyr Asn Thr Leu Ser Gly Tyr Glu Ala Ala Asp
   645 650 655
   Gly Arg Asn Ser Trp Asp Ser Phe Lys His Leu Val Ser Glu Glu Gln
   660 665 670
   Ser Leu Ser Glu Phe Asp Ile Ser Ser Asn Val Arg Leu Met Val Ser
   675 680 685
   Ile Glu Asp Thr Gly Ile Gly Ile Pro Leu Val Ala Gln Gly Arg Val
   690 695 700
   Phe Met Pro Phe Met Gln Ala Asp Ser Ser Thr Ser Arg Asn Tyr Gly
   705 710 715 720
   Gly Thr Gly Ile Gly Leu Ser Ile Ser Lys Cys Leu Val Glu Leu Met
   725 730 735
   Arg Gly Gln Ile Asn Phe Ile Ser Arg Pro His Ile Gly Ser Thr Phe
   740 745 750
   Trp Phe Thr Ala Val Leu Glu Lys Cys Asp Lys Cys Ser Ala Ile Asn
   755 760 765
   His Met Lys Lys Pro Asn Val Glu His Leu Pro Ser Thr Phe Lys Gly
   770 775 780
   Met Lys Ala Ile Val Val Asp Ala Lys Pro Val Arg Ala Ala Val Thr
   785 790 795 800
   Arg Tyr His Met Lys Arg Leu Gly Ile Asn Val Asp Val Val Thr Ser
   805 810 815
   Leu Lys Thr Ala Val Val Ala Ala Ala Ala Phe Glu Arg Asn Gly Ser
   820 825 830
   Pro Leu Pro Thr Lys Pro Gln Leu Asp Met Ile Leu Val Glu Lys Asp
   835 840 845
   Ser Trp Ile Ser Thr Glu Asp Asn Asp Ser Glu Ile Arg Leu Leu Asn
   850 855 860
   Ser Arg Thr Asn Gly Asn Val His His Lys Ser Pro Lys Leu Ala Leu
   865 870 875 880
   Phe Ala Thr Asn Ile Thr Asn Ser Glu Phe Asp Arg Ala Lys Ser Ala
                   885                     890                     895
   Gly Phe Ala Asp Thr Val Ile Met Lys Pro Leu Arg Ala Ser Met Ile
               900                     905                     910
   Gly Ala Cys Leu Gln Gln Val Leu Glu Leu Arg Lys Thr Arg Gln Gln
           915                     920                     925
   His Pro Glu Gly Ser Ser Pro Ala Thr Leu Lys Ser Leu Leu Thr Gly
       930                     935                     940
   Lys Lys Ile Leu Val Val Asp Asp Asn Ile Val Asn Arg Arg Val Ala
   945                     950                     955                     960
   Ala Gly Ala Leu Lys Lys Phe Gly Ala Glu Val Val Cys Ala Glu Ser
                   965                     970                     975
   Gly Gln Val Ala Leu Gly Leu Leu Gln Ile Pro His Thr Phe Asp Ala
               980                     985                     990
   Cys Phe Met Asp Ile Gln Met Pro  Gln Met Asp Gly Phe  Glu Ala Thr
           995                     1000                     1005
   Arg Gln  Ile Arg Met Met Glu  Lys Glu Ala Lys Glu  Lys Thr Asn
       1010                     1015                     1020
   Leu Glu  Trp His Leu Pro Ile  Leu Ala Met Thr Ala  Asp Val Ile
       1025                     1030                     1035
   His Ala  Thr Tyr Glu Glu Cys  Leu Lys Ser Gly Met  Asp Gly Tyr
       1040                     1045                     1050
   Val Ser  Lys Pro Phe Glu Glu  Glu Asn Leu Tyr Lys  Ser Val Ala
       1055                     1060                     1065
   Lys Ser  Phe Lys Pro Asn Pro  Ile Ser Pro Ser Ser
       1070                     1075                     1080
<210> 10
   <211> 5474
   <212> DNA
   <213> Oryza (rice)
<220>
   <221> exon
   <222> (1)..(348)
<220>
   <221> Intron
   <222> (349)..(711)
<220>
   <221> exon
   <222> (712)..(945)
<220>
   <221> Intron
   <222> (946)..(1040)
<220>
   <221> exon
   <222> (1041)..(1207)
<220>
   <221> Intron
   <222> (1208)..(1508)
<220>
   <221> exon
   <222> (1509)..(1727)
<220>
   <221> Intron
   <222> (1728)..(1818)
<220>
   <221> exon
   <222> (1819)..(2005)
<220>
   <221> Intron
   <222> (2006)..(2087)
<220>
   <221> exon
   <222> (2088)..(2139)
<220>
   <221> Intron
   <222> (2140)..(2233)
<220>
   <221> exon
   <222> (2234)..(2463)
<220>
   <221> Intron
   <222> (2464)..(2694)
<220>
   <221> exon
   <222> (2695)..(2793)
<220>
   <221> Intron
   <222> (2794)..(2910)
<220>
   <221> exon
   <222> (2911)..(3641)
<220>
   <221> Intron
   <222> (3642)..(4079)
<220>
   <221> exon
   <222> (4080)..(4595)
<220>
   <221> Intron
   <222> (4596) .. (5215)
<220>
   <221> exon
   <222> (5216)..(5474)
<400> 10
   atg ggt gtg gga gga ggc gga gga gga gga gga ggg gag gcg gcg gcg        48
   Met Gly Val Gly Gly Gly Gly Gly Gly Gly Gly Gly Glu Ala Ala Ala
   1               5                   10                  15
   gcg gtg gcg gtg gag ggg gat gag gcg ggg aag ggg agg agg tgg tgg        96
   Ala Val Ala Val Glu Gly Asp Glu Ala Gly Lys Gly Arg Arg Trp Trp
               20                  25                  30
   agg gtg aag gtg aag ctg agc acg gtg gcg gtg gtg gcg tgg gtg ctg       144
   Arg Val Lys Val Lys Leu Ser Thr Val Ala Val Val Ala Trp Val Leu
           35                  40                  45
   gcg tcg gcg gcg ctc tgg gcg ggg ctg cac tgg cgc ttc cgc cgc gcg       192
   Ala Ser Ala Ala Leu Trp Ala Gly Leu His Trp Arg Phe Arg Arg Ala
       50                  55                  60
   gcg ctg cac aag gcc gag gag gcc ctc gtc tgc atg tgc gag gag cgc       240
   Ala Leu His Lys Ala Glu Glu Ala Leu Val Cys Met Cys Glu Glu Arg
   65                  70                  75                  80
   gcc cgc atg ctg cag gac cag ttc gcc gtc tcc gtc aac cac gtc cac       288
   Ala Arg Met Leu Gln Asp Gln Phe Ala Val Ser Val Asn His Val His
                   85                  90                  95
   gcc ctc gcc atc ctc gtc gcc acc ttc cac tac gac aag cac cct ccc       336
   Ala Leu Ala Ile Leu Val Ala Thr Phe His Tyr Asp Lys His Pro Pro
               100                 105                 110
   gcc ctc gac cag gtcggcccga actccgacga gctcttccgc cgccgccgcg           388
   Ala Leu Asp Gln
           115
   atgatcctgt tgcatctgtt gtttttttgc cccccgcggt taattgcgat aatgcctcga     448
   tttttactcc acatcttgcc cgtgtacttc gctctgctgc ttcttcggct tcatttaatt     508
   ctaccgtgac cttccgtgtc agccatggaa gccatggatt actgttgctg tctcttgcta     568
   ttatatggag cgcacttttt gttgggaggg agtgaattga ttgtgcttgc ttgcttctgt     628
   tggtagtagt actagtgatt tctttggctg tgtggctgat gaatcttctt cgatgtgttg    688
   tgcgtgcgtg cgcgtgtgtg cag gac acg ttc gcc gtg tac gcc gcg agg acg    741
                             Asp Thr Phe Ala Val Tyr Ala Ala Arg Thr
                                         120                 125
   tcc ttc gag cgg ccg ctg ctg agc ggc gtg gcg tac gcg cag cgg gtg      789
   Ser Phe Glu Arg Pro Leu Leu Ser Gly Val Ala Tyr Ala Gln Arg Val
               130                 135                 140
   gtg cac gcc gac agg gag agc ttc gag cgg cag cag ggg tgg atc atc      837
   Val His Ala Asp Arg Glu Ser Phe Glu Arg Gln Gln Gly Trp Ile Ile
           145                 150                 155
   aag acc atg aag cac gag ccg tcc ccg gcg cag gac gag tac gcc ccg      885
   Lys Thr Met Lys His Glu Pro Ser Pro Ala Gln Asp Glu Tyr Ala Pro
       160                 165                 170
   gtg atc tac tcc cag gag acc atc tcc tac atc gag ggc ctc gac gtc      933
   Val Ile Tyr Ser Gln Glu Thr Ile Ser Tyr Ile Glu Gly Leu Asp Val
   175                 180                 185                 190
   atg tcc ggc gag gtgcgtttct tgggttacag cttcgcagct gctgctgcgg          985
   Met Ser Gly Glu
   ttatcgccat gtccgctgct ctgaactgtg ctgctgggtg tgcttcggcc tgcag gag    1043
                                                                Glu
                                                                195
   gac agg gag aac atc ttg agg gcg agg gcg aca ggg aag gcc gtc ctc     1091
   Asp Arg Glu Asn Ile Leu Arg Ala Arg Ala Thr Gly Lys Ala Val Leu
                   200                 205                 210
   acg agg ccg ttc cgg ctg atg tcg aat cac ttg ggt gtt gtc ttg acg     1139
   Thr Arg Pro Phe Arg Leu Met Ser Asn His Leu Gly Val Val Leu Thr
               215                 220                 225
   ttt cct gtc tac ctc gtc gat ctc cca aat gat acc gcg gtg gag gat     1187
   Phe Pro Val Tyr Leu Val Asp Leu Pro Asn Asp Thr Ala Val Glu Asp
           230                 235                 240
   cgt gtt gct gct act gca gg  gtgagggatt actttacttt tctgaatgaa        1237
   Arg Val Ala Ala Thr Ala Gly
       245
   gattattctc tccaactgat tcctcttctg tctggaatcc actgccttca gctcttcgtt   1297
   ttgttgcagt cgttgttgga tgcttttagt agtggaaatg tgtgcgtttc agggatattt   1357
   gatcacatgc aacattttca ctcataactg gctgaaaaag ttttgcatta atagagctga   1417
   aatgtctaga tggataagca attgcagtgg tattttaagt acaacatgtg caacgaatgg   1477
   ctccatttaa ctttttcttt ttgttcggca g a tac ctt gga gga gca ttt gat    1530
                                        Tyr Leu Gly Gly Ala Phe Asp
                                                        255
   gtg gag tca cta gta gaa aat ttg ttt aga cag tta gct ggt aac cag     1578
   Val Glu Ser Leu Val Glu Asn Leu Phe Arg Gln Leu Ala Gly Asn Gln
           260                 265                 270
   gaa ttg gtg gtc aat gtt tat gat gtc aca aac cac tca aac cct ctt      1626
   Glu Leu Val Val Asn Val Tyr Asp Val Thr Asn His Ser Asn Pro Leu
       275                 280                 285
   gtg atg tat gga tct gag gtt cct ctt ggt att ccc tca cca tca cac      1674
   Val Met Tyr Gly Ser Glu Val Pro Leu Gly Ile Pro Ser Pro Ser His
   290                 295                 300                 305
   acc tat acg ttg gat ttt ggt gat cca ttg aga aag cat cag atg gtt      1722
   Thr Tyr Thr Leu Asp Phe Gly Asp Pro Leu Arg Lys His Gln Met Val
                   310                 315                 320
   tgc ag  gtaaatttgt gtgaattgat cgttggtttt cccattttat attatagaac       1777
   Cys Arg
   gatcggtttt tttaacatcc attggccata aatctgagca g a tac aga aac aag      1831
                                                   Tyr Arg Asn Lys
                                                       325
   ctt cat gtt tca tgg tct gca att act aca cca tca ggg gtc ttt gtt      1879
   Leu His Val Ser Trp Ser Ala Ile Thr Thr Pro Ser Gly Val Phe Val
           330                 335                 340
   ata tgt atg ctg gtg ggc tac ata ata tat gct gct tgg agt cgc tac      1927
   Ile Cys Met Leu Val Gly Tyr Ile Ile Tyr Ala Ala Trp Ser Arg Tyr
       345                 350                 355
   gat aat gtt aag gaa gat tgc cgg aaa atg gaa gcg ctg aaa aaa cgg      1975
   Asp Asn Val Lys Glu Asp Cys Arg Lys Met Glu Ala Leu Lys Lys Arg
   360                 365                 370                 375
   gca gaa gcg gct gat att gct aaa tct cag gtatagttgg atgttgtttg        2025
   Ala Glu Ala Ala Asp Ile Ala Lys Ser Gln
                   380                 385
   cttctctatt tctattgcaa gcttattgtt atatctaaaa ggttcttatt catttatgac   2085
   ag ttc ctt gca act gtt tct cat gag atc aga aca ccc atg aat ggc       2132
      Phe Leu Ala Thr Val Ser His Glu Ile Arg Thr Pro Met Asn Gly
                      390                 395                 400
   gtg ctg g gtattttctt tgatcttaca acacattcag tttaatgtta tgcaactcat     2189
   Val Leu
   ttcttttgaa aaaatggaat catctctttg tttcttttcc ctag ga  atg ctt gat     2244
                                                   Gly Met Leu Asp
                                                           405
   atg tta tta gac aca gag ctg aag tca acc cag agg gat tat gca caa      2292
   Met Leu Leu Asp Thr Glu Leu Lys Ser Thr Gln Arg Asp Tyr Ala Gln
               410                 415                 420
   aca gcc caa gtc tgt gga aag gca tta ata tcc ctg att aac gaa gtg      2340
   Thr Ala Gln Val Cys Gly Lys Ala Leu Ile Ser Leu Ile Asn Glu Val
           425                 430                 435
   ctt gac agg gcc aaa atc gag gct ggc aag ata gat ctc gag tca gta      2388
   Leu Asp Arg Ala Lys Ile Glu Ala Gly Lys Ile Asp Leu Glu Ser Val
       440                 445                 450
   cca ttt gac ctg agg tcc atc ctt gat gat gtc atc tcg tta ttt tct      2436
   Pro Phe Asp Leu Arg Ser Ile Leu Asp Asp Val Ile Ser Leu Phe Ser
   455                 460                 465                 470
   tca aaa tca aga gag aaa gga att gag gttagttaaa ctgatttcgg           2483
   Ser Lys Ser Arg Glu Lys Gly Ile Glu
                   475
   tcatggttgg acaaagatca ctaaacgtat taagtttctg ccagccatca attatttctt   2543
   ttaggaaaat atcatgcact agttccaccg acatctttta gtctcttagc ttgatactct   2603
   ttccatgaac ttctctgcat taccgtcatg caccatgcac gtttaacttt gtttaatccc   2663
   agttgatttt cttctatgtt gtaacttcca g ctt gct gta tat gtt tct gaa      2715
                                      Leu Ala Val Tyr Val Ser Glu
                                      480                 485
   aga gtt cct gaa atc ctg ttg ggc gac cct gga agg ttt cgt cag ata      2763
   Arg Val Pro Glu Ile Leu Leu Gly Asp Pro Gly Arg Phe Arg Gln Ile
               490                 495                 500
   att aca aac ttg gtg gga aac tcg atc aag gtaaatgcgc ataacctttg       2813
   Ile Thr Asn Leu Val Gly Asn Ser Ile Lys
           505                 510
   tatccattca tgattttctt taacgatacc aatagttctc accaatgaca tcaggcaact   2873
   tgtttcttag tatactattg ttcaatgtga acacaag ata aca ata ttt acc ttg    2928
                                            Ile Thr Ile Phe Thr Leu
                                                    515
   tcg cag ttc aca gaa cgg ggg cac att ttt gta caa gtt cac ctg gca      2976
   Ser Gln Phe Thr Glu Arg Gly His Ile Phe Val Gln Val His Leu Ala
       520                 525                 530
   gat cac tca aat ctt gca aca gaa gca aaa att gaa cca gta gtc aat      3024
   Asp His Ser Asn Leu Ala Thr Glu Ala Lys Ile Glu Pro Val Val Asn
   535                 540                 545                 550
   ggg atg aat gga cat aaa gac gag gct att gct ata ccc acc agt ggg      3072
   Gly Met Asn Gly His Lys Asp Glu Ala Ile Ala Ile Pro Thr Ser Gly
                   555                 560                 565
   tct cat aac act tta agt ggt ttt gaa gca gct gat agc cga aat aac      3120
   Ser His Asn Thr Leu Ser Gly Phe Glu Ala Ala Asp Ser Arg Asn Asn
               570                 575                 580
   tgg gaa aac ttc aag ctt ttg ctc tct tac gag aaa aat gaa atg cca      3168
   Trp Glu Asn Phe Lys Leu Leu Leu Ser Tyr Glu Lys Asn Glu Met Pro
           585                 590                 595
   tat gaa agt gat tct gat aaa gta act ctt gtt gtt agt gtg gaa gat      3216
   Tyr Glu Ser Asp Ser Asp Lys Val Thr Leu Val Val Ser Val Glu Asp
       600                 605                 610
   act ggg ata ggc ata cca ctg cat gcc caa ggc cgg gtc ttc acg cct      3264
   Thr Gly Ile Gly Ile Pro Leu His Ala Gln Gly Arg Val Phe Thr Pro
   615                 620                 625                 630
   ttc atg caa gct gac agc tca act tct agg aac tat ggt gga act ggc     3312
   Phe Met Gln Ala Asp Ser Ser Thr Ser Arg Asn Tyr Gly Gly Thr Gly
                   635                 640                 645
   att gga ttg agc atc agc aaa tgt ctt gtt gaa ata atg ggt ggt cag     3360
   Ile Gly Leu Ser Ile Ser Lys Cys Leu Val Glu Ile Met Gly Gly Gln
               650                 655                 660
   ata aac ttt gtc agc cga cct ctt gtt ggg agc aca ttc aca ttc act     3408
   Ile Asn Phe Val Ser Arg Pro Leu Val Gly Ser Thr Phe Thr Phe Thr
           665                 670                 675
   gct gtt ctg aga agg tgt gac aaa aat gct att agt gac agt aag act     3456
   Ala Val Leu Arg Arg Cys Asp Lys Asn Ala Ile Ser Asp Ser Lys Thr
       680                 685                 690
   gtt gct ttg cac cca tta ccg tcc agt ttt aaa ggc tta tct gcg cta     3504
   Val Ala Leu His Pro Leu Pro Ser Ser Phe Lys Gly Leu Ser Ala Leu
   695                 700                 705                 710
   ttg gtt gat aaa aga cct gta aga gca act gtg act aag tat cat ttg     3552
   Leu Val Asp Lys Arg Pro Val Arg Ala Thr Val Thr Lys Tyr His Leu
                   715                 720                 725
   caa agg ctt gga atc act tct gaa gtt gtt ggt acc att gat ccg aca     3600
   Gln Arg Leu Gly Ile Thr Ser Glu Val Val Gly Thr Ile Asp Pro Thr
               730                 735                 740
   ttt ggt gtg ttg tct ggg aga aat ggc agt tct cta acc ag              3641
   Phe Gly Val Leu Ser Gly Arg Asn Gly Ser Ser Leu Thr Ser
           745                 750                 755
   gtacttctat cttctacatt cctttcaaaa aattgaaatc ctggagttaa taggctactt   3701
   ttctctggaa attagaataa acggagcatg cttgcatact aacttcttat gcaaatatca   3761
   ctgttctatg tataaatgat tacacgataa tgattgtttt ttggtaagta ttactgggta   3821
   atacttggcc atcatagttc ttgcctttat tttgtatcac ttcggtatat tgctacttct   3881
   gcggcagttc tctttacgct accgaatgtg atatatttaa ttgaaaattg attttatttt   3941
   aatctgtgaa aagaacattt ttttaaggcc ccacaattct caaataacta gtaagctgat   4001
   tgcagatgat atttgaacta tcttgacacc cagttttttt ttttaatcta actcgttttg   4061
   ttccaaattt gttgtcag c att ggt aag aag cag cca tgc atg ttg cta atc   4113
                         Ile Gly Lys Lys Gln Pro Cys Met Leu Leu Ile
                                     760                 765
   gag agt gat tcc tgg gga cca cag atg gat gtc tcc tta cat gct aga     4161
   Glu Ser Asp Ser Trp Gly Pro Gln Met Asp Val Ser Leu His Ala Arg
           770                 775                 780
   ctt cag gag atg aaa cag agt gat cgc ata cat gta ttg ccc aag gtt     4209
   Leu Gln Glu Met Lys Gln Ser Asp Arg Ile His Val Leu Pro Lys Val
       785                 790                 795
   ttc ctt ctt tct gct gca gaa tca gac aaa gta aag aag ata cat gca     4257
   Phe Leu Leu Ser Ala Ala Glu Ser Asp Lys Val Lys Lys Ile His Ala
   800                 805                 810                 815
   gtt gat tct gtg ata cca aag cct ctg aaa gca agt gca ctt gcg gcc      4305
   Val Asp Ser Val Ile Pro Lys Pro Leu Lys Ala Ser Ala Leu Ala Ala
                   820                 825                 830
   tgt ctg ttc caa gca ctt ggt atc aca cag ccg agc cat gag aaa cgt      4353
   Cys Leu Phe Gln Ala Leu Gly Ile Thr Gln Pro Ser His Glu Lys Arg
               835                 840                 845
   gac gat tca ggt tct ctt cat ggg cgt gat ggt tca ggt tct ctt cat      4401
   Asp Asp Ser Gly Ser Leu His Gly Arg Asp Gly Ser Gly Ser Leu His
           850                 855                 860
   ggg ttg ctt ctt ggc aag aac ata ttg gta gtt gat gac aac aag gta      4449
   Gly Leu Leu Leu Gly Lys Asn Ile Leu Val Val Asp Asp Asn Lys Val
       865                 870                 875
   aac ctc aga gtg gcc gct ggt aca ttg aag aaa tat ggg gca aag gtg      4497
   Asn Leu Arg Val Ala Ala Gly Thr Leu Lys Lys Tyr Gly Ala Lys Val
   880                 885                 890                 895
   gag tgt gtg gag agt gga aaa gat gct ctt tcc ctt cta caa gtg ccg      4545
   Glu Cys Val Glu Ser Gly Lys Asp Ala Leu Ser Leu Leu Gln Val Pro
                   900                 905                 910
   cac aag ttt gat ctg tgt ctc atg gac att cag atg ccg gag atg gat      4593
   His Lys Phe Asp Leu Cys Leu Met Asp Ile Gln Met Pro Glu Met Asp
               915                 920                 925
   gg  gtaagcttat gtcccgttca agatttattt cttttgaatt tgagcctttt           4645
   Gly
   atttcatatg atccaaagcg tgacatgtca tctggataag tgccacctcg ttgactatca   4705
   atttattcac gcaatgaatc aggctttctg cctctttttg aagaaaaaaa aaatgttatt    4765
   cacacttcgt ggttaatgga aacaagcata tacattgctg acgccaaata ctgattataa   4825
   ctagtaaaaa tgtcagtctt gtgttagttt tttatgtgcg aaaaatgccg tgtctaattc   4885
   attatgtgaa aattttcaaa gacattagcc ctgatatcgt cctgcattat gccaaacagt   4945
   actttaaata ttaatatcag aaggtctaaa atccgtgcag ttaccatttg ttcattgtag   5005
   ttttaaccat catactgcaa tatgcagttc ttgggctaat gaacataatg gtgtgctaac   5065
   accctcatga ccaattaact tatgtctctt gaacacttgc tgcatattga catctctgtt   5125
   cctatatttt ctgaatagta accaagtaat gtcaaaccat gtcattattt tgtcttcgtt   5185
   taatcaacat agttttgctt catgtgctag a ttt gag gca act cgg caa ata      5237
                                      Phe Glu Ala Thr Arg Gln Ile
                                          930                 935
   cga gca atg gaa ggg aag gca aat gag cag gca gac gac agc gaa tcg      5285
   Arg Ala Met Glu Gly Lys Ala Asn Glu Gln Ala Asp Asp Ser Glu Ser
                   940                 945                 950
   ggt tca gaa atc gca gca aag acg gcc aaa tgg cac ttg cca atc ctg      5333
   Gly Ser Glu Ile Ala Ala Lys Thr Ala Lys Trp His Leu Pro Ile Leu
               955                 960                 965
   gca atg acc gct gat gtc atc cag gcc acc cac gag gaa tgc aca aag      5381
   Ala Met Thr Ala Asp Val Ile Gln Ala Thr His Glu Glu Cys Thr Lys
           970                 975                 980
   tgc ggg atg gat ggc tac gtc tcg aag ccc ttt gag gag aag cag ctc      5429
   Cys Gly Met Asp Gly Tyr Val Ser Lys Pro Phe Glu Glu Lys Gln Leu
       985                 990                 995
   ttc  cag gca gta cag aag  ttc ttg ggc cca tgc  gtt tcc agc tga       5474
   Phe  Gln Ala Val Gln Lys  Phe Leu Gly Pro Cys  Val Ser Ser
   1000                 1005                 1010
<210> 11
   <211> 3046
   <212> DNA
   <213> Zea mays
<220>
   <221> 5'UTR
   <222> (1)..(15)
<220>
   <221> CDS
   <222> (16) .. (3003)
<220>
   <221> 3'UTR
   <222> (3004)..(3046)
<400> 11
   ggggtggagc ctggg atg ggg gtg gga ggc gga ggc gga ggg gag gcc gct        51
                    Met Gly Val Gly Gly Gly Gly Gly Gly Glu Ala Ala
                    1               5                   10
   gcg gtg tcg gcg ccg gcg ccg gcg gag gag gcg ggg aag gac gcg gag         99
   Ala Val Ser Ala Pro Ala Pro Ala Glu Glu Ala Gly Lys Asp Ala Glu
           15                  20                  25
   gat ggc ggc ggc tgg acc ttg aag gcg aag ctg atc gcc gta gcg gtg        147
   Asp Gly Gly Gly Trp Thr Leu Lys Ala Lys Leu Ile Ala Val Ala Val
       30                  35                  40
   ctg gtg tgg gtg ctg ggg gcc ttg gcg ctc ggg gtg ttc ctg cac tcc        195
   Leu Val Trp Val Leu Gly Ala Leu Ala Leu Gly Val Phe Leu His Ser
   45                  50                  55                  60
   tac ttc cgc cac gcg gcg ctg cgc aag gcg gag gaa ggg ctc gtc agc        243
   Tyr Phe Arg His Ala Ala Leu Arg Lys Ala Glu Glu Gly Leu Val Ser
                   65                  70                  75
   atg tgc gag gag cgc gcg cgc atg ctg cag gac cag ttc gcc gtc tcc        291
   Met Cys Glu Glu Arg Ala Arg Met Leu Gln Asp Gln Phe Ala Val Ser
               80                  85                  90
   gtc aac cac gtc cac gcc ctc gcc atc ctc gtc gcc acc ttc cac tac        339
   Val Asn His Val His Ala Leu Ala Ile Leu Val Ala Thr Phe His Tyr
           95                  100                 105
   gag aag cgc ccg ccc gcg ctc gac cag aac acg ttc gcg gac tac acc      387
   Glu Lys Arg Pro Pro Ala Leu Asp Gln Asn Thr Phe Ala Asp Tyr Thr
       110                 115                 120
   gcc agg acg tcg ttc gag cgg ccg ctg ctg agc ggg gtg gcg tac gcg      435
   Ala Arg Thr Ser Phe Glu Arg Pro Leu Leu Ser Gly Val Ala Tyr Ala
   125                 130                 135                 140
   cag cgg gtg gtg cac ggc gac agg gag agc ttc gag cgc cag cag ggc      483
   Gln Arg Val Val His Gly Asp Arg Glu Ser Phe Glu Arg Gln Gln Gly
                   145                 150                 155
   tgg atc atc aag acc atg aag cac gag ccg tct ccg gtg cag gat gag      531
   Trp Ile Ile Lys Thr Met Lys His Glu Pro Ser Pro Val Gln Asp Glu
               160                 165                 170
   tac gcg ccg gtc gtt tac tcg cag gag acc gtc tcc tac att gag ggg      579
   Tyr Ala Pro Val Val Tyr Ser Gln Glu Thr Val Ser Tyr Ile Glu Gly
           175                 180                 185
   ctc gac atg atg tcc ggc gag gag gac cgt gag aac att ttg agg tca      627
   Leu Asp Met Met Ser Gly Glu Glu Asp Arg Glu Asn Ile Leu Arg Ser
       190                 195                 200
   aga gca tcg ggg aag gca gtt ctt act aga ccg ttc cgg ctc atg tcg      675
   Arg Ala Ser Gly Lys Ala Val Leu Thr Arg Pro Phe Arg Leu Met Ser
   205                 210                 215                 220
   aat cac ctg ggt gtc gta ttg act ttt cct gtc tac cat gtc gat ctt      723
   Asn His Leu Gly Val Val Leu Thr Phe Pro Val Tyr His Val Asp Leu
                   225                 230                 235
   tct tct gat gcc aag gag gag gat cgt gtt gct gcc acc gca gga tac      771
   Ser Ser Asp Ala Lys Glu Glu Asp Arg Val Ala Ala Thr Ala Gly Tyr
               240                 245                 250
   ctt ggg gga tca ttt gat gta gaa tca tta gtg gaa aat ttg ttt agg      819
   Leu Gly Gly Ser Phe Asp Val Glu Ser Leu Val Glu Asn Leu Phe Arg
           255                 260                 265
   cag cta gct ggc aat cag gaa ttg gtg gta aat gtt tat gat gtc aca      867
   Gln Leu Ala Gly Asn Gln Glu Leu Val Val Asn Val Tyr Asp Val Thr
       270                 275                 280
   aac agt tcg aac cct ctt gtc atg tat gga tcg gaa gtt tct ctt ggc      915
   Asn Ser Ser Asn Pro Leu Val Met Tyr Gly Ser Glu Val Ser Leu Gly
   285                 290                 295                 300
   aac ccc tca cca tcg cac atc tgc atg cta gat ttt ggc gat cca ttc      963
   Asn Pro Ser Pro Ser His Ile Cys Met Leu Asp Phe Gly Asp Pro Phe
                   305                 310                 315
   aga aag cat cat atg gtt tgc aga tac aga aac aag cct cag ctc cca     1011
   Arg Lys His His Met Val Cys Arg Tyr Arg Asn Lys Pro Gln Leu Pro
               320                 325                 330
   tgg tct gca ata tct tcg tca tct ggt gta ttt gtc ata tgt atg ctt     1059
   Trp Ser Ala Ile Ser Ser Ser Ser Gly Val Phe Val Ile Cys Met Leu
           335                 340                 345
   gtg ggg tac atc gtg ggt gcc gct tgg agt cgt tat gat aat gtt aag      1107
   Val Gly Tyr Ile Val Gly Ala Ala Trp Ser Arg Tyr Asp Asn Val Lys
       350                 355                 360
   gaa gat tgc cgg aaa atg gag gag ctg aaa aaa cag gca gaa gca gcc      1155
   Glu Asp Cys Arg Lys Met Glu Glu Leu Lys Lys Gln Ala Glu Ala Ala
   365                 370                 375                 380
   gat gtt gct aaa tct cag ttc ctt gca act gtt tct cat gag atc aga      1203
   Asp Val Ala Lys Ser Gln Phe Leu Ala Thr Val Ser His Glu Ile Arg
                   385                 390                 395
   acg ccc atg aat gga gtt cta ggg atg ctt gat atg ctg tta gac act      1251
   Thr Pro Met Asn Gly Val Leu Gly Met Leu Asp Met Leu Leu Asp Thr
               400                 405                 410
   gac cta acg tcg acc cag agg gat ttt gca caa aca gct caa gtc tgt      1299
   Asp Leu Thr Ser Thr Gln Arg Asp Phe Ala Gln Thr Ala Gln Val Cys
           415                 420                 425
   gga aag gct tta ata tca cta atc aat gaa gtg ctt gac aga gcg aaa      1347
   Gly Lys Ala Leu Ile Ser Leu Ile Asn Glu Val Leu Asp Arg Ala Lys
       430                 435                 440
   att gaa gcc gga aag ttg gat ctt gag tct gta cca ttt gac ctg aga      1395
   Ile Glu Ala Gly Lys Leu Asp Leu Glu Ser Val Pro Phe Asp Leu Arg
   445                 450                 455                 460
   tcc atc ctt gat gat gtc atc tca tta ttt tct tca aag tca aga gag      1443
   Ser Ile Leu Asp Asp Val Ile Ser Leu Phe Ser Ser Lys Ser Arg Glu
                   465                 470                 475
   aag gga att gag ctt gct gta tat gtc tct gaa aga gtt cct gaa ctc      1491
   Lys Gly Ile Glu Leu Ala Val Tyr Val Ser Glu Arg Val Pro Glu Leu
               480                 485                 490
   ttg ttg ggt gat cct gga agg ttt cgg cag ata att aca aat tta gtg      1539
   Leu Leu Gly Asp Pro Gly Arg Phe Arg Gln Ile Ile Thr Asn Leu Val
           495                 500                 505
   ggc aac tca att aag ttc aca gaa cgg gga cat att ttt gta caa gtt      1587
   Gly Asn Ser Ile Lys Phe Thr Glu Arg Gly His Ile Phe Val Gln Val
       510                 515                 520
   cat ctg gca gat cac tca aat ctt gca aca gaa tcc aaa gtt gag tca      1635
   His Leu Ala Asp His Ser Asn Leu Ala Thr Glu Ser Lys Val Glu Ser
   525                 530                 535                 540
   gtg gct aac ggg atg aat gga cat aaa gat gag aaa act gct gta gca      1683
   Val Ala Asn Gly Met Asn Gly His Lys Asp Glu Lys Thr Ala Val Ala
                   545                 550                 555
   acc agt gtt tct ctc aac aca cta agt ggt ttt gaa gct gct gat agc      1731
   Thr Ser Val Ser Leu Asn Thr Leu Ser Gly Phe Glu Ala Ala Asp Ser
               560                 565                 570
   cga aat agt tgg gaa aac ttc aag ctt ttg ctt tct tat gag aaa aat      1779
   Arg Asn Ser Trp Glu Asn Phe Lys Leu Leu Leu Ser Tyr Glu Lys Asn
           575                 580                 585
   gag atg ccc tat gaa agt gta tct gat aaa gtt act ctt gta gta agt      1827
   Glu Met Pro Tyr Glu Ser Val Ser Asp Lys Val Thr Leu Val Val Ser
       590                 595                 600
   gtg gaa gat aca ggg ata ggt ata cca ttg gat gcc caa gcc aag gtg      1875
   Val Glu Asp Thr Gly Ile Gly Ile Pro Leu Asp Ala Gln Ala Lys Val
   605                 610                 615                 620
   ttc act cct ttc atg cag gcc gat agt tcg act tcc agg aca tat ggt      1923
   Phe Thr Pro Phe Met Gln Ala Asp Ser Ser Thr Ser Arg Thr Tyr Gly
                   625                 630                 635
   ggt act gga att gga ttg agc atc agc aaa tgt ctt gtt gaa cta atg      1971
   Gly Thr Gly Ile Gly Leu Ser Ile Ser Lys Cys Leu Val Glu Leu Met
               640                 645                 650
   ggt ggt cag ata aac ttt gtt agc cga cca cat gtt ggg agt aca ttc      2019
   Gly Gly Gln Ile Asn Phe Val Ser Arg Pro His Val Gly Ser Thr Phe
           655                 660                 665
   aca ttt act gca gct ctc caa aga tgt gac aga agc gct att ggt gac      2067
   Thr Phe Thr Ala Ala Leu Gln Arg Cys Asp Arg Ser Ala Ile Gly Asp
       670                 675                 680
   agt aag cct gtt atg ttg cac cct ctt cca tcc agt ttc aaa ggt tta      2115
   Ser Lys Pro Val Met Leu His Pro Leu Pro Ser Ser Phe Lys Gly Leu
   685                 690                 695                 700
   tct gca tta ttg gtt gat aga aga cca gta aga gct act gta act aag      2163
   Ser Ala Leu Leu Val Asp Arg Arg Pro Val Arg Ala Thr Val Thr Lys
                   705                 710                 715
   tat cac ttg caa agg ctg gga att gcc tgc gat gtt gtt gct acc att      2211
   Tyr His Leu Gln Arg Leu Gly Ile Ala Cys Asp Val Val Ala Thr Ile
               720                 725                 730
   gaa ttg gct ctt ggt gtg ctg tcc ggg aga aat ggc agt tct cta acc      2259
   Glu Leu Ala Leu Gly Val Leu Ser Gly Arg Asn Gly Ser Ser Leu Thr
           735                 740                 745
   agc acg aag caa cca tgc atg tta ttg att gag agt gat tca tgg ggc      2307
   Ser Thr Lys Gln Pro Cys Met Leu Leu Ile Glu Ser Asp Ser Trp Gly
       750                 755                 760
   ttc aag att gat gta cct tta cga tct cga ctc ctg gag atg aag cag      2355
   Phe Lys Ile Asp Val Pro Leu Arg Ser Arg Leu Leu Glu Met Lys Gln
   765                 770                 775                 780
   aat ggt cca cct gga ttg ccc aaa act atc ctt ctc gca gct gca gaa      2403
   Asn Gly Pro Pro Gly Leu Pro Lys Thr Ile Leu Leu Ala Ala Ala Glu
                   785                 790                 795
   tcg ggc aaa ctc aaa gca cac tat gca gtt gat tct gtg atc acg aag      2451
   Ser Gly Lys Leu Lys Ala His Tyr Ala Val Asp Ser Val Ile Thr Lys
               800                 805                 810
   cct ctg aaa gca agc gga ctt gcc gct tgt cta ttc caa aca ctt ggc      2499
   Pro Leu Lys Ala Ser Gly Leu Ala Ala Cys Leu Phe Gln Thr Leu Gly
           815                 820                 825
   atc aca cag tca agc aac gag aga cgc gac aac tca ggt tcc ctt cat      2547
   Ile Thr Gln Ser Ser Asn Glu Arg Arg Asp Asn Ser Gly Ser Leu His
       830                 835                 840
   ggg ttg ctc ctt ggc aag aac ata ttg gtg gtt gat gac aac aag gta      2595
   Gly Leu Leu Leu Gly Lys Asn Ile Leu Val Val Asp Asp Asn Lys Val
   845                 850                 855                 860
   aat ctt aga gtg gct gct ggc aca tta aag aaa ttc gga gcg aag gtg      2643
   Asn Leu Arg Val Ala Ala Gly Thr Leu Lys Lys Phe Gly Ala Lys Val
                   865                 870                 875
   gag tgc gtg gag agt gga aaa gat gct ctc gcc agc cta caa gtt cca      2691
   Glu Cys Val Glu Ser Gly Lys Asp Ala Leu Ala Ser Leu Gln Val Pro
               880                 885                 890
   cat aag ttc cat ctt tgt ctc atg gac att cag atg ccc gaa atg gat      2739
   His Lys Phe His Leu Cys Leu Met Asp Ile Gln Met Pro Glu Met Asp
           895                 900                 905
   ggg ttc gag gcc acc aag caa ata agg gca atg gaa gcg aag gca aat      2787
   Gly Phe Glu Ala Thr Lys Gln Ile Arg Ala Met Glu Ala Lys Ala Asn
       910                 915                 920
   gag cag gca gtc gcc tgt gac gat tca gat acg gat ggc gcg aca agg      2835
   Glu Gln Ala Val Ala Cys Asp Asp Ser Asp Thr Asp Gly Ala Thr Arg
   925                 930                 935                 940
   gcg gca aga tgg cac ctg cct gtc ctt gca atg acc gcc gat gtc atc      2883
   Ala Ala Arg Trp His Leu Pro Val Leu Ala Met Thr Ala Asp Val Ile
                   945                 950                 955
   cag gcc acc cat gag gag tgc aca aag tac ggg atg gat ggg tac gtc      2931
   Gln Ala Thr His Glu Glu Cys Thr Lys Tyr Gly Met Asp Gly Tyr Val
               960                 965                 970
   acg aag ccc ttc gag gag aag cag ctc ttc cag gcg ctg cag aag ttc      2979
   Thr Lys Pro Phe Glu Glu Lys Gln Leu Phe Gln Ala Leu Gln Lys Phe
           975                 980                 985
   ttg gac cct ggc atg tcc agc taa cacccaagtg ctgcgttcgt tgcaagtgag    3033
   Leu Asp Pro Gly Met Ser Ser
       990                 995
   gcaccattct cct                                                       3046
<210> 12
   <211> 995
   <212> PRT
   <213> Zea mays
<400> 12
   Met Gly Val Gly Gly Gly Gly Gly Gly Glu Ala Ala Ala Val Ser Ala
   1               5                   10                  15
   Pro Ala Pro Ala Glu Glu Ala Gly Lys Asp Ala Glu Asp Gly Gly Gly
               20                  25                  30
   Trp Thr Leu Lys Ala Lys Leu Ile Ala Val Ala Val Leu Val Trp Val
           35                  40                  45
   Leu Gly Ala Leu Ala Leu Gly Val Phe Leu His Ser Tyr Phe Arg His
       50                  55                  60
   Ala Ala Leu Arg Lys Ala Glu Glu Gly Leu Val Ser Met Cys Glu Glu
   65                  70                  75                  80
   Arg Ala Arg Met Leu Gln Asp Gln Phe Ala Val Ser Val Asn His Val
                   85                  90                  95
   His Ala Leu Ala Ile Leu Val Ala Thr Phe His Tyr Glu Lys Arg Pro
               100                 105                 110
   Pro Ala Leu Asp Gln Asn Thr Phe Ala Asp Tyr Thr Ala Arg Thr Ser
           115                 120                 125
   Phe Glu Arg Pro Leu Leu Ser Gly Val Ala Tyr Ala Gln Arg Val Val
       130                 135                 140
   His Gly Asp Arg Glu Ser Phe Glu Arg Gln Gln Gly Trp Ile Ile Lys
   145                 150                 155                 160
   Thr Met Lys His Glu Pro Ser Pro Val Gln Asp Glu Tyr Ala Pro Val
                   165                 170                 175
   Val Tyr Ser Gln Glu Thr Val Ser Tyr Ile Glu Gly Leu Asp Met Met
               180                 185                 190
   Ser Gly Glu Glu Asp Arg Glu Asn Ile Leu Arg Ser Arg Ala Ser Gly
           195                 200                 205
   Lys Ala Val Leu Thr Arg Pro Phe Arg Leu Met Ser Asn His Leu Gly
       210                 215                 220
   Val Val Leu Thr Phe Pro Val Tyr His Val Asp Leu Ser Ser Asp Ala
   225                 230                 235                 240
   Lys Glu Glu Asp Arg Val Ala Ala Thr Ala Gly Tyr Leu Gly Gly Ser
                   245                 250                 255
   Phe Asp Val Glu Ser Leu Val Glu Asn Leu Phe Arg Gln Leu Ala Gly
               260                 265                 270
   Asn Gln Glu Leu Val Val Asn Val Tyr Asp Val Thr Asn Ser Ser Asn
           275                 280                 285
   Pro Leu Val Met Tyr Gly Ser Glu Val Ser Leu Gly Asn Pro Ser Pro
       290                 295                 300
   Ser His Ile Cys Met Leu Asp Phe Gly Asp Pro Phe Arg Lys His His
   305                 310                 315                 320
   Met Val Cys Arg Tyr Arg Asn Lys Pro Gln Leu Pro Trp Ser Ala Ile
                   325                 330                 335
   Ser Ser Ser Ser Gly Val Phe Val Ile Cys Met Leu Val Gly Tyr Ile
               340                 345                 350
   Val Gly Ala Ala Trp Ser Arg Tyr Asp Asn Val Lys Glu Asp Cys Arg
           355                 360                 365
   Lys Met Glu Glu Leu Lys Lys Gln Ala Glu Ala Ala Asp Val Ala Lys
       370                 375                 380
   Ser Gln Phe Leu Ala Thr Val Ser His Glu Ile Arg Thr Pro Met Asn
   385                 390                 395                 400
   Gly Val Leu Gly Met Leu Asp Met Leu Leu Asp Thr Asp Leu Thr Ser
                   405                 410                 415
   Thr Gln Arg Asp Phe Ala Gln Thr Ala Gln Val Cys Gly Lys Ala Leu
               420                 425                 430
   Ile Ser Leu Ile Asn Glu Val Leu Asp Arg Ala Lys Ile Glu Ala Gly
           435                 440                 445
   Lys Leu Asp Leu Glu Ser Val Pro Phe Asp Leu Arg Ser Ile Leu Asp
       450                 455                 460
   Asp Val Ile Ser Leu Phe Ser Ser Lys Ser Arg Glu Lys Gly Ile Glu
   465                 470                 475                 480
   Leu Ala Val Tyr Val Ser Glu Arg Val Pro Glu Leu Leu Leu Gly Asp
                   485                 490                 495
   Pro Gly Arg Phe Arg Gln Ile Ile Thr Asn Leu Val Gly Asn Ser Ile
               500                 505                 510
   Lys Phe Thr Glu Arg Gly His Ile Phe Val Gln Val His Leu Ala Asp
   515 520 525
   His Ser Asn Leu Ala Thr Glu Ser Lys Val Glu Ser Val Ala Asn Gly
   530 535 540
   Met Asn Gly His Lys Asp Glu Lys Thr Ala Val Ala Thr Ser Val Ser
   545 550 555 560
   Leu Asn Thr Leu Ser Gly Phe Glu Ala Ala Asp Ser Arg Asn Ser Trp
   565 570 575
   Glu Asn Phe Lys Leu Leu Leu Ser Tyr Glu Lys Asn Glu Met Pro Tyr
   580 585 590
   Glu Ser Val Ser Asp Lys Val Thr Leu Val Val Ser Val Glu Asp Thr
   595 600 605
   Gly Ile Gly Ile Pro Leu Asp Ala Gln Ala Lys Val Phe Thr Pro Phe
   610 615 620
   Met Gln Ala Asp Ser Ser Thr Ser Arg Thr Tyr Gly Gly Thr Gly Ile
   625 630 635 640
   Gly Leu Ser Ile Ser Lys Cys Leu Val Glu Leu Met Gly Gly Gln Ile
   645 650 655
   Asn Phe Val Ser Arg Pro His Val Gly Ser Thr Phe Thr Phe Thr Ala
   660 665 670
   Ala Leu Gln Arg Cys Asp Arg Ser Ala Ile Gly Asp Ser Lys Pro Val
   675 680 685
   Met Leu His Pro Leu Pro Ser Ser Phe Lys Gly Leu Ser Ala Leu Leu
   690 695 700
   Val Asp Arg Arg Pro Val Arg Ala Thr Val Thr Lys Tyr His Leu Gln
   705 710 715 720
   Arg Leu Gly Ile Ala Cys Asp Val Val Ala Thr Ile Glu Leu Ala Leu
   725 730 735
   Gly Val Leu Ser Gly Arg Asn Gly Ser Ser Leu Thr Ser Thr Lys Gln
   740 745 750
   Pro Cys Met Leu Leu Ile Glu Ser Asp Ser Trp Gly Phe Lys Ile Asp
   755 760 765
   Val Pro Leu Arg Ser Arg Leu Leu Glu Met Lys Gln Asn Gly Pro Pro
       770                 775                 780
   Gly Leu Pro Lys Thr Ile Leu Leu Ala Ala Ala Glu Ser Gly Lys Leu
   785                 790                 795                 800
   Lys Ala His Tyr Ala Val Asp Ser Val Ile Thr Lys Pro Leu Lys Ala
                   805                 810                 815
   Ser Gly Leu Ala Ala Cys Leu Phe Gln Thr Leu Gly Ile Thr Gln Ser
               820                 825                 830
   Ser Asn Glu Arg Arg Asp Asn Ser Gly Ser Leu His Gly Leu Leu Leu
           835                 840                 845
   Gly Lys Asn Ile Leu Val Val Asp Asp Asn Lys Val Asn Leu Arg Val
       850                 855                 860
   Ala Ala Gly Thr Leu Lys Lys Phe Gly Ala Lys Val Glu Cys Val Glu
   865                 870                 875                 880
   Ser Gly Lys Asp Ala Leu Ala Ser Leu Gln Val Pro His Lys Phe His
                   885                 890                 895
   Leu Cys Leu Met Asp Ile Gln Met Pro Glu Met Asp Gly Phe Glu Ala
               900                 905                 910
   Thr Lys Gln Ile Arg Ala Met Glu Ala Lys Ala Asn Glu Gln Ala Val
           915                 920                 925
   Ala Cys Asp Asp Ser Asp Thr Asp Gly Ala Thr Arg Ala Ala Arg Trp
       930                 935                 940
   His Leu Pro Val Leu Ala Met Thr Ala Asp Val Ile Gln Ala Thr His
   945                 950                 955                 960
   Glu Glu Cys Thr Lys Tyr Gly Met Asp Gly Tyr Val Thr Lys Pro Phe
                   965                 970                 975
   Glu Glu Lys Gln Leu Phe Gln Ala Leu Gln Lys Phe Leu Asp Pro Gly
               980                 985                 990
   Met Ser Ser
           995
<210> 13
   <211> 3318
   <212> DNA
   <213> Cucurbita maxima
<220>
   <221> CDS
   <222> (1) .. (2946)
<220>
   <221> 3'UTR
   <222> (2947)..(3318)
<400> 13
   atg cag gtg agc gat aac tct gtg ggt ttg aag tgg aat gag caa atg        48
   Met Gln Val Ser Asp Asn Ser Val Gly Leu Lys Trp Asn Glu Gln Met
   1               5                   10                  15
   gga aca aca aag aag ggt tac aca ttt gtt caa gct aac agg gct tgg        96
   Gly Thr Thr Lys Lys Gly Tyr Thr Phe Val Gln Ala Asn Arg Ala Trp
               20                  25                  30
   ctt aga aag tat ctt ctg ttc tgg att atg ggg atg gcg ttt atc agc       144
   Leu Arg Lys Tyr Leu Leu Phe Trp Ile Met Gly Met Ala Phe Ile Ser
           35                  40                  45
   atg tta atc tat aat ggc atg gat gct gat atc aaa gtg agg agg aat       192
   Met Leu Ile Tyr Asn Gly Met Asp Ala Asp Ile Lys Val Arg Arg Asn
       50                  55                  60
   gaa gtg ttg ggg agt atg tgt gag cag agg gca agg atg ttg cag gat       240
   Glu Val Leu Gly Ser Met Cys Glu Gln Arg Ala Arg Met Leu Gln Asp
   65                  70                  75                  80
   caa ttc aat gtt agt gtt aac cat gtt cat gcc ttg gct gtc ctt gtt       288
   Gln Phe Asn Val Ser Val Asn His Val His Ala Leu Ala Val Leu Val
                   85                  90                  95
   tcc acc ttt cat tac ttc aaa aac cct tct gct att gat cag gaa act       336
   Ser Thr Phe His Tyr Phe Lys Asn Pro Ser Ala Ile Asp Gln Glu Thr
               100                 105                 110
   ttt gca gaa tac aca gcc aga act gct ttt gaa cgg cct cta ctc agt       384
   Phe Ala Glu Tyr Thr Ala Arg Thr Ala Phe Glu Arg Pro Leu Leu Ser
           115                 120                 125
   ggg gtg gcg tat gca caa aga gtg att cat tcg gag agg gat atc ttc       432
   Gly Val Ala Tyr Ala Gln Arg Val Ile His Ser Glu Arg Asp Ile Phe
       130                 135                 140
   gaa aag caa cac ggg tgg atg ata aga aca atg gaa aag gaa cct tcg       480
   Glu Lys Gln His Gly Trp Met Ile Arg Thr Met Glu Lys Glu Pro Ser
   145                 150                 155                 160
   ccc gat cga gat gaa tat gca cca gta ata ttt tct caa gaa aca gtc       528
   Pro Asp Arg Asp Glu Tyr Ala Pro Val Ile Phe Ser Gln Glu Thr Val
                   165                 170                 175
   tcg tat att gaa tcg ttg gat atg atg tca gga gag gag gac cgg gaa       576
   Ser Tyr Ile Glu Ser Leu Asp Met Met Ser Gly Glu Glu Asp Arg Glu
               180                 185                 190
   aat att ttg agg gct aga gca aca gga aag gct gtc tta aca aga ccc      624
   Asn Ile Leu Arg Ala Arg Ala Thr Gly Lys Ala Val Leu Thr Arg Pro
           195                 200                 205
   ttc agg ctg ctg ggt tcc cat cat ctt gga gtt gtt ttg aca ttt cct      672
   Phe Arg Leu Leu Gly Ser His His Leu Gly Val Val Leu Thr Phe Pro
       210                 215                 220
   gtt tac aaa ttc aaa ttg cca tcc ata ccg act gaa gaa gaa cgg ata      720
   Val Tyr Lys Phe Lys Leu Pro Ser Ile Pro Thr Glu Glu Glu Arg Ile
   225                 230                 235                 240
   gaa gca aca gca ggc tac gtt ggc gga gcc ttt gat gtt gag tca ctc      768
   Glu Ala Thr Ala Gly Tyr Val Gly Gly Ala Phe Asp Val Glu Ser Leu
                   245                 250                 255
   gtg gag aac ttg ttt ggg cag ctt gca ggg aat cag gcc att ttg gta      816
   Val Glu Asn Leu Phe Gly Gln Leu Ala Gly Asn Gln Ala Ile Leu Val
               260                 265                 270
   aat gta tat gat gtc acg aac tct tct gat ctt ctc gtg atg tat ggt      864
   Asn Val Tyr Asp Val Thr Asn Ser Ser Asp Leu Leu Val Met Tyr Gly
           275                 280                 285
   cat caa tat caa gat ggt gac ttg tcg ctt tca cat gag agc agc ctt      912
   His Gln Tyr Gln Asp Gly Asp Leu Ser Leu Ser His Glu Ser Ser Leu
       290                 295                 300
   gat ttc gga gat cca ttc agg aag cat ttg atg att tgt aga tat cag      960
   Asp Phe Gly Asp Pro Phe Arg Lys His Leu Met Ile Cys Arg Tyr Gln
   305                 310                 315                 320
   cag agg gct ccc aca tcc tgg act gcc cta act act gca ttc tta ttc     1008
   Gln Arg Ala Pro Thr Ser Trp Thr Ala Leu Thr Thr Ala Phe Leu Phe
                   325                 330                 335
   ttc gtg atc ggt ttg tta gtt gga tat att ttg tat ggt gca gca act     1056
   Phe Val Ile Gly Leu Leu Val Gly Tyr Ile Leu Tyr Gly Ala Ala Thr
               340                 345                 350
   cac att gtg aag gtt gaa gat gat ttt cat gaa atg caa gta ctg aaa     1104
   His Ile Val Lys Val Glu Asp Asp Phe His Glu Met Gln Val Leu Lys
           355                 360                 365
   gtt cga gcg gag gct gcc gat gta gca aaa tcc cag ttt ctt gca act     1152
   Val Arg Ala Glu Ala Ala Asp Val Ala Lys Ser Gln Phe Leu Ala Thr
       370                 375                 380
   gtt tct cat gaa att agg aca cca atg aat ggc atc ctc gga atg ctt     1200
   Val Ser His Glu Ile Arg Thr Pro Met Asn Gly Ile Leu Gly Met Leu
   385                 390                 395                 400
   gct ctg ctt ctg gat aca gat cta agt tcc aca cag aag gat tat gct     1248
   Ala Leu Leu Leu Asp Thr Asp Leu Ser Ser Thr Gln Lys Asp Tyr Ala
                   405                 410                 415
   caa act gcc cag gct tgt gga aag gca ttg ata gca tta ata aat gag     1296
   Gln Thr Ala Gln Ala Cys Gly Lys Ala Leu Ile Ala Leu Ile Asn Glu
               420                 425                 430
   gtt ctt gac cgg gca aaa att gaa gct gga aag tta gaa ctg gaa gca      1344
   Val Leu Asp Arg Ala Lys Ile Glu Ala Gly Lys Leu Glu Leu Glu Ala
           435                 440                 445
   gtt cca ttc gac att cga tca ata ctt gat gac gtg cta tct tta ttt      1392
   Val Pro Phe Asp Ile Arg Ser Ile Leu Asp Asp Val Leu Ser Leu Phe
       450                 455                 460
   tcc gag aag tcc aga caa aag ggt ctg gag ctg gca gtt ttt gtt tct      1440
   Ser Glu Lys Ser Arg Gln Lys Gly Leu Glu Leu Ala Val Phe Val Ser
   465                 470                 475                 480
   gat aaa gtt cca gaa att gta att gga gat cct gga aga ttc aga caa      1488
   Asp Lys Val Pro Glu Ile Val Ile Gly Asp Pro Gly Arg Phe Arg Gln
                   485                 490                 495
   att ata aca aat ctt gtg ggt aac tct gtt aag ttt act gaa aga gga      1536
   Ile Ile Thr Asn Leu Val Gly Asn Ser Val Lys Phe Thr Glu Arg Gly
               500                 505                 510
   cat ata ttt gtt aaa gta cac cta gct gag aat tca aaa gtc tcc atg      1584
   His Ile Phe Val Lys Val His Leu Ala Glu Asn Ser Lys Val Ser Met
           515                 520                 525
   gac tcg gaa tac gtc aac gga ata tcc gac agt ggc tta ttc gta ttg      1632
   Asp Ser Glu Tyr Val Asn Gly Ile Ser Asp Ser Gly Leu Phe Val Leu
       530                 535                 540
   gat ggt cgt gaa ttt caa act ttg agt gga cgc gag gca gcc gat gat      1680
   Asp Gly Arg Glu Phe Gln Thr Leu Ser Gly Arg Glu Ala Ala Asp Asp
   545                 550                 555                 560
   cag aac agt tgg gat aac ttc aag cat cta atc gct gac gac aac ttc      1728
   Gln Asn Ser Trp Asp Asn Phe Lys His Leu Ile Ala Asp Asp Asn Phe
                   565                 570                 575
   cag tcg aat gcc gct tca aac aac tca gca gtt acc aac aag ggt tgt      1776
   Gln Ser Asn Ala Ala Ser Asn Asn Ser Ala Val Thr Asn Lys Gly Cys
               580                 585                 590
   gat cat gtt act ttg atg gta agt gtg gag gat act gga att ggg atc      1824
   Asp His Val Thr Leu Met Val Ser Val Glu Asp Thr Gly Ile Gly Ile
           595                 600                 605
   ctt tta cat gcc caa aat cga gtt ttc aca ccc ttc atg caa gca gat      1872
   Leu Leu His Ala Gln Asn Arg Val Phe Thr Pro Phe Met Gln Ala Asp
       610                 615                 620
   agc tcg acc tcc cga aat tat gga ggg act ggt att ggt ttg agt atc      1920
   Ser Ser Thr Ser Arg Asn Tyr Gly Gly Thr Gly Ile Gly Leu Ser Ile
   625                 630                 635                 640
   agc aaa tgt tta gtt gag tta atg ggt ggt cag atc aac ttc ata agc      1968
   Ser Lys Cys Leu Val Glu Leu Met Gly Gly Gln Ile Asn Phe Ile Ser
                   645                 650                 655
   cgg cct cag att gga agc acg ttt tcc ttc act gct gta ttt gga aaa      2016
   Arg Pro Gln Ile Gly Ser Thr Phe Ser Phe Thr Ala Val Phe Gly Lys
               660                 665                 670
   tgt aag aaa aac tcg atg aat gat atg aaa aag ccc aac tct gaa gaa      2064
   Cys Lys Lys Asn Ser Met Asn Asp Met Lys Lys Pro Asn Ser Glu Glu
           675                 680                 685
   ctt ccc ccc agt ttt aga gga atg aaa gca ata gta gtt gat agc aaa      2112
   Leu Pro Pro Ser Phe Arg Gly Met Lys Ala Ile Val Val Asp Ser Lys
       690                 695                 700
   cat gta cga gct tct gta acc agg tat cat ttg aag aga ctt ggt atc      2160
   His Val Arg Ala Ser Val Thr Arg Tyr His Leu Lys Arg Leu Gly Ile
   705                 710                 715                 720
   ata gtt gaa gtc acc aat agc atc aac atg gca gct tct tta ttc aga      2208
   Ile Val Glu Val Thr Asn Ser Ile Asn Met Ala Ala Ser Leu Phe Arg
                   725                 730                 735
   gaa aat gga tcc aca ctg cca aga aac aca atc ctt cca gat atg atc      2256
   Glu Asn Gly Ser Thr Leu Pro Arg Asn Thr Ile Leu Pro Asp Met Ile
               740                 745                 750
   tta gtt gaa aag gac ata cta aat tct gat gag gaa tgt ggg atc att      2304
   Leu Val Glu Lys Asp Ile Leu Asn Ser Asp Glu Glu Cys Gly Ile Ile
           755                 760                 765
   cat cat ctg aac tgg aaa ccg aac ggt agt tcg gtt aag ttt cca aag      2352
   His His Leu Asn Trp Lys Pro Asn Gly Ser Ser Val Lys Phe Pro Lys
       770                 775                 780
   ctg atc ctt ctc gct acc aat att gcc act gct gaa cta gac aag gca      2400
   Leu Ile Leu Leu Ala Thr Asn Ile Ala Thr Ala Glu Leu Asp Lys Ala
   785                 790                 795                 800
   aga gca gca ggt ttt gca gac acc gtg atc atg aag ccg ttg agg gcg      2448
   Arg Ala Ala Gly Phe Ala Asp Thr Val Ile Met Lys Pro Leu Arg Ala
                   805                 810                 815
   act atg gtg gct gcc tgt ctt caa caa gta ctc ggg gtt aag aat cag      2496
   Thr Met Val Ala Ala Cys Leu Gln Gln Val Leu Gly Val Lys Asn Gln
               820                 825                 830
   aga cgg ccg aat ggt tct gct ttc ctc cag agc ctt ctc tgt ggc aag      2544
   Arg Arg Pro Asn Gly Ser Ala Phe Leu Gln Ser Leu Leu Cys Gly Lys
           835                 840                 845
   aga atc tta att gtt gat gac aac cga gta aac cgt cgg gtc gct gca      2592
   Arg Ile Leu Ile Val Asp Asp Asn Arg Val Asn Arg Arg Val Ala Ala
       850                 855                 860
   ggc gct ctg aag aaa ttt ggt gca gat gtt gag tgt gca gat agc ggg      2640
   Gly Ala Leu Lys Lys Phe Gly Ala Asp Val Glu Cys Ala Asp Ser Gly
   865                 870                 875                 880
   aaa tct gca ctg aag ttg ctt cag cta ccg cat aat ttt gat gct tgc      2688
   Lys Ser Ala Leu Lys Leu Leu Gln Leu Pro His Asn Phe Asp Ala Cys
                   885                 890                 895
   ttc atg gat att caa atg cct gaa atg gat ggg ttt gag gcg act cgt      2736
   Phe Met Asp Ile Gln Met Pro Glu Met Asp Gly Phe Glu Ala Thr Arg
               900                 905                 910
   cgt atc agg aca atg gag gtc gag gca aac aaa gga gga ttg tct gca      2784
   Arg Ile Arg Thr Met Glu Val Glu Ala Asn Lys Gly Gly Leu Ser Ala
           915                 920                 925
   aca gaa ggc aaa cgg cct ata cca ata tta gca atg act gca gac gtg      2832
   Thr Glu Gly Lys Arg Pro Ile Pro Ile Leu Ala Met Thr Ala Asp Val
       930                 935                 940
   att cat gct acg tac gaa gaa tgc ctg aaa tgc ggt atg aat ggt tac      2880
   Ile His Ala Thr Tyr Glu Glu Cys Leu Lys Cys Gly Met Asn Gly Tyr
   945                 950                 955                 960
   gtc tcg aaa ccc ttt gaa gaa gaa aat cta tac aag gaa gtt gcc cga      2928
   Val Ser Lys Pro Phe Glu Glu Glu Asn Leu Tyr Lys Glu Val Ala Arg
                   965                 970                 975
   ttt ttc aaa aaa cca tag tccatcaaaa gcttcatgaa tgacaagagg             2976
   Phe Phe Lys Lys Pro
               980
   tcatcagctg tagagctcct tttggtgggt tggaaagtac cagcaagttt tgacaccatt   3036
   gctggtgcta actgtcctgt tgctggtgac cgaggaaccg agttcaacga gggcgtcggg   3096
   aattctcgac tgccattgag actcgggttt gagctgctac catttctaac caactaaata   3156
   ttttattttg actagaatgt gagtacctac ctgtatacta caccagaaat atccatcccc   3216
   aaatggatgt ataattatgg ttgcaagggg aaggagctaa attgtaaatg ctcatatttc   3276
   taagacctct tctaaacatc tttatagttg gttggcttag gc                       3318
<210> 14
   <211> 981
   <212> PRT
   <213> Cucurbita maxima
<400> 14
   Met Gln Val Ser Asp Asn Ser Val Gly Leu Lys Trp Asn Glu Gln Met
   1               5                   10                  15
   Gly Thr Thr Lys Lys Gly Tyr Thr Phe Val Gln Ala Asn Arg Ala Trp
               20                  25                  30
   Leu Arg Lys Tyr Leu Leu Phe Trp Ile Met Gly Met Ala Phe Ile Ser
           35                  40                  45
   Met Leu Ile Tyr Asn Gly Met Asp Ala Asp Ile Lys Val Arg Arg Asn
       50                  55                  60
   Glu Val Leu Gly Ser Met Cys Glu Gln Arg Ala Arg Met Leu Gln Asp
   65                  70                  75                  80
   Gln Phe Asn Val Ser Val Asn His Val His Ala Leu Ala Val Leu Val
   85 90 95
   Ser Thr Phe His Tyr Phe Lys Asn Pro Ser Ala Ile Asp Gln Glu Thr
   100 105 110
   Phe Ala Glu Tyr Thr Ala Arg Thr Ala Phe Glu Arg Pro Leu Leu Ser
   115 120 125
   Gly Val Ala Tyr Ala Gln Arg Val Ile His Ser Glu Arg Asp Ile Phe
   130 135 140
   Glu Lys Gln His Gly Trp Met Ile Arg Thr Met Glu Lys Glu Pro Ser
   145 150 155 160
   Pro Asp Arg Asp Glu Tyr Ala Pro Val Ile Phe Ser Gln Glu Thr Val
   165 170 175
   Ser Tyr Ile Glu Ser Leu Asp Met Met Ser Gly Glu Glu Asp Arg Glu
   180 185 190
   Asn Ile Leu Arg Ala Arg Ala Thr Gly Lys Ala Val Leu Thr Arg Pro
   195 200 205
   Phe Arg Leu Leu Gly Ser His His Leu Gly Val Val Leu Thr Phe Pro
   210 215 220
   Val Tyr Lys Phe Lys Leu Pro Ser Ile Pro Thr Glu Glu Glu Arg Ile
   225 230 235 240
   Glu Ala Thr Ala Gly Tyr Val Gly Gly Ala Phe Asp Val Glu Ser Leu
   245 250 255
   Val Glu Asn Leu Phe Gly Gln Leu Ala Gly Asn Gln Ala Ile Leu Val
   260 265 270
   Asn Val Tyr Asp Val Thr Asn Ser Ser Asp Leu Leu Val Met Tyr Gly
   275 280 285
   His Gln Tyr Gln Asp Gly Asp Leu Ser Leu Ser His Glu Ser Ser Leu
   290 295 300
   Asp Phe Gly Asp Pro Phe Arg Lys His Leu Met Ile Cys Arg Tyr Gln
   305 310 315 320
   Gln Arg Ala Pro Thr Ser Trp Thr Ala Leu Thr Thr Ala Phe Leu Phe
   325 330 335
   Phe Val Ile Gly Leu Leu Val Gly Tyr Ile Leu Tyr Gly Ala Ala Thr
   340 345 350
   His Ile Val Lys Val Glu Asp Asp Phe His Glu Met Gln Val Leu Lys
   355 360 365
   Val Arg Ala Glu Ala Ala Asp Val Ala Lys Ser Gln Phe Leu Ala Thr
   370 375 380
   Val Ser His Glu Ile Arg Thr Pro Met Asn Gly Ile Leu Gly Met Leu
   385 390 395 400
   Ala Leu Leu Leu Asp Thr Asp Leu Ser Ser Thr Gln Lys Asp Tyr Ala
   405 410 415
   Gln Thr Ala Gln Ala Cys Gly Lys Ala Leu Ile Ala Leu Ile Asn Glu
   420 425 430
   Val Leu Asp Arg Ala Lys Ile Glu Ala Gly Lys Leu Glu Leu Glu Ala
   435 440 445
   Val Pro Phe Asp Ile Arg Ser Ile Leu Asp Asp Val Leu Ser Leu Phe
   450 455 460
   Ser Glu Lys Ser Arg Gln Lys Gly Leu Glu Leu Ala Val Phe Val Ser
   465 470 475 480
   Asp Lys Val Pro Glu Ile Val Ile Gly Asp Pro Gly Arg Phe Arg Gln
   485 490 495
   Ile Ile Thr Asn Leu Val Gly Asn Ser Val Lys Phe Thr Glu Arg Gly
   500 505 510
   His Ile Phe Val Lys Val His Leu Ala Glu Asn Ser Lys Val Ser Met
   515 520 525
   Asp Ser Glu Tyr Val Asn Gly Ile Ser Asp Ser Gly Leu Phe Val Leu
   530 535 540
   Asp Gly Arg Glu Phe Gln Thr Leu Ser Gly Arg Glu Ala Ala Asp Asp
   545 550 555 560
   Gln Asn Ser Trp Asp Asn Phe Lys His Leu Ile Ala Asp Asp Asn Phe
   565 570 575
   Gln Ser Asn Ala Ala Ser Asn Asn Ser Ala Val Thr Asn Lys Gly Cys
               580                 585                 590
   Asp His Val Thr Leu Met Val Ser Val Glu Asp Thr Gly Ile Gly Ile
           595                 600                 605
   Leu Leu His Ala Gln Asn Arg Val Phe Thr Pro Phe Met Gln Ala Asp
       610                 615                 620
   Ser Ser Thr Ser Arg Asn Tyr Gly Gly Thr Gly Ile Gly Leu Ser Ile
   625                 630                 635                 640
   Ser Lys Cys Leu Val Glu Leu Met Gly Gly Gln Ile Asn Phe Ile Ser
                   645                 650                 655
   Arg Pro Gln Ile Gly Ser Thr Phe Ser Phe Thr Ala Val Phe Gly Lys
               660                 665                 670
   Cys Lys Lys Asn Ser Met Asn Asp Met Lys Lys Pro Asn Ser Glu Glu
           675                 680                 685
   Leu Pro Pro Ser Phe Arg Gly Met Lys Ala Ile Val Val Asp Ser Lys
       690                 695                 700
   His Val Arg Ala Ser Val Thr Arg Tyr His Leu Lys Arg Leu Gly Ile
   705                 710                 715                 720
   Ile Val Glu Val Thr Asn Ser Ile Asn Met Ala Ala Ser Leu Phe Arg
                   725                 730                 735
   Glu Asn Gly Ser Thr Leu Pro Arg Asn Thr Ile Leu Pro Asp Met Ile
               740                 745                 750
   Leu Val Glu Lys Asp Ile Leu Asn Ser Asp Glu Glu Cys Gly Ile Ile
           755                 760                 765
   His His Leu Asn Trp Lys Pro Asn Gly Ser Ser Val Lys Phe Pro Lys
       770                 775                 780
   Leu Ile Leu Leu Ala Thr Asn Ile Ala Thr Ala Glu Leu Asp Lys Ala
   785                 790                 795                 800
   Arg Ala Ala Gly Phe Ala Asp Thr Val Ile Met Lys Pro Leu Arg Ala
                   805                 810                 815
   Thr Met Val Ala Ala Cys Leu Gln Gln Val Leu Gly Val Lys Asn Gln
   820 825 830
   Arg Arg Pro Asn Gly Ser Ala Phe Leu Gln Ser Leu Leu Cys Gly Lys
   835 840 845
   Arg Ile Leu Ile Val Asp Asp Asn Arg Val Asn Arg Arg Val Ala Ala
   850 855 860
   Gly Ala Leu Lys Lys Phe Gly Ala Asp Val Glu Cys Ala Asp Ser Gly
   865 870 875 880
   Lys Ser Ala Leu Lys Leu Leu Gln Leu Pro His Asn Phe Asp Ala Cys
   885 890 895
   Phe Met Asp Ile Gln Met Pro Glu Met Asp Gly Phe Glu Ala Thr Arg
   900 905 910
   Arg Ile Arg Thr Met Glu Val Glu Ala Asn Lys Gly Gly Leu Ser Ala
   915 920 925
   Thr Glu Gly Lys Arg Pro Ile Pro Ile Leu Ala Met Thr Ala Asp Val
   930 935 940
   Ile His Ala Thr Tyr Glu Glu Cys Leu Lys Cys Gly Met Asn Gly Tyr
   945 950 955 960
   Val Ser Lys Pro Phe Glu Glu Glu Asn Leu Tyr Lys Glu Val Ala Arg
   965 970 975
   Phe Phe Lys Lys Pro
   980
<210> 15
   <211> 993
   <212> PRT
   <213> Lotus corniculatus
<220>
   <221> Mutation
   <222> (266) .. (266)
   <223> X: is Xaa (any amino acid other than Leu)
<220>
   <221> misc feature
   <222> (266) .. (266)
   <223> Xaa can be any naturally occurring amino acid
<400> 15
   Met Gly Leu Gly Phe Lys Met Gln Gln Ser His His Pro Val Ala Leu
   1               5                   10                  15
   Lys Leu His Glu Gln Ala Gly Ser Gln Arg Lys Phe Thr Phe Ile Gln
               20                  25                  30
   Asn Phe Arg Asn Trp Phe Leu Pro Leu Leu Phe Val Trp Phe Ile Val
           35                  40                  45
   Met Ala Ala Phe Gly Ala Cys Ile Tyr His Lys Met Asp Ala Glu Thr
       50                  55                  60
   Lys Val Arg Arg Lys Glu Val Leu Gly Ser Leu Cys Asp Gln Arg Ala
   65                  70                  75                  80
   Arg Met Leu Gln Asp Gln Phe Ser Val Ser Val Asn His Val His Ala
                   85                  90                  95
   Leu Ala Ile Leu Val Ser Thr Phe His Tyr Tyr Arg Asn Thr Ser Ala
               100                 105                 110
   Ile Asp Gln Glu Thr Phe Ala Glu Tyr Thr Ala Arg Thr Ala Phe Glu
           115                 120                 125
   Arg Pro Leu Met Ser Gly Val Ala Tyr Ala Gln Arg Val Val His Ser
       130                 135                 140
   Glu Arg Glu Arg Phe Glu Lys Gln His Gly Trp Val Ile Lys Thr Met
   145                 150                 155                 160
   Glu Arg Val Pro Ser Gly Val Arg Asp Glu Tyr Ala Ala Val Ile Phe
                   165                 170                 175
   Ala Gln Glu Thr Val Ser Tyr Leu Glu Ser Ile Asp Met Met Ser Gly
               180                 185                 190
   Glu Glu Asp Arg Glu Asn Ile Leu Arg Ala Arg Ala Thr Gly Lys Ala
           195                 200                 205
   Val Leu Thr Ser Pro Phe Arg Leu Leu Asp Ser His His Leu Gly Val
       210                 215                 220
   Val Leu Thr Phe Pro Val Tyr Lys Ser Lys Leu Pro Pro Glu Pro Thr
   225                 230                 235                 240
   Thr Glu Glu Val Ile Lys Ala Ile Ala Gly Tyr Ile Gly Gly Ser Phe
                   245                 250                 255
   Asp Val Glu Ser Leu Val Glu Asn Leu Xaa Gly Gln Leu Ala Gly Asn
               260                 265                 270
   Gln Ala Ile Leu Val Lys Val Tyr Asp Ile Thr Asn Ser Ser Asp Pro
           275                 280                 285
   Leu Ile Met Tyr Gly Ser Gln Tyr Glu Glu Gly Asp Met Ser Leu Val
       290                 295                 300
   His Glu Ser Lys Leu Asp Phe Gly Asp Pro Tyr Arg Lys His His Met
   305                 310                 315                 320
   Ile Cys Arg Tyr His Gln Gln Ala Pro Thr Asn Trp Ile Ala Tyr Thr
                   325                 330                 335
   Thr Ala Phe Leu Phe Phe Val Ile Leu Cys Leu Val Gly Tyr Ile Leu
               340                 345                 350
   Tyr Ala Ala Gly Thr His Ile Val Lys Val Glu Asp Asp Tyr Asn Ala
           355                 360                 365
   Met Gln Asp Leu Lys Val Lys Ala Glu Ala Ala Asp Ile Ala Lys Ser
       370                 375                 380
   Gln Phe Leu Ala Thr Val Ser His Glu Ile Arg Thr Pro Met Asn Gly
   385                 390                 395                 400
   Ile Leu Gly Met Leu Gly Leu Leu Leu Arg Thr Glu Leu Ser Ser Thr
                   405                 410                 415
   Gln Arg Asp Tyr Ala Gln Thr Ala Gln Ala Cys Gly Lys Ala Leu Ile
               420                 425                 430
   Ala Leu Ile Asn Glu Val Leu Asp Arg Ala Lys Ile Glu Ala Gly Lys
           435                 440                 445
   Leu Glu Leu Glu Ala Val Pro Phe Asp Leu Arg Ser Ile Leu Asp Asp
       450                 455                 460
   Val Leu Ser Leu Phe Ser Glu Lys Ser Arg His Lys Gly Leu Glu Leu
   465                 470                 475                 480
   Ala Val Phe Val Ser Asp Lys Val Pro Asp Ile Val Met Gly Asp Pro
   485 490 495
   Gly Arg Phe Arg Gln Ile Val Thr Asn Leu Val Gly Asn Ser Val Lys
   500 505 510
   Phe Thr Glu Arg Gly His Ile Phe Val Lys Val His Leu Ala Glu Lys
   515 520 525
   Arg Gln Cys Thr Met Asn Gly Lys Cys Glu Thr Phe Leu Asn Gly Gly
   530 535 540
   Cys Asp Asp Val Leu His Val Ser Gly Ser Tyr Asn Leu Lys Thr Leu
   545 550 555 560
   Ser Gly Tyr Glu Ala Ala Asp Glu Arg Asn Ser Trp Asp Asn Phe Lys
   565 570 575
   His His Ile Ala Asp Glu Glu Phe Phe Phe Asp Ala Ser Val Lys Lys
   580 585 590
   Leu Ala Ser Ser Glu Ser Tyr Glu Gln Val Thr Leu Met Val Ser Val
   595 600 605
   Glu Asp Thr Gly Ile Gly Ile Ser Phe Ser Ala Gln Asp Ser Ile Phe
   610 615 620
   Met Pro Phe Val Gln Ala Asp Ser Ser Thr Ser Arg Asn Tyr Gly Gly
   625 630 635 640
   Thr Gly Ile Gly Leu Ser Ile Ser Lys Cys Leu Val Glu Leu Met Gly
   645 650 655
   Gly Gln Ile Asn Phe Ile Ser Arg Pro Gln Val Gly Ser Thr Phe Ser
   660 665 670
   Phe Thr Ala Asp Phe Gly Thr Phe Lys Lys Asn Ser Thr Thr Asp Met
   675 680 685
   Lys Lys Leu Asn Phe Glu Asp Leu Pro Ser Ser Phe Arg Gly Leu Lys
   690 695 700
   Ala Ile Val Val Asp Gly Lys Pro Val Arg Ala Ala Val Thr Arg Tyr
   705 710 715 720
   His Leu Lys Arg Leu Gly Ile Gln Ala Lys Val Ala Ile Ser Ile Asn
   725 730 735
   Lys Ala Val Ser Leu Cys Gly Lys Asn Gly Ser Leu Thr Ser Ala Leu
   740 745 750
   Phe Gln Pro Asp Ile Ile Phe Val Glu Lys Asp Ser Trp Val Ser Gly
   755 760 765
   Glu Asp Gly Gly Ile Phe Asn Ala Phe Lys Met Pro Gln Met Ile Leu
   770 775 780
   Leu Ala Thr Asn Ile Cys Asn Ala Glu Phe Asp Lys Ala Lys Ala Ala
   785 790 795 800
   Gly Phe Ser Asp Thr Val Ile Met Lys Pro Leu Arg Ala Ser Met Leu
   805 810 815
   Ala Ala Cys Leu Gln Gln Val Phe Gly Thr Gly Lys Thr Arg Gln Phe
   820 825 830
   Gly Lys Asp Met Ser Asn Gly Ser Ser Val Arg Ser Leu Leu Cys Gly
   835 840 845
   Lys Lys Ile Leu Val Val Asp Asp Asn Leu Val Asn Arg Arg Val Ala
   850 855 860
   Ala Gly Ala Leu Lys Asn Phe Gly Ala Asp Val Lys Cys Ala Ala Ser
   865 870 875 880
   Gly Lys Ala Ala Leu Glu Met Leu Gln Tyr Pro His Asp Phe Asp Ala
   885 890 895
   Cys Phe Met Asp Ile Gln Met Pro Glu Met Asp Gly Phe Glu Ala Thr
   900 905 910
   Arg Arg Ile Arg Met Met Glu Arg Glu Ala Ser Glu Gln Leu Lys Ser
   915 920 925
   Glu Ser Gly Glu Glu Asn Gly Lys Lys Ser Glu Phe His Met Pro Ile
   930 935 940
   Leu Ala Met Thr Ala Asp Val Ile His Ala Thr Tyr Asp Lys Cys Leu
   945 950 955 960
   Asn Cys Gly Met Asp Gly Tyr Val Ser Lys Pro Phe Glu Glu Glu Asn
   965 970 975
   Leu Tyr Gln Ala Val Ala Lys Phe Phe Lys Ser Lys Pro Ala Ser Asp
               980                 985                 990
   Ser
<210> 16
   <211> 1003
   <212> PRT
   <213> Medicago truncatula
<220>
   <221> Mutation
   <222> (267)..(267)
   <223> X = Xaa (any amino aicd other than Leu)
<220>
   <221> misc feature
   <222> (267)..(267)
   <223> Xaa can be any naturally occurring amino acid
<400> 16
   Met Gly Leu Leu Leu Lys Met Lys Met Gln Asn Gln His His Pro Leu
   1               5                   10                  15
   Ala Ser Lys Leu Gln Glu Gln Thr Gly Asn Lys Arg Tyr Thr Phe Ile
               20                  25                  30
   Gln Ala His Arg Ala Trp Leu Leu Lys Leu Met Phe Leu Trp Ile Leu
           35                  40                  45
   Leu Met Ala Leu Ile Ser Arg Ile Ile Tyr Ser Lys Met Asp Val Gly
       50                  55                  60
   Thr Lys Val Arg Arg Lys Glu Val Leu Gly Ser Leu Cys Asp Gln Arg
   65                  70                  75                  80
   Ala Arg Met Leu Gln Asp Gln Phe Ser Val Ser Val Asn His Val His
                   85                  90                  95
   Ala Leu Ala Ile Leu Val Ser Thr Phe His Tyr Tyr Arg Asn Pro Ser
               100                 105                 110
   Ala Ile Asp Gln Glu Thr Phe Ala Glu Tyr Thr Ala Arg Thr Ala Phe
           115                 120                 125
   Glu Arg Pro Leu Leu Ser Gly Val Ala Tyr Ala Gln Arg Val Val Asn
       130                 135                 140
   Ser Glu Arg Glu Gln Phe Glu Lys Gln His Gly Val Val Ile Lys Thr
   145                 150                 155                 160
   Met Glu Arg Glu Ala Ser Pro Val Arg Asp Glu Tyr Ala Pro Val Ile
                   165                 170                 175
   Phe Ala Gln Glu Thr Val Ser Tyr Leu Glu Ser Ile Asp Met Met Ser
               180                 185                 190
   Gly Glu Glu Asp Arg Glu Asn Ile Met Arg Ala Arg Ala Thr Gly Lys
           195                 200                 205
   Ala Val Leu Thr Ser Pro Phe Arg Leu Leu Gly Ser His His Leu Gly
       210                 215                 220
   Val Val Leu Thr Phe Pro Val Tyr Lys Ser Lys Leu Pro Pro Asn Pro
   225                 230                 235                 240
   Thr Thr Glu Glu Leu Ile Lys Ala Thr Ala Gly Tyr Val Gly Gly Ser
                   245                 250                 255
   Phe Asp Val Glu Ser Leu Val Glu Asn Leu Xaa Gly Gln Leu Ala Gly
               260                 265                 270
   His Gln Ala Ile Leu Val Asn Val Tyr Asp Val Thr Asn Ser Ser Asp
           275                 280                 285
   Pro Leu Ile Met Tyr Gly Asn Gln Tyr Glu Glu Gly Asp Val Ser Leu
       290                 295                 300
   Val His Glu Ser Lys Leu Asp Phe Gly Asp Pro Tyr Arg Lys His Gln
   305                 310                 315                 320
   Met Ile Cys Arg Tyr His Gln Lys Ala Pro Pro Asn Trp Thr Ala Leu
                   325                 330                 335
   Ser Thr Ala Ile Leu Phe Phe Val Ile Leu Leu Leu Ile Gly Tyr Ile
               340                 345                 350
   Leu Tyr Gly Ala Gly Asn His Ile Val Lys Val Glu Asp Asp Phe His
           355                 360                 365
   Glu Met Gln Glu Leu Lys Val Arg Ala Glu Ala Ala Asp Val Ala Lys
       370                 375                 380
   Ser Gln Phe Leu Ala Thr Val Ser His Glu Ile Arg Thr Pro Met Asn
   385 390 395 400
   Gly Ile Leu Gly Met Leu Gly Leu Leu Leu Arg Thr Glu Leu Asn Ser
   405 410 415
   Thr Gln Arg Asp Tyr Ala Gln Thr Ala Gln Ala Cys Gly Lys Ala Leu
   420 425 430
   Ile Ala Leu Ile Asn Glu Val Leu Asp Arg Ala Lys Ile Glu Ala Gly
   435 440 445
   Lys Leu Glu Leu Glu Ala Val Pro Phe Asp Leu Arg Ser Ile Leu Asp
   450 455 460
   Asp Val Leu Ser Leu Phe Ser Glu Lys Ser Arg His Lys Gly Leu Glu
   465 470 475 480
   Leu Ala Val Phe Val Ser Asp Lys Val Pro Asp Ile Val Met Gly Asp
   485 490 495
   Pro Gly Arg Phe Arg Gln Ile Val Thr Asn Leu Val Gly Asn Ser Val
   500 505 510
   Lys Phe Thr Glu Arg Gly His Ile Phe Val Lys Val His Leu Ser Glu
   515 520 525
   Asn Arg Lys Pro Val Thr Asn Gly Lys His Glu Thr Tyr Arg Asn Gly
   530 535 540
   Gly Ser Glu Glu Val Val His Ala Ser Gly Gly Tyr Asn Leu Lys Thr
   545 550 555 560
   Leu Ser Gly Tyr Glu Ala Ala Asp Glu Arg Asn Asn Trp Asp Asn Phe
   565 570 575
   Asn His Leu Ile Ala Asp Glu Glu Phe Phe Cys Asp Ala Ser Thr Lys
   580 585 590
   Lys Val Ala Ser Asn Glu Phe Tyr Glu Gln Val Thr Leu Met Val Cys
   595 600 605
   Val Glu Asp Thr Gly Ile Gly Ile Pro Phe Ser Ala Gln Asp Arg Ile
   610 615 620
   Phe Met Pro Phe Val Gln Ala Asp Ser Ser Thr Ser Arg Asn Tyr Gly
   625                 630                 635                 640
   Gly Thr Gly Ile Gly Leu Ser Ile Ser Lys Cys Leu Val Glu Leu Met
                   645                 650                 655
   Gly Gly Gln Ile Asn Phe Ile Ser Arg Pro Gln Val Gly Ser Thr Phe
               660                 665                 670
   Ser Phe Thr Ala Asp Phe Gly Ile Phe Lys Lys Asn Pro Ile Thr Glu
           675                 680                 685
   Val Lys Lys Val Asn Tyr Glu Asp Leu Pro Ser Ser Phe Arg Gly Leu
       690                 695                 700
   Lys Ala Val Val Val Asp Gly Lys Pro Val Arg Ala Ala Val Thr Arg
   705                 710                 715                 720
   Tyr His Leu Lys Arg Leu Gly Ile Gln Val Lys Val Ala Asn Ala Ile
                   725                 730                 735
   Asn Lys Ala Val Ser Leu Cys Gly Lys Asn Gly Ala Ser Ser Thr Gly
               740                 745                 750
   Leu Phe Gln Pro Asp Ile Ile Phe Val Glu Lys Asp Ser Trp Val Cys
           755                 760                 765
   Gly Glu Asp Gly Ile Phe Ser Val Arg Gln Leu Asp Trp Lys Gln Asn
       770                 775                 780
   Gly His Ile Phe Lys Met Pro Gln Met Ile Leu Leu Ala Thr Asn Ile
   785                 790                 795                 800
   Ser Asn Asp Glu Phe Asp Lys Ala Lys Ser Ala Gly Phe Ser Asp Thr
                   805                 810                 815
   Val Ile Met Lys Pro Leu Arg Ala Ser Met Val Gly Ala Cys Leu Gln
               820                 825                 830
   Gln Val Leu Gly Thr Gly Lys Lys Arg Gln Leu Gly Lys Glu Met Pro
           835                 840                 845
   Asn Gly Ser Thr Ser Val Arg Ser Leu Leu Phe Gly Lys Lys Ile Leu
       850                 855                 860
   Val Val Asp Asp Asn Val Val Asn Arg Arg Val Ala Ala Gly Ala Leu
   865 870 875 880
   Lys Asn Phe Gly Ala Asp Val Lys Cys Ala Asp Ser Gly Lys Ala Ala
   885 890 895
   Leu Glu Met Leu Gln Phe Pro His Lys Phe Asp Ala Cys Phe Met Asp
   900 905 910
   Ile Gln Met Pro Glu Met Asp Gly Phe Glu Ala Thr Arg Arg Ile Arg
   915 920 925
   Glu Met Glu Arg Thr Ala Asn Glu Glu Thr Asn Ser Glu Cys Gly Glu
   930 935 940
   Arg Lys Ser Glu Phe His Leu Pro Ile Leu Ala Met Thr Ala Asp Val
   945 950 955 960
   Ile His Ala Thr Tyr Glu Glu Cys Leu Lys Cys Gly Met Asp Gly Tyr
   965 970 975
   Val Ser Lys Pro Phe Glu Glu Glu Asn Leu Tyr Gln Ala Val Ala Lys
   980 985 990
   Phe Phe Gln Thr Lys Pro Thr Ser Val Asp Ser
   995 1000
<210> 17
   <211> 1080
   <212> PRT
   <213> Arabidopsis thaliana
<220>
   <221> Mutation
   <222> (356)..(356)
   <223> X= Xaa (any amino acid other than Leucine)
<220>
   <221> misc_feature
   <222> (356)..(356)
   <223> Xaa can be any naturally occurring amino acid
<400> 17
   Met Arg Arg Asp Phe Val Tyr Asn Asn Asn Ala Met Phe Asn Pro Leu
   1 5 10 15
   Thr Thr His Tyr Ser Ser Asp Met Asn Trp Ala Leu Asn Asn His Gln
   20 25 30
   Glu Glu Glu Glu Glu Pro Arg Arg Ile Glu Ile Ser Asp Ser Glu Ser
           35                  40                  45
   Leu Glu Asn Leu Lys Ser Ser Asp Phe Tyr Gln Leu Gly Gly Gly Gly
       50                  55                  60
   Ala Leu Asn Ser Ser Glu Lys Pro Arg Lys Ile Asp Phe Trp Arg Ser
   65                  70                  75                  80
   Gly Leu Met Gly Phe Ala Lys Met Gln Gln Gln Gln Gln Leu Gln His
                   85                  90                  95
   Ser Val Ala Val Lys Met Asn Asn Asn Asn Asn Asn Asp Leu Met Gly
               100                 105                 110
   Asn Lys Lys Gly Ser Thr Phe Ile Gln Glu His Arg Ala Leu Leu Pro
           115                 120                 125
   Lys Ala Leu Ile Leu Trp Ile Ile Ile Val Gly Phe Ile Ser Ser Gly
       130                 135                 140
   Ile Tyr Gln Trp Met Asp Asp Ala Asn Lys Ile Arg Arg Glu Glu Val
   145                 150                 155                 160
   Leu Val Ser Met Cys Asp Gln Arg Ala Arg Met Leu Gln Asp Gln Phe
                   165                 170                 175
   Ser Val Ser Val Asn His Val His Ala Leu Ala Ile Leu Val Ser Thr
               180                 185                 190
   Phe His Tyr His Lys Asn Pro Ser Ala Ile Asp Gln Glu Thr Phe Ala
           195                 200                 205
   Glu Tyr Thr Ala Arg Thr Ala Phe Glu Arg Pro Leu Leu Ser Gly Val
       210                 215                 220
   Ala Tyr Ala Glu Lys Val Val Asn Phe Glu Arg Glu Met Phe Glu Arg
   225                 230                 235                 240
   Gln His Asn Trp Val Ile Lys Thr Met Asp Arg Gly Glu Pro Ser Pro
                   245                 250                 255
   Val Arg Asp Glu Tyr Ala Pro Val Ile Phe Ser Gln Asp Ser Val Ser
               260                 265                 270
   Tyr Leu Glu Ser Leu Asp Met Met Ser Gly Glu Glu Asp Arg Glu Asn
   275 280 285
   Ile Leu Arg Ala Arg Glu Thr Gly Lys Ala Val Leu Thr Ser Pro Phe
   290 295 300
   Arg Leu Leu Glu Thr His His Leu Gly Val Val Leu Thr Phe Pro Val
   305 310 315 320
   Tyr Lys Ser Ser Leu Pro Glu Asn Pro Thr Val Glu Glu Arg Ile Ala
   325 330 335
   Ala Thr Ala Gly Tyr Leu Gly Gly Ala Phe Asp Val Glu Ser Leu Val
   340 345 350
   Glu Asn Leu Xaa Gly Gln Leu Ala Gly Asn Gln Ala Ile Val Val His
   355 360 365
   Val Tyr Asp Ile Thr Asn Ala Ser Asp Pro Leu Val Met Tyr Gly Asn
   370 375 380
   Gln Asp Glu Glu Ala Asp Arg Ser Leu Ser His Glu Ser Lys Leu Asp
   385 390 395 400
   Phe Gly Asp Pro Phe Arg Lys His Lys Met Ile Cys Arg Tyr His Gln
   405 410 415
   Lys Ala Pro Ile Pro Leu Asn Val Leu Thr Thr Val Pro Leu Phe Phe
   420 425 430
   Ala Ile Gly Phe Leu Val Gly Tyr Ile Leu Tyr Gly Ala Ala Met His
   435 440 445
   Ile Val Lys Val Glu Asp Asp Phe His Glu Met Gln Glu Leu Lys Val
   450 455 460
   Arg Ala Glu Ala Ala Asp Val Ala Lys Ser Gln Phe Leu Ala Thr Val
   465 470 475 480
   Ser His Glu Ile Arg Thr Pro Met Asn Gly Ile Leu Gly Met Leu Ala
   485 490 495
   Met Leu Leu Asp Thr Glu Leu Ser Ser Thr Gln Arg Asp Tyr Ala Gln
   500 505 510
   Thr Ala Gln Val Cys Gly Lys Ala Leu Ile Ala Leu Ile Asn Glu Val
   515 520 525
   Leu Asp Arg Ala Lys Ile Glu Ala Gly Lys Leu Glu Leu Glu Ser Val
       530                 535                 540
   Pro Phe Asp Ile Arg Ser Ile Leu Asp Asp Val Leu Ser Leu Phe Ser
   545                 550                 555                 560
   Glu Glu Ser Arg Asn Lys Gly Ile Glu Leu Ala Val Phe Val Ser Asp
                   565                 570                 575
   Lys Val Pro Glu Ile Val Lys Gly Asp Ser Gly Arg Phe Arg Gln Ile
               580                 585                 590
   Ile Ile Asn Leu Val Gly Asn Ser Val Lys Phe Thr Glu Lys Gly His
           595                 600                 605
   Ile Phe Val Lys Val His Leu Ala Glu Gln Ser Lys Asp Glu Ser Glu
       610                 615                 620
   Pro Lys Asn Ala Leu Asn Gly Gly Val Ser Glu Glu Met Ile Val Val
   625                 630                 635                 640
   Ser Lys Gln Ser Ser Tyr Asn Thr Leu Ser Gly Tyr Glu Ala Ala Asp
                   645                 650                 655
   Gly Arg Asn Ser Trp Asp Ser Phe Lys His Leu Val Ser Glu Glu Gln
               660                 665                 670
   Ser Leu Ser Glu Phe Asp Ile Ser Ser Asn Val Arg Leu Met Val Ser
           675                 680                 685
   Ile Glu Asp Thr Gly Ile Gly Ile Pro Leu Val Ala Gln Gly Arg Val
       690                 695                 700
   Phe Met Pro Phe Met Gln Ala Asp Ser Ser Thr Ser Arg Asn Tyr Gly
   705                 710                 715                 720
   Gly Thr Gly Ile Gly Leu Ser Ile Ser Lys Cys Leu Val Glu Leu Met
                   725                 730                 735
   Arg Gly Gln Ile Asn Phe Ile Ser Arg Pro His Ile Gly Ser Thr Phe
               740                 745                 750
   Trp Phe Thr Ala Val Leu Glu Lys Cys Asp Lys Cys Ser Ala Ile Asn
           755                 760                 765
   His Met Lys Lys Pro Asn Val Glu His Leu Pro Ser Thr Phe Lys Gly
       770                 775                 780
   Met Lys Ala Ile Val Val Asp Ala Lys Pro Val Arg Ala Ala Val Thr
   785                 790                 795                 800
   Arg Tyr His Met Lys Arg Leu Gly Ile Asn Val Asp Val Val Thr Ser
                   805                 810                 815
   Leu Lys Thr Ala Val Val Ala Ala Ala Ala Phe Glu Arg Asn Gly Ser
               820                 825                 830
   Pro Leu Pro Thr Lys Pro Gln Leu Asp Met Ile Leu Val Glu Lys Asp
           835                 840                 845
   Ser Trp Ile Ser Thr Glu Asp Asn Asp Ser Glu Ile Arg Leu Leu Asn
       850                 855                 860
   Ser Arg Thr Asn Gly Asn Val His His Lys Ser Pro Lys Leu Ala Leu
   865                 870                 875                 880
   Phe Ala Thr Asn Ile Thr Asn Ser Glu Phe Asp Arg Ala Lys Ser Ala
                   885                 890                 895
   Gly Phe Ala Asp Thr Val Ile Met Lys Pro Leu Arg Ala Ser Met Ile
               900                 905                 910
   Gly Ala Cys Leu Gln Gln Val Leu Glu Leu Arg Lys Thr Arg Gln Gln
           915                 920                 925
   His Pro Glu Gly Ser Ser Pro Ala Thr Leu Lys Ser Leu Leu Thr Gly
       930                 935                 940
   Lys Lys Ile Leu Val Val Asp Asp Asn Ile Val Asn Arg Arg Val Ala
   945                 950                 955                 960
   Ala Gly Ala Leu Lys Lys Phe Gly Ala Glu Val Val Cys Ala Glu Ser
                   965                 970                 975
   Gly Gln Val Ala Leu Gly Leu Leu Gln Ile Pro His Thr Phe Asp Ala
               980                 985                 990
   Cys Phe Met Asp Ile Gln Met Pro  Gln Met Asp Gly Phe  Glu Ala Thr
           995                 1000                1005
   Arg Gln  Ile Arg Met Met Glu  Lys Glu Ala Lys Glu  Lys Thr Asn
       1010                 1015                 1020
   Leu Glu  Trp His Leu Pro Ile  Leu Ala Met Thr Ala  Asp Val Ile
       1025                 1030                 1035
   His Ala  Thr Tyr Glu Glu Cys  Leu Lys Ser Gly Met  Asp Gly Tyr
       1040                 1045                 1050
   Val Ser  Lys Pro Phe Glu Glu  Glu Asn Leu Tyr Lys  Ser Val Ala
       1055                 1060                 1065
   Lys Ser  Phe Lys Pro Asn Pro  Ile Ser Pro Ser Ser
       1070                 1075                 1080
<210> 18
   <211> 1013
   <212> PRT
   <213> Oryza
<220>
   <221> Mutation
   <222> (266) .. (266)
   <223> X=Xaa (any amino acid other than Leu)
<220>
   <221> misc_feature
   <222> (266) .. (266)
   <223> Xaa can be any naturally occurring amino acid
<400> 18
   Met Gly Val Gly Gly Gly Gly Gly Gly Gly Gly Gly Glu Ala Ala Ala
   1               5                   10                  15
   Ala Val Ala Val Glu Gly Asp Glu Ala Gly Lys Gly Arg Arg Trp Trp
               20                  25                  30
   Arg Val Lys Val Lys Leu Ser Thr Val Ala Val Val Ala Trp Val Leu
           35                  40                  45
   Ala Ser Ala Ala Leu Trp Ala Gly Leu His Trp Arg Phe Arg Arg Ala
       50                  55                  60
   Ala Leu His Lys Ala Glu Glu Ala Leu Val Cys Met Cys Glu Glu Arg
   65                  70                  75                  80
   Ala Arg Met Leu Gln Asp Gln Phe Ala Val Ser Val Asn His Val His
                   85                  90                  95
   Ala Leu Ala Ile Leu Val Ala Thr Phe His Tyr Asp Lys His Pro Pro
               100                 105                 110
   Ala Leu Asp Gln Asp Thr Phe Ala Val Tyr Ala Ala Arg Thr Ser Phe
           115                 120                 125
   Glu Arg Pro Leu Leu Ser Gly Val Ala Tyr Ala Gln Arg Val Val His
       130                 135                 140
   Ala Asp Arg Glu Ser Phe Glu Arg Gln Gln Gly Trp Ile Ile Lys Thr
   145                 150                 155                 160
   Met Lys His Glu Pro Ser Pro Ala Gln Asp Glu Tyr Ala Pro Val Ile
                   165                 170                 175
   Tyr Ser Gln Glu Thr Ile Ser Tyr Ile Glu Gly Leu Asp Val Met Ser
               180                 185                 190
   Gly Glu Glu Asp Arg Glu Asn Ile Leu Arg Ala Arg Ala Thr Gly Lys
           195                 200                 205
   Ala Val Leu Thr Arg Pro Phe Arg Leu Met Ser Asn His Leu Gly Val
       210                 215                 220
   Val Leu Thr Phe Pro Val Tyr Leu Val Asp Leu Pro Asn Asp Thr Ala
   225                 230                 235                 240
   Val Glu Asp Arg Val Ala Ala Thr Ala Gly Tyr Leu Gly Gly Ala Phe
                   245                 250                 255
   Asp Val Glu Ser Leu Val Glu Asn Leu Xaa Arg Gln Leu Ala Gly Asn
               260                 265                 270
   Gln Glu Leu Val Val Asn Val Tyr Asp Val Thr Asn His Ser Asn Pro
           275                 280                 285
   Leu Val Met Tyr Gly Ser Glu Val Pro Leu Gly Ile Pro Ser Pro Ser
       290                 295                 300
   His Thr Tyr Thr Leu Asp Phe Gly Asp Pro Leu Arg Lys His Gln Met
   305                 310                 315                 320
   Val Cys Arg Tyr Arg Asn Lys Leu His Val Ser Trp Ser Ala Ile Thr
                   325                 330                 335
   Thr Pro Ser Gly Val Phe Val Ile Cys Met Leu Val Gly Tyr Ile Ile
   340 345 350
   Tyr Ala Ala Trp Ser Arg Tyr Asp Asn Val Lys Glu Asp Cys Arg Lys
   355 360 365
   Met Glu Ala Leu Lys Lys Arg Ala Glu Ala Ala Asp Ile Ala Lys Ser
   370 375 380
   Gln Phe Leu Ala Thr Val Ser His Glu Ile Arg Thr Pro Met Asn Gly
   385 390 395 400
   Val Leu Gly Met Leu Asp Met Leu Leu Asp Thr Glu Leu Lys Ser Thr
   405 410 415
   Gln Arg Asp Tyr Ala Gln Thr Ala Gln Val Cys Gly Lys Ala Leu Ile
   420 425 430
   Ser Leu Ile Asn Glu Val Leu Asp Arg Ala Lys Ile Glu Ala Gly Lys
   435 440 445
   Ile Asp Leu Glu Ser Val Pro Phe Asp Leu Arg Ser Ile Leu Asp Asp
   450 455 460
   Val Ile Ser Leu Phe Ser Ser Lys Ser Arg Glu Lys Gly Ile Glu Leu
   465 470 475 480
   Ala Val Tyr Val Ser Glu Arg Val Pro Glu Ile Leu Leu Gly Asp Pro
   485 490 495
   Gly Arg Phe Arg Gln Ile Ile Thr Asn Leu Val Gly Asn Ser Ile Lys
   500 505 510
   Ile Thr Ile Phe Thr Leu Ser Gln Phe Thr Glu Arg Gly His Ile Phe
   515 520 525
   Val Gln Val His Leu Ala Asp His Ser Asn Leu Ala Thr Glu Ala Lys
   530 535 540
   Ile Glu Pro Val Val Asn Gly Met Asn Gly His Lys Asp Glu Ala Ile
   545 550 555 560
   Ala Ile Pro Thr Ser Gly Ser His Asn Thr Leu Ser Gly Phe Glu Ala
   565 570 575
   Ala Asp Ser Arg Asn Asn Trp Glu Asn Phe Lys Leu Leu Leu Ser Tyr
   580 585 590
   Glu Lys Asn Glu Met Pro Tyr Glu Ser Asp Ser Asp Lys Val Thr Leu
   595 600 605
   Val Val Ser Val Glu Asp Thr Gly Ile Gly Ile Pro Leu His Ala Gln
   610 615 620
   Gly Arg Val Phe Thr Pro Phe Met Gln Ala Asp Ser Ser Thr Ser Arg
   625 630 635 640
   Asn Tyr Gly Gly Thr Gly Ile Gly Leu Ser Ile Ser Lys Cys Leu Val
   645 650 655
   Glu Ile Met Gly Gly Gln Ile Asn Phe Val Ser Arg Pro Leu Val Gly
   660 665 670
   Ser Thr Phe Thr Phe Thr Ala Val Leu Arg Arg Cys Asp Lys Asn Ala
   675 680 685
   Ile Ser Asp Ser Lys Thr Val Ala Leu His Pro Leu Pro Ser Ser Phe
   690 695 700
   Lys Gly Leu Ser Ala Leu Leu Val Asp Lys Arg Pro Val Arg Ala Thr
   705 710 715 720
   Val Thr Lys Tyr His Leu Gln Arg Leu Gly Ile Thr Ser Glu Val Val
   725 730 735
   Gly Thr Ile Asp Pro Thr Phe Gly Val Leu Ser Gly Arg Asn Gly Ser
   740 745 750
   Ser Leu Thr Ser Ile Gly Lys Lys Gln Pro Cys Met Leu Leu Ile Glu
   755 760 765
   Ser Asp Ser Trp Gly Pro Gln Met Asp Val Ser Leu His Ala Arg Leu
   770 775 780
   Gln Glu Met Lys Gln Ser Asp Arg Ile His Val Leu Pro Lys Val Phe
   785 790 795 800
   Leu Leu Ser Ala Ala Glu Ser Asp Lys Val Lys Lys Ile His Ala Val
   805 810 815
   Asp Ser Val Ile Pro Lys Pro Leu Lys Ala Ser Ala Leu Ala Ala Cys
   820 825 830
   Leu Phe Gln Ala Leu Gly Ile Thr Gln Pro Ser His Glu Lys Arg Asp
           835                 840                 845
   Asp Ser Gly Ser Leu His Gly Arg Asp Gly Ser Gly Ser Leu His Gly
       850                 855                 860
   Leu Leu Leu Gly Lys Asn Ile Leu Val Val Asp Asp Asn Lys Val Asn
   865                 870                 875                 880
   Leu Arg Val Ala Ala Gly Thr Leu Lys Lys Tyr Gly Ala Lys Val Glu
                   885                 890                 895
   Cys Val Glu Ser Gly Lys Asp Ala Leu Ser Leu Leu Gln Val Pro His
               900                 905                 910
   Lys Phe Asp Leu Cys Leu Met Asp Ile Gln Met Pro Glu Met Asp Gly
           915                 920                 925
   Phe Glu Ala Thr Arg Gln Ile Arg Ala Met Glu Gly Lys Ala Asn Glu
       930                 935                 940
   Gln Ala Asp Asp Ser Glu Ser Gly Ser Glu Ile Ala Ala Lys Thr Ala
   945                 950                 955                 960
   Lys Trp His Leu Pro Ile Leu Ala Met Thr Ala Asp Val Ile Gln Ala
                   965                 970                 975
   Thr His Glu Glu Cys Thr Lys Cys Gly Met Asp Gly Tyr Val Ser Lys
               980                 985                 990
   Pro Phe Glu Glu Lys Gln Leu Phe  Gln Ala Val Gln Lys  Phe Leu Gly
           995                 1000                1005
   Pro Cys  Val Ser Ser
       1010
<210> 19
   <211> 995
   <212> PRT
   <213> Zea mays
<220>
   <221> Mutation
   <222> (267)..(267)
   <223> X= Xaa (any amino acid other than Leu)
<220>
   <221> misc_feature
   <222> (267)..(267)
   <223> Xaa can be any naturally occurring amino acid
<400> 19
   Met Gly Val Gly Gly Gly Gly Gly Gly Glu Ala Ala Ala Val Ser Ala
   1               5                   10                  15
   Pro Ala Pro Ala Glu Glu Ala Gly Lys Asp Ala Glu Asp Gly Gly Gly
               20                  25                  30
   Trp Thr Leu Lys Ala Lys Leu Ile Ala Val Ala Val Leu Val Trp Val
           35                  40                  45
   Leu Gly Ala Leu Ala Leu Gly Val Phe Leu His Ser Tyr Phe Arg His
       50                  55                  60
   Ala Ala Leu Arg Lys Ala Glu Glu Gly Leu Val Ser Met Cys Glu Glu
   65                  70                  75                  80
   Arg Ala Arg Met Leu Gln Asp Gln Phe Ala Val Ser Val Asn His Val
                   85                  90                  95
   His Ala Leu Ala Ile Leu Val Ala Thr Phe His Tyr Glu Lys Arg Pro
               100                 105                 110
   Pro Ala Leu Asp Gln Asn Thr Phe Ala Asp Tyr Thr Ala Arg Thr Ser
           115                 120                 125
   Phe Glu Arg Pro Leu Leu Ser Gly Val Ala Tyr Ala Gln Arg Val Val
       130                 135                 140
   His Gly Asp Arg Glu Ser Phe Glu Arg Gln Gln Gly Trp Ile Ile Lys
   145                 150                 155                 160
   Thr Met Lys His Glu Pro Ser Pro Val Gln Asp Glu Tyr Ala Pro Val
                   165                 170                 175
   Val Tyr Ser Gln Glu Thr Val Ser Tyr Ile Glu Gly Leu Asp Met Met
               180                 185                 190
   Ser Gly Glu Glu Asp Arg Glu Asn Ile Leu Arg Ser Arg Ala Ser Gly
           195                 200                 205
   Lys Ala Val Leu Thr Arg Pro Phe Arg Leu Met Ser Asn His Leu Gly
       210                 215                 220
   Val Val Leu Thr Phe Pro Val Tyr His Val Asp Leu Ser Ser Asp Ala
   225                 230                 235                 240
   Lys Glu Glu Asp Arg Val Ala Ala Thr Ala Gly Tyr Leu Gly Gly Ser
                   245                 250                 255
   Phe Asp Val Glu Ser Leu Val Glu Asn Leu Xaa Arg Gln Leu Ala Gly
               260                 265                 270
   Asn Gln Glu Leu Val Val Asn Val Tyr Asp Val Thr Asn Ser Ser Asn
           275                 280                 285
   Pro Leu Val Met Tyr Gly Ser Glu Val Ser Leu Gly Asn Pro Ser Pro
       290                 295                 300
   Ser His Ile Cys Met Leu Asp Phe Gly Asp Pro Phe Arg Lys His His
   305                 310                 315                 320
   Met Val Cys Arg Tyr Arg Asn Lys Pro Gln Leu Pro Trp Ser Ala Ile
                   325                 330                 335
   Ser Ser Ser Ser Gly Val Phe Val Ile Cys Met Leu Val Gly Tyr Ile
               340                 345                 350
   Val Gly Ala Ala Trp Ser Arg Tyr Asp Asn Val Lys Glu Asp Cys Arg
           355                 360                 365
   Lys Met Glu Glu Leu Lys Lys Gln Ala Glu Ala Ala Asp Val Ala Lys
       370                 375                 380
   Ser Gln Phe Leu Ala Thr Val Ser His Glu Ile Arg Thr Pro Met Asn
   385                 390                 395                 400
   Gly Val Leu Gly Met Leu Asp Met Leu Leu Asp Thr Asp Leu Thr Ser
                   405                 410                 415
   Thr Gln Arg Asp Phe Ala Gln Thr Ala Gln Val Cys Gly Lys Ala Leu
               420                 425                 430
   Ile Ser Leu Ile Asn Glu Val Leu Asp Arg Ala Lys Ile Glu Ala Gly
           435                 440                 445
   Lys Leu Asp Leu Glu Ser Val Pro Phe Asp Leu Arg Ser Ile Leu Asp
       450                 455                 460
   Asp Val Ile Ser Leu Phe Ser Ser Lys Ser Arg Glu Lys Gly Ile Glu
   465                 470                 475                 480
   Leu Ala Val Tyr Val Ser Glu Arg Val Pro Glu Leu Leu Leu Gly Asp
                   485                 490                 495
   Pro Gly Arg Phe Arg Gln Ile Ile Thr Asn Leu Val Gly Asn Ser Ile
               500                 505                 510
   Lys Phe Thr Glu Arg Gly His Ile Phe Val Gln Val His Leu Ala Asp
           515                 520                 525
   His Ser Asn Leu Ala Thr Glu Ser Lys Val Glu Ser Val Ala Asn Gly
       530                 535                 540
   Met Asn Gly His Lys Asp Glu Lys Thr Ala Val Ala Thr Ser Val Ser
   545                 550                 555                 560
   Leu Asn Thr Leu Ser Gly Phe Glu Ala Ala Asp Ser Arg Asn Ser Trp
                   565                 570                 575
   Glu Asn Phe Lys Leu Leu Leu Ser Tyr Glu Lys Asn Glu Met Pro Tyr
               580                 585                 590
   Glu Ser Val Ser Asp Lys Val Thr Leu Val Val Ser Val Glu Asp Thr
           595                 600                 605
   Gly Ile Gly Ile Pro Leu Asp Ala Gln Ala Lys Val Phe Thr Pro Phe
       610                 615                 620
   Met Gln Ala Asp Ser Ser Thr Ser Arg Thr Tyr Gly Gly Thr Gly Ile
   625                 630                 635                 640
   Gly Leu Ser Ile Ser Lys Cys Leu Val Glu Leu Met Gly Gly Gln Ile
                   645                 650                 655
   Asn Phe Val Ser Arg Pro His Val Gly Ser Thr Phe Thr Phe Thr Ala
               660                 665                 670
   Ala Leu Gln Arg Cys Asp Arg Ser Ala Ile Gly Asp Ser Lys Pro Val
           675                 680                 685
   Met Leu His Pro Leu Pro Ser Ser Phe Lys Gly Leu Ser Ala Leu Leu
       690                 695                 700
   Val Asp Arg Arg Pro Val Arg Ala Thr Val Thr Lys Tyr His Leu Gln
   705 710 715 720
   Arg Leu Gly Ile Ala Cys Asp Val Val Ala Thr Ile Glu Leu Ala Leu
   725 730 735
   Gly Val Leu Ser Gly Arg Asn Gly Ser Ser Leu Thr Ser Thr Lys Gln
   740 745 750
   Pro Cys Met Leu Leu Ile Glu Ser Asp Ser Trp Gly Phe Lys Ile Asp
   755 760 765
   Val Pro Leu Arg Ser Arg Leu Leu Glu Met Lys Gln Asn Gly Pro Pro
   770 775 780
   Gly Leu Pro Lys Thr Ile Leu Leu Ala Ala Ala Glu Ser Gly Lys Leu
   785 790 795 800
   Lys Ala His Tyr Ala Val Asp Ser Val Ile Thr Lys Pro Leu Lys Ala
   805 810 815
   Ser Gly Leu Ala Ala Cys Leu Phe Gln Thr Leu Gly Ile Thr Gln Ser
   820 825 830
   Ser Asn Glu Arg Arg Asp Asn Ser Gly Ser Leu His Gly Leu Leu Leu
   835 840 845
   Gly Lys Asn Ile Leu Val Val Asp Asp Asn Lys Val Asn Leu Arg Val
   850 855 860
   Ala Ala Gly Thr Leu Lys Lys Phe Gly Ala Lys Val Glu Cys Val Glu
   865 870 875 880
   Ser Gly Lys Asp Ala Leu Ala Ser Leu Gln Val Pro His Lys Phe His
   885 890 895
   Leu Cys Leu Met Asp Ile Gln Met Pro Glu Met Asp Gly Phe Glu Ala
   900 905 910
   Thr Lys Gln Ile Arg Ala Met Glu Ala Lys Ala Asn Glu Gln Ala Val
   915 920 925
   Ala Cys Asp Asp Ser Asp Thr Asp Gly Ala Thr Arg Ala Ala Arg Trp
   930 935 940
   His Leu Pro Val Leu Ala Met Thr Ala Asp Val Ile Gln Ala Thr His
   945 950 955 960
   Glu Glu Cys Thr Lys Tyr Gly Met Asp Gly Tyr Val Thr Lys Pro Phe
   965 970 975
   Glu Glu Lys Gln Leu Phe Gln Ala Leu Gln Lys Phe Leu Asp Pro Gly
   980 985 990
   Met Ser Ser
   995
<210> 20
   <211> 981
   <212> PRT
   <213> Cucurbita maxima
<220>
   <221> Mutation
   <222> (261)..(261)
   <223> X= Xaa (any amino acid other than Leu)
<220>
   <221> misc_feature
   <222> (261)..(261)
   <223> Xaa can be any naturally occurring amino acid
<400> 20
   Met Gln Val Ser Asp Asn Ser Val Gly Leu Lys Trp Asn Glu Gln Met
   1 5 10 15
   Gly Thr Thr Lys Lys Gly Tyr Thr Phe Val Gln Ala Asn Arg Ala Trp
   20 25 30
   Leu Arg Lys Tyr Leu Leu Phe Trp Ile Met Gly Met Ala Phe Ile Ser
   35 40 45
   Met Leu Ile Tyr Asn Gly Met Asp Ala Asp Ile Lys Val Arg Arg Asn
   50 55 60
   Glu Val Leu Gly Ser Met Cys Glu Gln Arg Ala Arg Met Leu Gln Asp
   65 70 75 80
   Gln Phe Asn Val Ser Val Asn His Val His Ala Leu Ala Val Leu Val
   85 90 95
   Ser Thr Phe His Tyr Phe Lys Asn Pro Ser Ala Ile Asp Gln Glu Thr
   100 105 110
   Phe Ala Glu Tyr Thr Ala Arg Thr Ala Phe Glu Arg Pro Leu Leu Ser
           115                 120                 125
   Gly Val Ala Tyr Ala Gln Arg Val Ile His Ser Glu Arg Asp Ile Phe
       130                 135                 140
   Glu Lys Gln His Gly Trp Met Ile Arg Thr Met Glu Lys Glu Pro Ser
   145                 150                 155                 160
   Pro Asp Arg Asp Glu Tyr Ala Pro Val Ile Phe Ser Gln Glu Thr Val
                   165                 170                 175
   Ser Tyr Ile Glu Ser Leu Asp Met Met Ser Gly Glu Glu Asp Arg Glu
               180                 185                 190
   Asn Ile Leu Arg Ala Arg Ala Thr Gly Lys Ala Val Leu Thr Arg Pro
           195                 200                 205
   Phe Arg Leu Leu Gly Ser His His Leu Gly Val Val Leu Thr Phe Pro
       210                 215                 220
   Val Tyr Lys Phe Lys Leu Pro Ser Ile Pro Thr Glu Glu Glu Arg Ile
   225                 230                 235                 240
   Glu Ala Thr Ala Gly Tyr Val Gly Gly Ala Phe Asp Val Glu Ser Leu
                   245                 250                 255
   Val Glu Asn Leu Xaa Gly Gln Leu Ala Gly Asn Gln Ala Ile Leu Val
               260                 265                 270
   Asn Val Tyr Asp Val Thr Asn Ser Ser Asp Leu Leu Val Met Tyr Gly
           275                 280                 285
   His Gln Tyr Gln Asp Gly Asp Leu Ser Leu Ser His Glu Ser Ser Leu
       290                 295                 300
   Asp Phe Gly Asp Pro Phe Arg Lys His Leu Met Ile Cys Arg Tyr Gln
   305                 310                 315                 320
   Gln Arg Ala Pro Thr Ser Trp Thr Ala Leu Thr Thr Ala Phe Leu Phe
                   325                 330                 335
   Phe Val Ile Gly Leu Leu Val Gly Tyr Ile Leu Tyr Gly Ala Ala Thr
               340                 345                 350
   His Ile Val Lys Val Glu Asp Asp Phe His Glu Met Gln Val Leu Lys
   355 360 365
   Val Arg Ala Glu Ala Ala Asp Val Ala Lys Ser Gln Phe Leu Ala Thr
   370 375 380
   Val Ser His Glu Ile Arg Thr Pro Met Asn Gly Ile Leu Gly Met Leu
   385 390 395 400
   Ala Leu Leu Leu Asp Thr Asp Leu Ser Ser Thr Gln Lys Asp Tyr Ala
   405 410 415
   Gln Thr Ala Gln Ala Cys Gly Lys Ala Leu Ile Ala Leu Ile Asn Glu
   420 425 430
   Val Leu Asp Arg Ala Lys Ile Glu Ala Gly Lys Leu Glu Leu Glu Ala
   435 440 445
   Val Pro Phe Asp Ile Arg Ser Ile Leu Asp Asp Val Leu Ser Leu Phe
   450 455 460
   Ser Glu Lys Ser Arg Gln Lys Gly Leu Glu Leu Ala Val Phe Val Ser
   465 470 475 480
   Asp Lys Val Pro Glu Ile Val Ile Gly Asp Pro Gly Arg Phe Arg Gln
   485 490 495
   Ile Ile Thr Asn Leu Val Gly Asn Ser Val Lys Phe Thr Glu Arg Gly
   500 505 510
   His Ile Phe Val Lys Val His Leu Ala Glu Asn Ser Lys Val Ser Met
   515 520 525
   Asp Ser Glu Tyr Val Asn Gly Ile Ser Asp Ser Gly Leu Phe Val Leu
   530 535 540
   Asp Gly Arg Glu Phe Gln Thr Leu Ser Gly Arg Glu Ala Ala Asp Asp
   545 550 555 560
   Gln Asn Ser Trp Asp Asn Phe Lys His Leu Ile Ala Asp Asp Asn Phe
   565 570 575
   Gln Ser Asn Ala Ala Ser Asn Asn Ser Ala Val Thr Asn Lys Gly Cys
   580 585 590
   Asp His Val Thr Leu Met Val Ser Val Glu Asp Thr Gly Ile Gly Ile
   595 600 605
   Leu Leu His Ala Gln Asn Arg Val Phe Thr Pro Phe Met Gln Ala Asp
       610                 615                 620
   Ser Ser Thr Ser Arg Asn Tyr Gly Gly Thr Gly Ile Gly Leu Ser Ile
   625                 630                 635                 640
   Ser Lys Cys Leu Val Glu Leu Met Gly Gly Gln Ile Asn Phe Ile Ser
                   645                 650                 655
   Arg Pro Gln Ile Gly Ser Thr Phe Ser Phe Thr Ala Val Phe Gly Lys
               660                 665                 670
   Cys Lys Lys Asn Ser Met Asn Asp Met Lys Lys Pro Asn Ser Glu Glu
           675                 680                 685
   Leu Pro Pro Ser Phe Arg Gly Met Lys Ala Ile Val Val Asp Ser Lys
       690                 695                 700
   His Val Arg Ala Ser Val Thr Arg Tyr His Leu Lys Arg Leu Gly Ile
   705                 710                 715                 720
   Ile Val Glu Val Thr Asn Ser Ile Asn Met Ala Ala Ser Leu Phe Arg
                   725                 730                 735
   Glu Asn Gly Ser Thr Leu Pro Arg Asn Thr Ile Leu Pro Asp Met Ile
               740                 745                 750
   Leu Val Glu Lys Asp Ile Leu Asn Ser Asp Glu Glu Cys Gly Ile Ile
           755                 760                 765
   His His Leu Asn Trp Lys Pro Asn Gly Ser Ser Val Lys Phe Pro Lys
       770                 775                 780
   Leu Ile Leu Leu Ala Thr Asn Ile Ala Thr Ala Glu Leu Asp Lys Ala
   785                 790                 795                 800
   Arg Ala Ala Gly Phe Ala Asp Thr Val Ile Met Lys Pro Leu Arg Ala
                   805                 810                 815
   Thr Met Val Ala Ala Cys Leu Gln Gln Val Leu Gly Val Lys Asn Gln
               820                 825                 830
   Arg Arg Pro Asn Gly Ser Ala Phe Leu Gln Ser Leu Leu Cys Gly Lys
           835                 840                 845
   Arg Ile Leu Ile Val Asp Asp Asn Arg Val Asn Arg Arg Val Ala Ala
       850                 855                 860
   Gly Ala Leu Lys Lys Phe Gly Ala Asp Val Glu Cys Ala Asp Ser Gly
   865                 870                 875                 880
   Lys Ser Ala Leu Lys Leu Leu Gln Leu Pro His Asn Phe Asp Ala Cys
                   885                 890                 895
   Phe Met Asp Ile Gln Met Pro Glu Met Asp Gly Phe Glu Ala Thr Arg
               900                 905                 910
   Arg Ile Arg Thr Met Glu Val Glu Ala Asn Lys Gly Gly Leu Ser Ala
           915                 920                 925
   Thr Glu Gly Lys Arg Pro Ile Pro Ile Leu Ala Met Thr Ala Asp Val
       930                 935                 940
   Ile His Ala Thr Tyr Glu Glu Cys Leu Lys Cys Gly Met Asn Gly Tyr
   945                 950                 955                 960
   Val Ser Lys Pro Phe Glu Glu Glu Asn Leu Tyr Lys Glu Val Ala Arg
                   965                 970                 975
   Phe Phe Lys Lys Pro
               980